(19) Europäisches Patentamt
European Patent Office
Office européen des brevets

(11) EP 4 667 463 A1

(12) EUROPEAN PATENT APPLICATION
published in accordance with Art. 153(4) EPC

(43) Date of publication:
**24.12.2025 Bulletin 2025/52**

(21) Application number: **24756286.1**

(22) Date of filing: **08.02.2024**

(51) International Patent Classification (IPC):
***C07D 401/14*** *(2006.01)* ***C07D 401/04*** *(2006.01)*
***A61K 47/68*** *(2017.01)* ***A61K 39/00*** *(2006.01)*
***A61P 35/00*** *(2006.01)*

(52) Cooperative Patent Classification (CPC):
**A61K 39/00; A61K 47/68; A61P 35/00; C07D 401/04; C07D 401/14**

(86) International application number:
**PCT/CN2024/076883**

(87) International publication number:
**WO 2024/169913 (22.08.2024 Gazette 2024/34)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **15.02.2023 CN 202310116140**

(71) Applicant: **CSPC Megalith Biopharmaceutical Co., Ltd.**
**Shijiazhuang, Hebei 050025 (CN)**

(72) Inventors:
- **WU, Shuai**
  **Shijiazhuang, Hebei 050025 (CN)**
- **BAO, Bin**
  **Shijiazhuang, Hebei 050025 (CN)**
- **FANG, Wei**
  **Shijiazhuang, Hebei 050025 (CN)**
- **ZHANG, Xiaodan**
  **Shijiazhuang, Hebei 050025 (CN)**
- **HONG, Dingjun**
  **Shijiazhuang, Hebei 050025 (CN)**
- **YAO, Bing**
  **Shijiazhuang, Hebei 050025 (CN)**
- **XU, Hanqian**
  **Shijiazhuang, Hebei 050025 (CN)**
- **DAN, Mo**
  **Shijiazhuang, Hebei 050025 (CN)**

(74) Representative: **Gille Hrabal Partnerschaftsgesellschaft mbB**
**Patentanwälte**
**Brucknerstraße 20**
**40593 Düsseldorf (DE)**

Remarks:
The complete document including Reference Table(s) and the Sequence Listing(s) can be downloaded from the EPO website

# (54) DOMIDE MOLECULAR GLUE DERIVATIVE AND USE THEREOF

(57) Provided are a molecular glue derivative or a tautomer, mesomer, racemate, enantiomer and diastereomer thereof, a pharmaceutically acceptable salt, hydrate and solvate thereof, a use thereof, and a preparation method therefor.

EP 4 667 463 A1

## Description

The present disclosure claims priority to Chinese Patent Application No. 202310116140.4 filed with China National Intellectual Property Administration on February 15, 2023 and entitled "DOMIDE MOLECULAR GLUE DERIVATIVE AND USE THEREOF", which is incorporated herein by reference in its entirety.

## TECHNICAL FIELD

The present disclosure belongs to the technical field of pharmaceuticals, and particularly relates to a domide molecular glue derivative and use thereof for combating tumors.

## BACKGROUND

Domide compounds have been used as clinical therapeutic drugs in the fields of immunomodulation and antitumor therapy for many years. For example, thalidomide has been used for a long time in leprosy and a variety of skin diseases, such as discoid lupus erythematosus, subacute cutaneous lupus erythematosus, and Behcet's syndrome. Pomalidomide and lenalidomide are commonly used in the treatment of multiple myeloma. In recent years, studies have shown that the mechanism of action of such domide molecules is similar to that of "molecular glue", which achieves therapeutic effects by degrading the corresponding target proteins. The marketed domide small molecules mainly have problems such as high toxicity and undesirable efficacy. For example, thalidomide is the culprit behind the famous "thalidomide tragedy". An increasing number of studies are focusing on structural modifications of such domide compounds. Orum Therapeutics has developed Smol006 through such modifications, and obtained the antibody-drug conjugate ORM-5029 by integrating antibody-conjugated delivery technology. The antibody-drug conjugate shows excellent anti-tumor activity and good safety. However, during research, it was found that the activity of Smol006 is only at a moderate level. For tumors with low receptor expression, Smol006 is far from sufficient as an active moiety of antibody-drug conjugates. Therefore, there is an urgent need to develop compounds with better activity.

## SUMMARY

The present disclosure provides a domide molecular glue derivative having a more excellent anti-tumor effect and higher safety.

The present disclosure provides a compound having a structure represented by formula (I), or a tautomer, a mesomer, a racemate, an enantiomer or a diastereoisomer thereof, and a pharmaceutically acceptable salt, a hydrate or a solvate thereof:

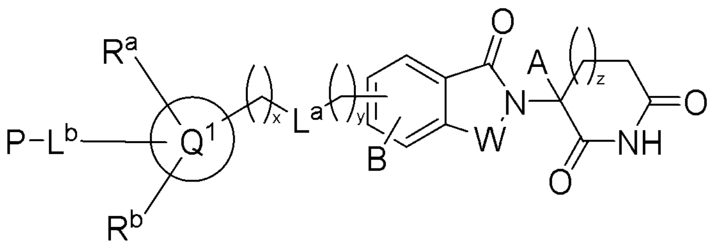

I

wherein,

A is selected from H, D, halogen, $C_{1-6}$ alkyl, $C_{1-6}$ deuterated alkyl, and $C_{1-6}$ haloalkyl;
B is selected from H, D, halogen, $-NH_2$, $-NO_2$, $C_{1-6}$ alkyl, $C_{1-6}$ deuterated alkyl, and $C_{1-6}$ haloalkyl;
W is selected from -C(=O)- or $-CH_2-$;
$L^a$ is an alkylene chain of 1 to 4 carbons, wherein each alkylene may be independently replaced with -NH-, -O-, -C(=O)-, -C(=NH)-, -C(=S)-, $-CF_2-$, -S(=O)-, $-S(=O)_2-$, -C(-OH)H-, $-C(NH_2)H-$, or -C(=N-C=N)-;
x is selected from an integer of 0-4, such as 0, 1, 2, 3, or 4;
y is selected from an integer of 0-4, such as 0, 1, 2, 3, or 4;
z is selected from an integer of 0-4, such as 0, 1, 2, 3, or 4;

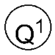 is selected from $C_{6-14}$ aryl, 5- to 6-membered heteroaryl, and 3- to 18-membered cycloalkyl;
$R^a$ and $R^b$ are each independently selected from H, D, halogen, -OH, -CN, $-NO_2$, $-NH_2$, $C_{1-6}$ alkyl, $C_{1-6}$ haloalkyl, $C_{1-6}$

alkoxy, $C_{1-6}$ haloalkoxy, $-R^c$, $-OR^c$, $-CH(R^c)OH$, $-CH_2CH(R^c)OH$, and $-NH(R^c)$;

$R^c$ is selected from H, 3- to 8-membered cycloalkyl, $C_{6-14}$ aryl, benzyl, and $C_{1-6}$ alkyl;

$L^b$ is selected from optionally substituted $C_{1-20}$ (such as $C_{1-18}$, $C_{1-16}$, $C_{1-14}$, $C_{1-12}$, $C_{1-10}$, $C_{1-8}$, $C_{1-6}$, $C_{1-4}$, $C_{3-12}$, $C_1$, $C_2$, $C_3$, $C_4$, $C_5$, $C_6$, $C_7$, $C_8$, $C_9$, $C_{10}$, $C_{11}$, $C_{12}$, $C_{13}$, $C_{14}$, $C_{15}$, and $C_{16}$) alkylene, wherein each alkylene may be independently replaced with $-CR^eR^f-$, -O-, -S-, $-NR^d-$, 3- to 8-membered cycloalkylene, or 3- to 8-membered heterocyclylene;

$R^d$ is selected from H, $C_{1-12}$ alkyl, 3- to 8-membered cycloalkyl, benzyl, $C_{6-14}$ aryl, 5- to 6-membered heteroaromatic ring, $C_{1-12}$ alkoxycarbonyl, -Boc, -Cbz, -Fmoc, acetyl, and trifluoroacetyl;

$R^e$ and $R^f$ are each independently selected from H, $C_{1-12}$ alkyl, 3- to 8-membered cycloalkyl, benzyl, $C_{6-14}$ aryl, and 5- to 6-membered heteroaryl;

P is selected from -H; when the atom attached to P is a nitrogen atom, P may be selected from linear or branched aliphatic oxycarbonyl of 1-12 carbons, -Boc, -Cbz, -Fmoc, formyl, acetyl, and trifluoroacetyl; when the atom attached to P is an oxygen atom, P may be selected from acetyl, trifluoroacetyl, trimethylsilyl, dimethyl *tert*-butylsilyl, and diphenyl tert-butylsilyl.

In some embodiments of the present disclosure, A is selected from hydrogen, deuterium, methyl, ethyl, n-propyl, isopropyl, deuterated methyl, deuterated ethyl, fluorine, chlorine, bromine, iodine, trifluoromethyl, and pentafluoroethyl.

In some embodiments of the present disclosure, B is selected from hydrogen, deuterium, methyl, ethyl, n-propyl, isopropyl, deuterated methyl, deuterated ethyl, fluorine, chlorine, bromine, iodine, trifluoromethyl, pentafluoroethyl, amino, and nitro.

In some embodiments of the present disclosure, W is selected from $-CH_2-$.

In some embodiments of the present disclosure, $L^a$ is an alkylene chain of 1 to 4 carbon atoms, wherein each alkylene may be independently replaced with -NH-, -C(=O)-, -C(=S)-, or $-CF_2-$; Q1 is selected from phenyl, naphthyl, pyridinyl, pyrazolyl, furanyl, thiazolyl, cyclopentyl, cyclohexyl, and cycloheptyl.

In some embodiments of the present disclosure, $R^a$ and $R^b$ are each independently selected from hydrogen, deuterium, fluorine, chlorine, bromine, iodine, cyano, nitro, amino, methyl, ethyl, n-propyl, isopropyl, trifluoromethyl, difluoromethyl, monofluoromethyl, pentafluoroethyl, methoxy, ethoxy, n-propoxy, isopropoxy, trifluoromethoxy, difluoromethoxy, monofluoromethoxy, pentafluoroethoxy, hydroxyl, $-R^c$, $-OR^c$, $-CH(R^c)OH$, - $CH_2CH(R^c)OH$, and $-NH(R^c)$, wherein $R^c$ is selected from hydrogen, cyclopropyl, cyclobutyl, cyclopentyl, cyclohexyl, phenyl, benzyl, methyl, ethyl, n-propyl, and isopropyl.

In some embodiments of the present disclosure, $L^b$ is selected from optionally substituted $C_{1-20}$ (such as $C_{1-18}$, $C_{1-16}$, $C_{1-14}$, $C_{1-12}$, $C_{1-10}$, $C_{1-8}$, $C_{1-6}$, $C_{1-4}$, $C_{3-12}$, $C_1$, $C_2$, $C_3$, $C_4$, $C_5$, $C_6$, $C_7$, $C_8$, $C_9$, $C_{10}$, $C_{11}$, $C_{12}$, $C_{13}$, $C_{14}$, $C_{15}$, and $C_{16}$) alkylene, wherein each alkylene may be independently replaced with $-CR^eR^f-$, -O-, -S-, $-NR^d-$, cyclopropylene, cyclobutylene, cyclopentylene, cyclohexylene, piperidinylene, tetrahydropyranylene, pyrrolidinylene, or tetrahydrofuranylene.

In some embodiments of the present disclosure, $R^d$ is selected from H, methyl, ethyl, n-propyl, isopropyl, cyclopropyl, cyclobutyl, cyclopentyl, cyclohexyl, phenyl, benzyl, pyridinyl, pyrazolyl, thiazolyl, methoxycarbonyl, ethoxycarbonyl, *n*-propoxycarbonyl, isopropoxycarbonyl, -Boc, -Cbz, -Fmoc, acetyl, and trifluoroacetyl.

In some embodiments of the present disclosure, $R^e$ and $R^f$ are each independently selected from H, methyl, ethyl, n-propyl, isopropyl, cyclopropyl, cyclobutyl, cyclopentyl, cyclohexyl, phenyl, benzyl, pyridinyl, pyrazolyl, and thiazolyl.

In some embodiments of the present disclosure, P is selected from -H; when the atom connected to P is a nitrogen atom, P may be selected from methoxycarbonyl, ethoxycarbonyl, n-propoxycarbonyl, isopropyloxycarbonyl, -Boc, -Cbz, -Fmoc, formyl, acetyl, and trifluoroacetyl; when the atom connected to P is an oxygen atom, P may be selected from acetyl, trifluoroacetyl, trimethylsilyl, dimethyl tert-butylsilyl, and diphenyl tert-butylsilyl.

In some embodiments of the present disclosure, provided is a compound having a structure represented by formula (I-1), or a tautomer, a mesomer, a racemate, an enantiomer or a diastereoisomer thereof, and a pharmaceutically acceptable salt, a hydrate or a solvate thereof:

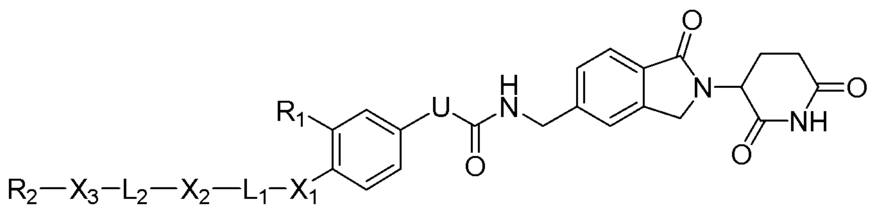

I-1

wherein U is selected from -NH-, $-CH_2-$, and $-CF_2-$;

$R_1$ is selected from hydrogen, halogen, $C_{1-6}$ alkyl, $C_{1-6}$ haloalkyl, and 3- to 8-membered cycloalkyl;

$X_1$ and $X_2$ are each independently selected from -O-, -S-, and a bond;
$X_3$ is selected from $-N(R_7)-$ and a bond;
$L_1$ and $L_2$ are each independently selected from: a bond, $-C_{1-6}$ alkyl-, $-C_{1-6}$ alkyl-$CH(R_3)$-$C_{1-6}$ alkyl-, $-C(R_3)(R_4)$-$C_{1-6}$ alkyl-, $-C_{1-6}$ alkyl-3- to 8-membered cycloalkyl-$C_{1-6}$ alkyl-, -3- to 8-membered cycloalkyl-$C_{1-6}$ alkyl-, $-C_{1-6}$ alkyl-3- to 8-membered heterocyclyl-$C_{1-6}$ alkyl-, and -3- to 8-membered heterocyclyl-$C_{1-6}$ alkyl-;
$R_3$ and $R_4$ are each independently selected from H, $C_{1-6}$ alkyl, $C_{6-14}$ aryl, and 3- to 8-membered cycloalkyl, or $R_3$ and $R_4$, together with the carbon atom attached thereto, form 3- to 8-membered cycloalkyl or 3- to 8-membered heterocyclyl;
$R_2$ is selected from H and the following optionally substituted substituents: $C_{1-6}$ alkyl, 3- to 16-membered cycloalkyl, 3- to 16-membered heterocyclyl, $-C_{1-6}$ alkyl-$C(R_5)(R_6)$-$C_{1-6}$ alkyl-, and $-C_{1-6}$ alkyl-$C(R_5)(R_6)$-;
$R_5$ and $R_6$ are each independently selected from: H, $C_{1-6}$ alkyl, $C_{6-14}$ aryl, and 3- to 8-membered cycloalkyl, or $R_5$ and $R_6$, together with the carbon atom attached thereto, form 3- to 8-membered cycloalkyl or 3- to 8-membered heterocyclyl;
$R_7$ is selected from: H and the following optionally substituted substituents: $C_{1-6}$ alkyl, 3- to 8-membered cycloalkyl, and 3- to 8-membered heterocyclyl, or $-N(R_7)(R_2)$ forms 3- to 16-membered heterocyclyl,
wherein the optionally substituted substituents are selected from -OH, $C_{1-6}$ alkyl, $(R_8)(R_9)NH$-, 3- to 8-membered heterocyclyl, $C_{1-6}$ alkyloxycarbonyl (such as tert-butoxycarbonyl (Boc), methoxycarbonyl, ethoxycarbonyl, and propoxycarbonyl), benzyloxycarbonyl (Cbz), fluorenylmethoxycarbonyl (Fmoc), and allyloxycarbonyl (Alloc);
$R_8$ and $R_9$ are each independently selected from H, $C_{1-6}$ alkyl, 3- to 8-membered cycloalkyl, 3- to 8-membered heterocyclyl, $C_{6-14}$ aryl, and benzyl;
heteroatoms of the heterocyclyl are selected from O, S, and N.

In some embodiments of the present disclosure, $R_1$ is selected from hydrogen, fluorine, chlorine, bromine, iodine, $C_{1-3}$ alkyl, $C_{1-3}$ haloalkyl, and 3- to 6-membered cycloalkyl.

In some embodiments of the present disclosure, $L_1$ and $L_2$ are each independently selected from: a bond, $-C_{1-3}$ alkyl-, $-C_{1-3}$ alkyl-$CH(R_3)$-$C_{1-3}$ alkyl-, $-C(R_3)(R_4)$-$C_{1-3}$ alkyl-, $-C_{1-3}$ alkyl-3- to 6-membered cycloalkyl-$C_{1-3}$ alkyl-, -3- to 6-membered cycloalkyl-$C_{1-3}$ alkyl-, $-C_{1-3}$ alkyl-5- to 6-membered heterocyclyl-$C_{1-3}$ alkyl-, and -5- to 6-membered heterocyclyl-$C_{1-3}$ alkyl-.

In some embodiments of the present disclosure, $R_3$ and $R_4$ are each independently selected from H, $C_{1-3}$ alkyl, phenyl, and 3- to 6-membered cycloalkyl, or $R_3$ and $R_4$, together with the carbon atom attached thereto, form 3- to 6-membered cycloalkyl or 5- to 6-membered heterocyclyl.

In some embodiments of the present disclosure, $R_2$ is selected from H and the following optionally substituted substituents: $C_{1-3}$ alkyl, 3- to 8-membered monocyclic cycloalkyl, 6- to 14-membered spiro cycloalkyl, 5- to 14-membered fused cycloalkyl, 5- to 14-membered bridged cycloalkyl, 3- to 8-membered monocyclic heterocyclyl, 6-to 14-membered spiro heterocyclyl, 5- to 14-membered fused heterocyclyl, 5- to 14-membered bridged heterocyclyl, $-C_{1-3}$ alkyl-$C(R_5)(R_6)$-$C_{1-3}$ alkyl-, and $-C_{1-3}$ alkyl-$C(R_5)(R_6)$-.

In some embodiments of the present disclosure, $R_5$ and $R_6$ are each independently selected from: H, $C_{1-3}$ alkyl, phenyl, and 3- to 6-membered cycloalkyl, or $R_5$ and $R_6$, together with the carbon atom attached thereto, form 3- to 6-membered cycloalkyl or 5- to 6-membered heterocyclyl.

In some embodiments of the present disclosure, $R_7$ is selected from: H and the following optionally substituted substituents: $C_{1-3}$ alkyl, 3- to 6-membered cycloalkyl, and 5- to 6-membered heterocyclyl, or $-N(R_7)(R_2)$ forms 3-to 8-membered monocyclic heterocyclyl, 6- to 14-membered spiro heterocyclyl, 5- to 14-membered fused heterocyclyl, or 5- to 14-membered bridged heterocyclyl.

In some embodiments of the present disclosure, the optionally substituted substituents are selected from OH-, $C_{1-3}$ alkyl, $(R_8)(R_9)NH$-, 5- to 6-membered heterocyclyl, and $C_{1-3}$ alkyloxycarbonyl.

In some embodiments of the present disclosure, $R_8$ and $R_9$ are each independently selected from H, $C_{1-3}$ alkyl, 3- to 6-membered cycloalkyl, 5- to 6-membered heterocyclyl, phenyl, and benzyl.

In some embodiments of the present disclosure, $R_1$ is selected from hydrogen, fluorine, chlorine, bromine, iodine, methyl, ethyl, n-propyl, isopropyl, n-butyl, isobutyl, tert-butyl, trifluoromethyl, difluoromethyl, monofluoromethyl, pentafluoroethyl, cyclopropyl, cyclobutyl, cyclopentyl, and cyclohexyl.

In some embodiments of the present disclosure, $L_1$ and $L_2$ are each independently selected from: a bond, methylene, ethylene, *n*-propylene, isopropylene, *n*-butylene, isobutylene, *tert*-butylene, *n*-pentylene, *n*-hexylene, -methyl-$C(R_3)(R_4)$-methyl-, -methyl-$C(R_3)(R_4)$-ethyl-, -methyl-$C(R_3)(R_4)$-propyl-, -ethyl-$C(R_3)(R_4)$-ethyl-, -propyl-$C(R_3)(R_4)$-ethyl-, -propyl-$C(R_3)(R_4)$-propyl-, $-C(R_3)(R_4)$-methyl-, $-C(R_3)(R_4)$-ethyl-, $-C(R_3)(R_4)$-propyl-, - $C(R_3)(R_4)$-butyl-, $-C(R_3)(R_4)$-pentyl-, $-C_{1-3}$ alkyl-cyclopropyl-$C_{1-3}$ alkyl-, $-C_{1-3}$ alkyl-cyclobutyl-$C_{1-3}$ alkyl-, $-C_{1-3}$ alkyl-cyclopentyl-$C_{1-3}$ alkyl-, $-C_{1-3}$ alkyl-cyclohexyl-$C_{1-3}$ alkyl-, $-C_{1-3}$ alkyl-cyclopropyl-, $-C_{1-3}$ alkyl-cyclobutyl-, - $C_{1-3}$ alkyl-cyclopentyl-, $-C_{1-3}$ alkyl-cyclohexyl-, $-C_{1-3}$ alkyl-tetrahydropyranyl-$C_{1-3}$ alkyl-, $-C_{1-3}$ alkyl-piperidinyl-$C_{1-3}$ alkyl-, $-C_{1-3}$ alkyl-tetrahydrofuranyl-$C_{1-3}$ alkyl-, $-C_{1-3}$ alkyl-pyrrolidinyl-$C_{1-3}$ alkyl-, $-C_{1-3}$ alkyl-tetrahydropyranyl, $-C_{1-3}$ alkyl-piperidinyl, $-C_{1-3}$ alkyl-tetrahydrofur-

anyl-, and -$C_{1-3}$ alkyl-pyrrolidinyl-.

In some embodiments of the present disclosure, $R_3$ and $R_4$ are each independently selected from H, methyl, ethyl, *n*-propyl, isopropyl, *n*-butyl, isobutyl, *tert*-butyl, phenyl, cyclopropyl, cyclobutyl, cyclopentyl, and cyclohexyl, or $R_3$ and $R_4$, together with the carbon atom connected thereto, form cyclopropyl, cyclobutyl, cyclopentyl, cyclohexyl, tetrahydropyranyl, piperidinyl, tetrahydrofuranyl, or pyrrolidinyl.

In some embodiments of the present disclosure, $R_2$ is selected from H and the following optionally substituted substituents: methyl, ethyl, *n*-propyl, isopropyl, *n*-butyl, isobutyl, *tert*-butyl, cyclopropyl, cyclobutyl, cyclopentyl, cyclohexyl, -methyl-C($R_5$)($R_6$)-methyl-, -methyl-C($R_5$)($R_6$)-ethyl-, -methyl-C($R_5$)($R_6$)-propyl-, -ethyl-C($R_5$)($R_6$)-ethyl-, -propyl-C($R_5$)($R_6$)-ethyl-, -propyl-C($R_5$)($R_6$)-propyl-, -C($R_5$)($R_6$)-methyl-, -C($R_5$)($R_6$)-ethyl-, -C($R_5$)($R_6$)-propyl-, -C($R_5$)($R_6$)-butyl-, -C($R_5$)($R_6$)-pentyl-, azetidinyl, piperidinyl, piperazinyl, tetrahydropyranyl, pyrrolidinyl, tetrahydrofuranyl, tetrahydrothienyl, phenyl,

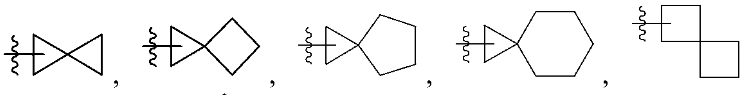 , , , , ,

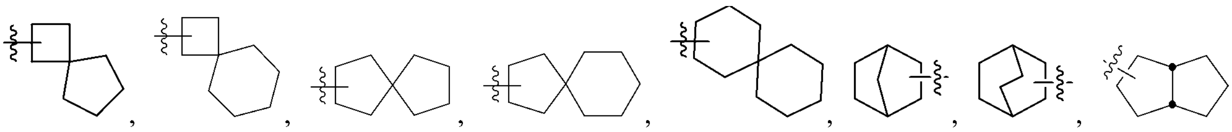 , , , , , , , ,

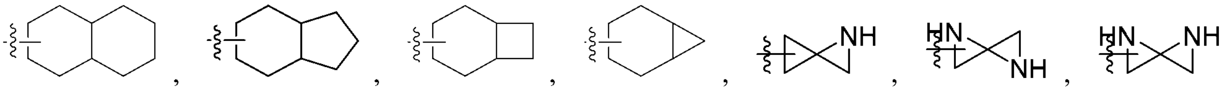 , , , , , , ,

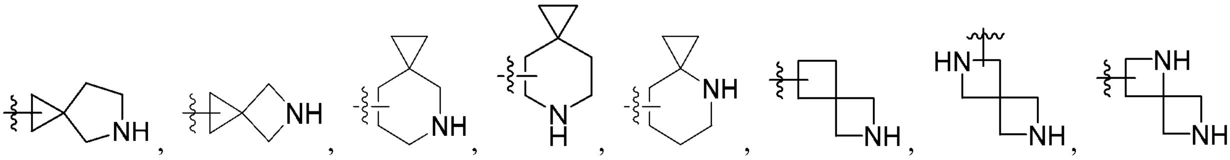 , , , , , , , ,

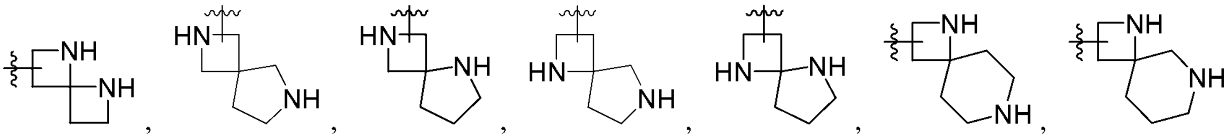 , , , , , , ,

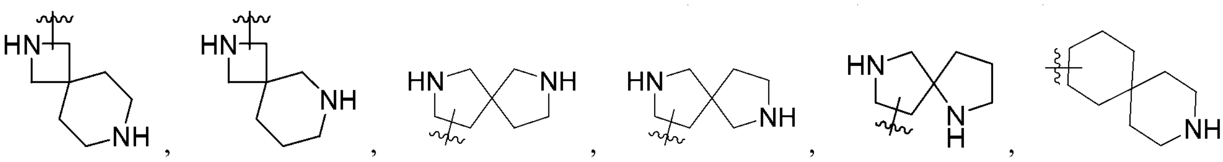 , , , , , ,

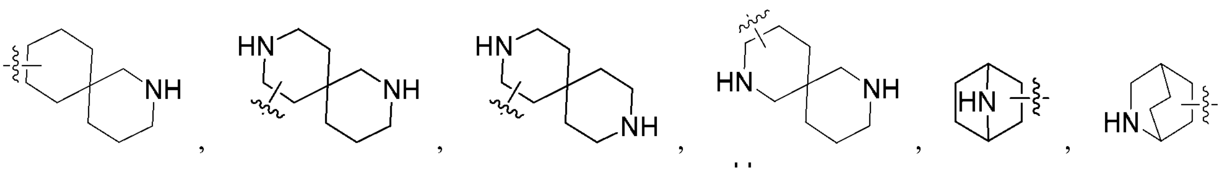 , , , , , ,

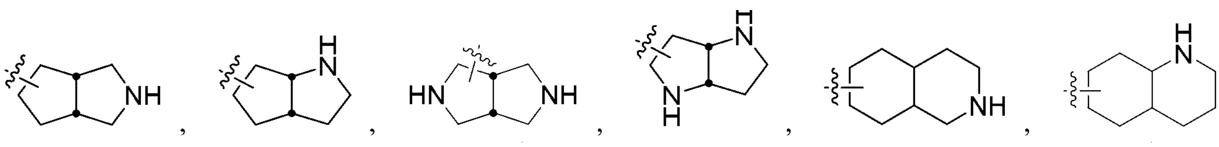 , , , , , ,

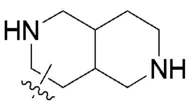, 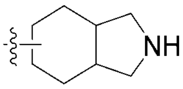, 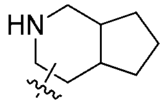, 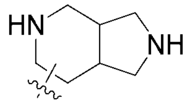, 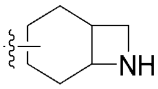, 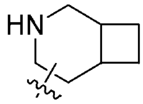,

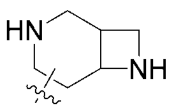, 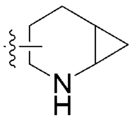, and 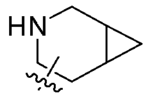.

In some embodiments of the present disclosure, $R_5$ and $R_6$ are each independently selected from H, methyl, ethyl, *n*-propyl, isopropyl, *n*-butyl, isobutyl, *tert*-butyl, cyclopropyl, cyclobutyl, cyclopentyl, cyclohexyl and phenyl, or $R_5$ and $R_6$, together with the carbon atom attached thereto, form cyclobutyl, cyclopentyl, cyclohexyl, azetidinyl, piperidinyl, piperazinyl, tetrahydropyranyl, pyrrolidinyl, tetrahydrofuranyl, or tetrahydrothienyl.

In some embodiments of the present disclosure, $R_7$ is selected from H and the following optionally substituted substituents: methyl, ethyl, n-propyl, isopropyl, n-butyl, isobutyl, tert-butyl, cyclopropyl, cyclobutyl, cyclopentyl, cyclohexyl, azetidinyl, piperidinyl, piperazinyl, tetrahydropyranyl, pyrrolidinyl, tetrahydrofuranyl, and tetrahydrothienyl, or -N($R_7$)($R_2$) forms

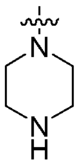, 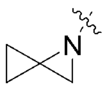, 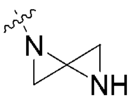, 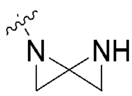, 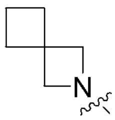, 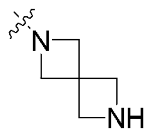,

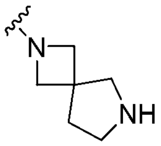, 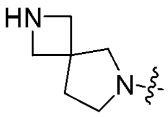, 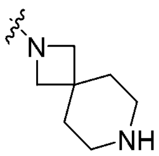, 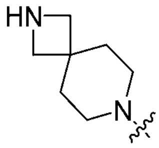, 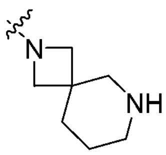, 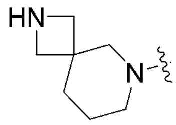,

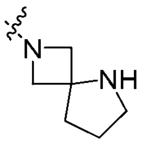, 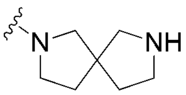, 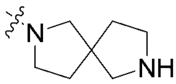, 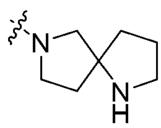, 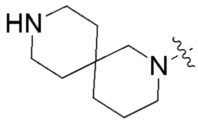, 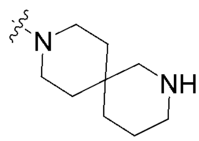,

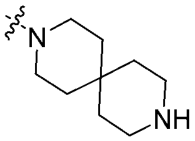, 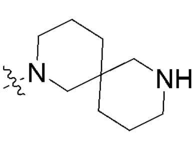, 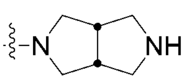, 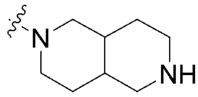, 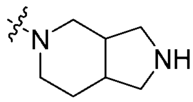,

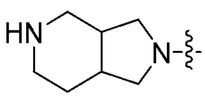, 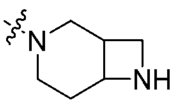, or 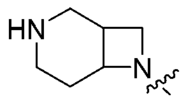.

In some embodiments of the present disclosure, the optionally substituted substituents are selected from OH, methyl, ethyl, *n*-propyl, isopropyl, *n*-butyl, isobutyl, *tert*-butyl, cyclopropyl, cyclobutyl, cyclopentyl, cyclohexyl, ($R_8$)($R_9$)N-, azetidinyl, piperidinyl, piperazinyl, tetrahydropyranyl, pyrrolidinyl, tetrahydrofuranyl, tetrahydrothienyl, tert-butoxycarbonyl (Boc), methoxycarbonyl, ethoxycarbonyl, propoxycarbonyl, benzyloxycarbonyl (Cbz), fluorenylmethoxycarbonyl (Fmoc), allyloxycarbonyl (Alloc), and a sulfonic acid group. In some embodiments of the present disclosure, $R_8$ and $R_9$ are each independently selected from H, methyl, ethyl, *n*-propyl, isopropyl, *n*-butyl, isobutyl, *tert*-butyl, cyclopropyl, cyclobutyl, cyclopentyl, cyclohexyl, azetidinyl, piperidinyl, piperazinyl, tetrahydropyranyl, pyrrolidinyl, tetrahydrofuranyl, tetrahydrothienyl, phenyl, and benzyl. In some embodiments of the present disclosure, $R_2$ is selected from H and the following optionally substituted substituents: methyl, ethyl, n-propyl, isopropyl, n-butyl, isobutyl, tert-butyl, cyclopropyl,

cyclobutyl, cyclopentyl, cyclohexyl, -methyl-$C(R_5)(R_6)$-methyl-, -methyl-$C(R_5)(R_6)$-ethyl-, -methyl-$C(R_5)(R_6)$-propyl-, -ethyl-$C(R_5)(R_6)$-ethyl-, -propyl-$C(R_5)(R_6)$-ethyl-, -propyl-$C(R_5)(R_6)$-propyl-, -$C(R_5)(R_6)$-methyl-, -$C(R_5)(R_6)$-ethyl-, -$C(R_5)(R_6)$-propyl-, -$C(R_5)(R_6)$-butyl-, -$C(R_5)(R_6)$-pentyl-, phenyl,

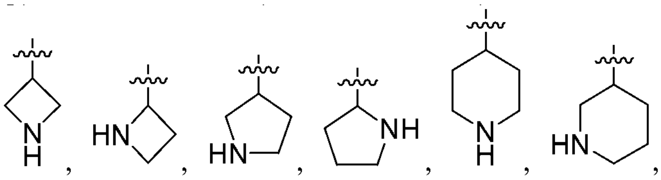

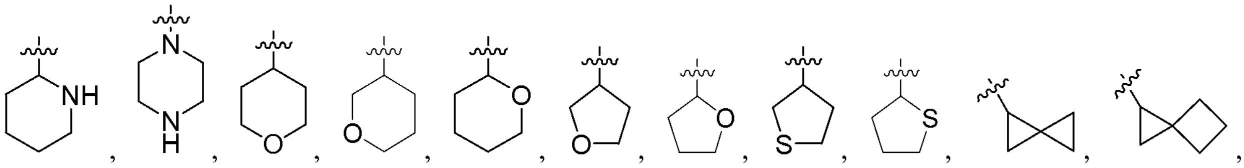

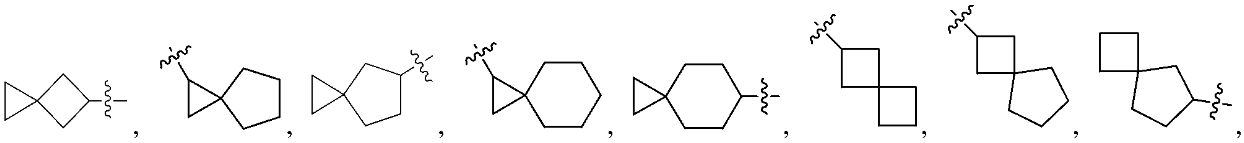

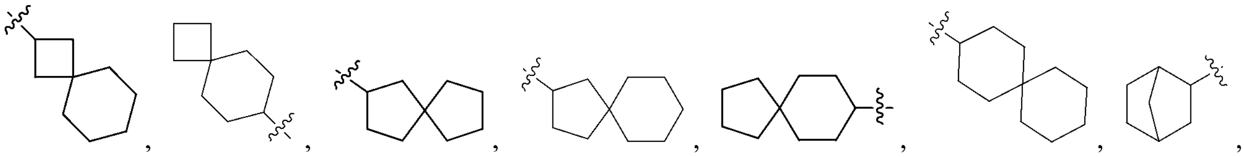

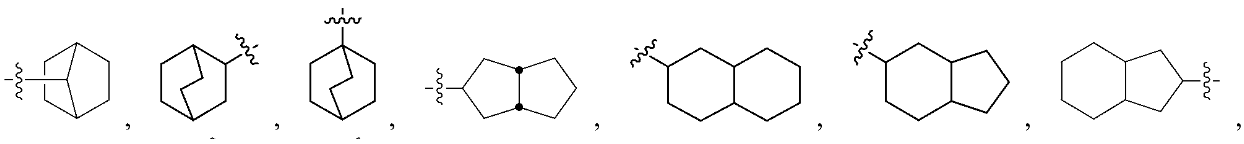

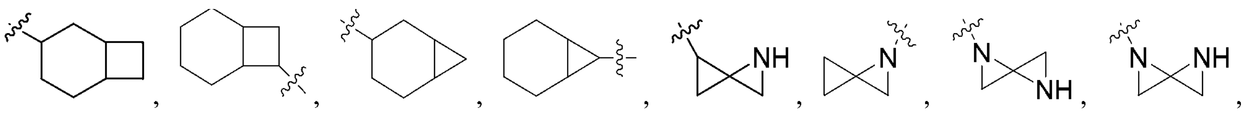

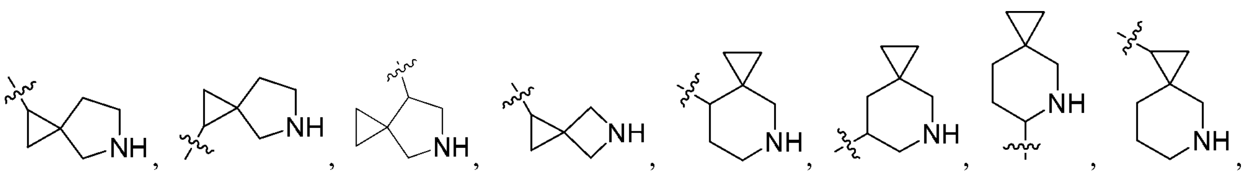

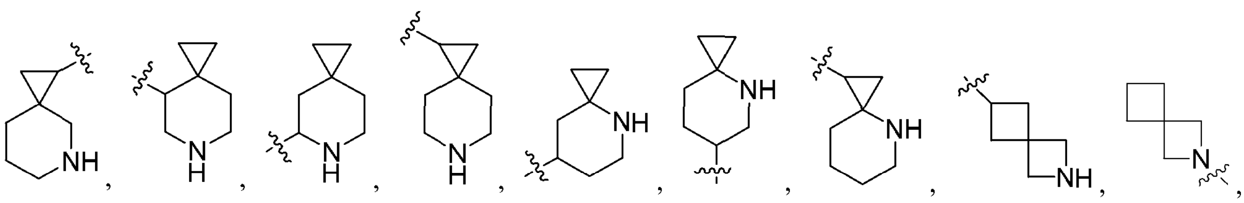

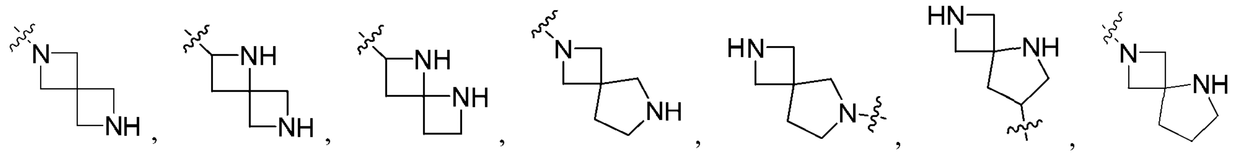, , , , , , ,

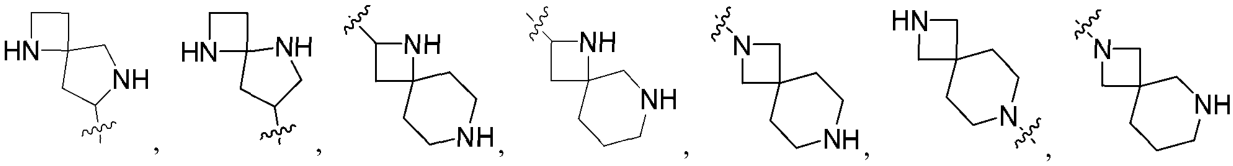, , , , , , ,

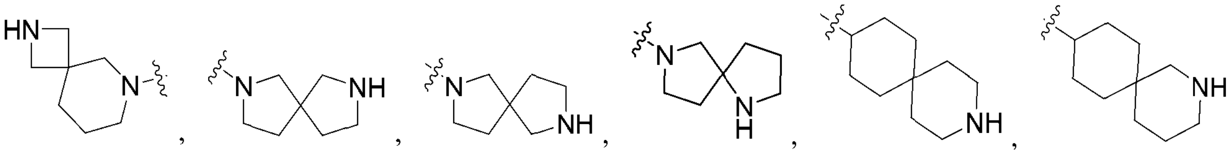, , , , , ,

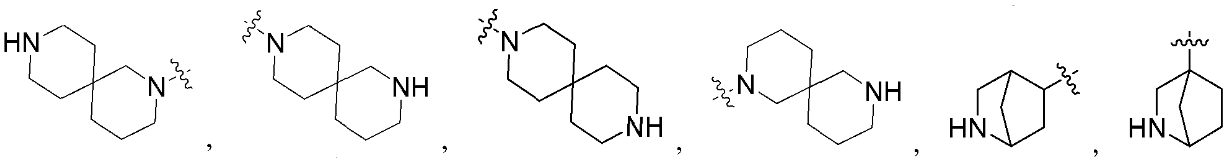, , , , , ,

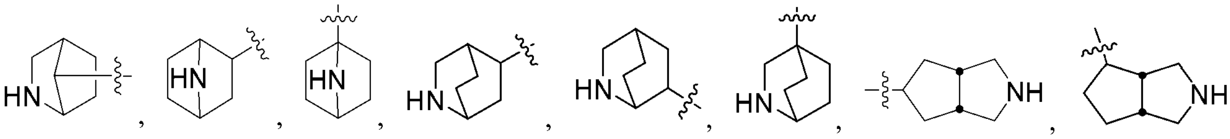, , , , , , , ,

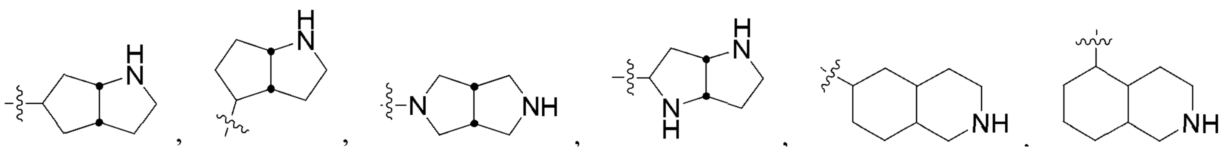, , , , , ,

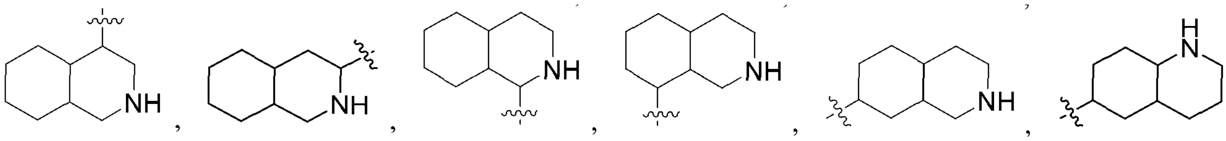, , , , , ,

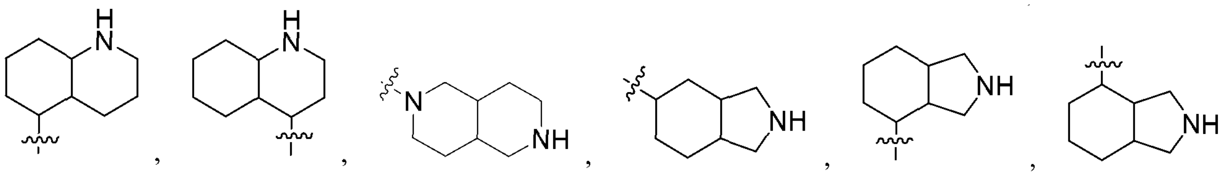, , , , , ,

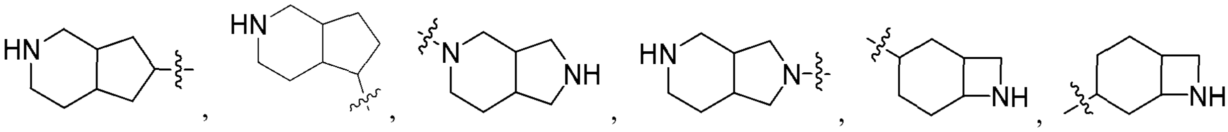, , , , , ,

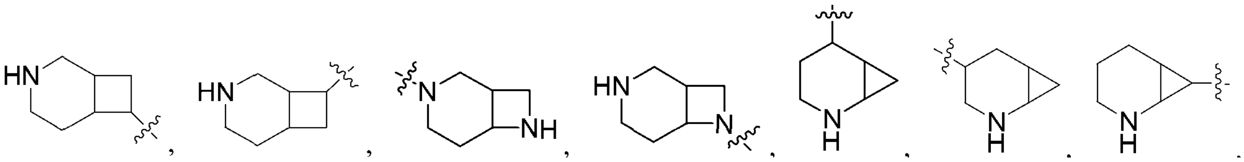

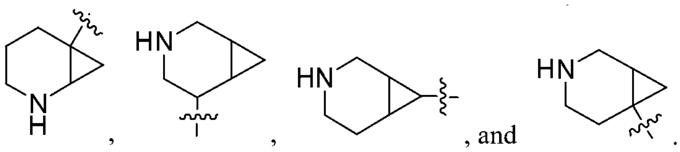

In some embodiments of the present disclosure, $R_2$ is selected from hydrogen, methyl, ethyl, n-propyl, isopropyl, $CH_3NH$-, cyclopropyl, cyclohexyl, phenyl,

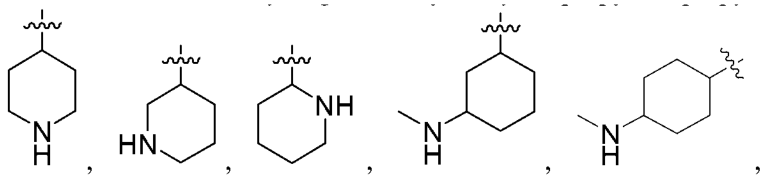

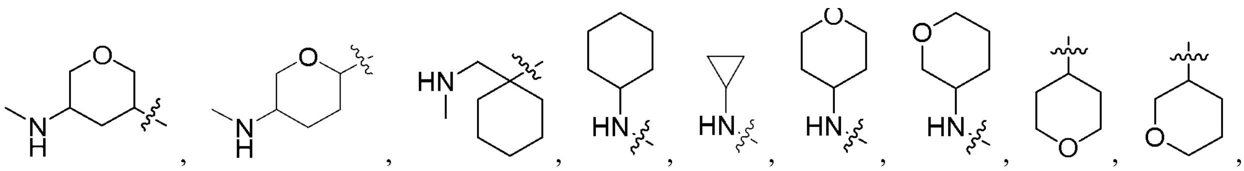

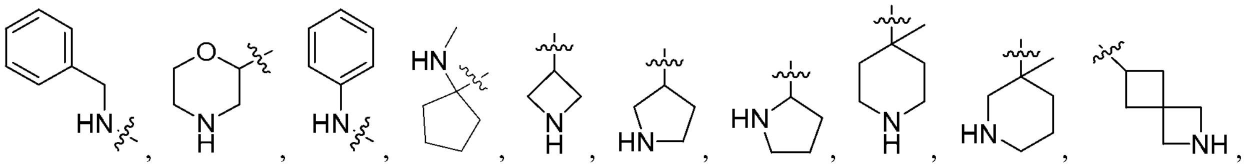

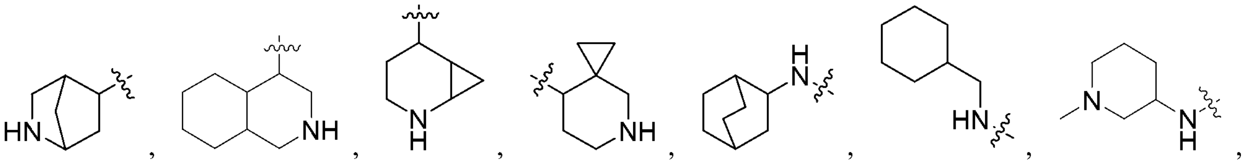

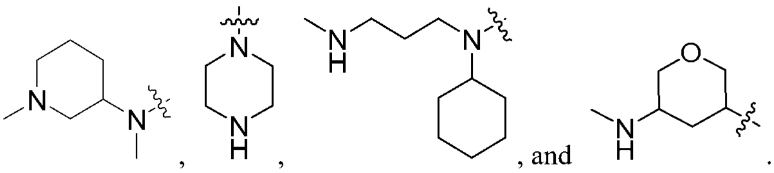

In some embodiments of the present disclosure, $R_2$ is selected from

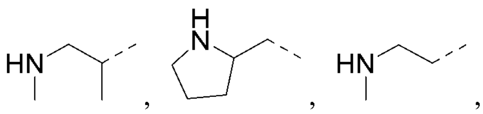

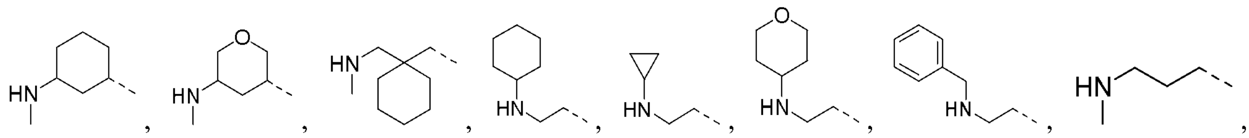

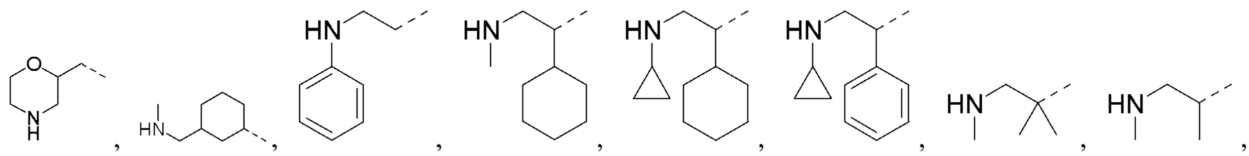

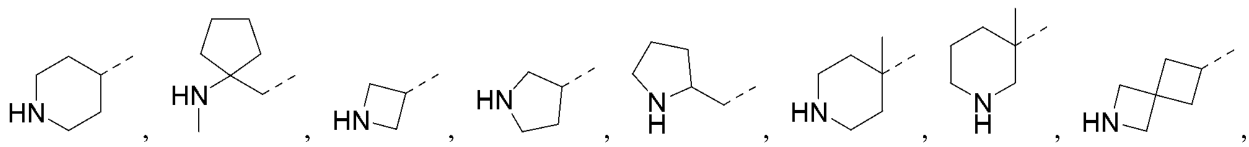

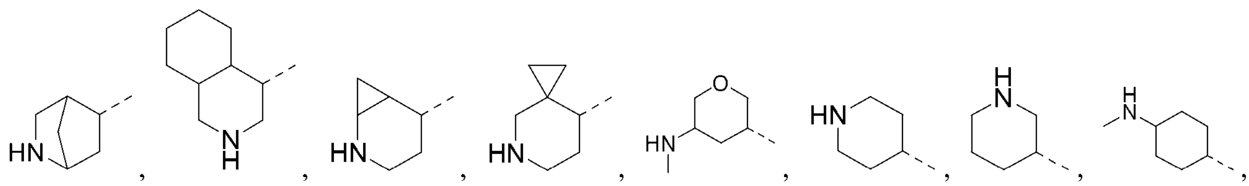

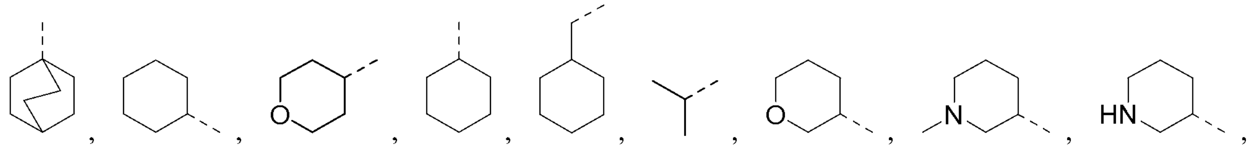

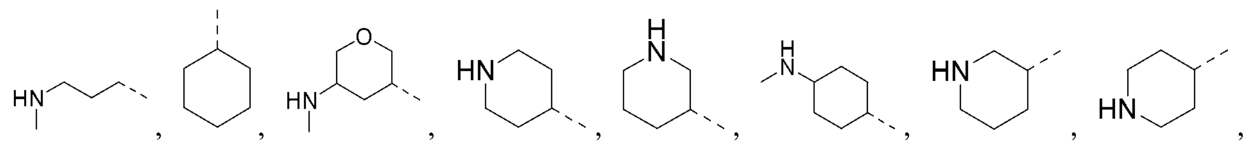

and

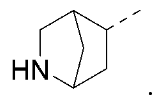

In some embodiments of the present disclosure, $L_1$ and $L_2$ are each independently selected from a bond, methylene, ethylene, n-propylene, isopropylene, n-butylene, isobutylene, tert-butylene, n-pentylene, isopentylene, neopentylene, *n*-hexylene, 2-methylpentylene, 3-methylpentylene,

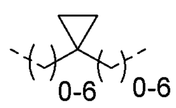 , 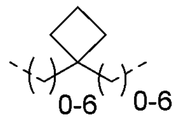 ,

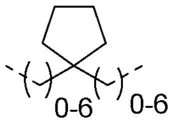, 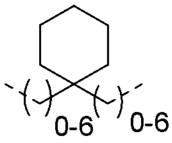, 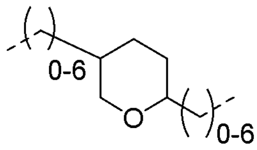, and 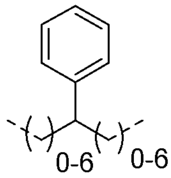.

In some embodiments of the present disclosure, $L_1$ and $L_2$ are each independently selected from a bond, methylene, ethylene, n-propylene, isopropylene, n-butylene, isobutylene, tert-butylene, n-pentylene, isopentylene, neopentylene, n-hexylene, 2-methylpentylene, 3-methylpentylene,

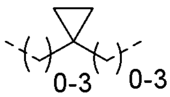, 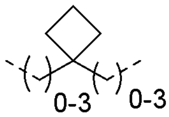,

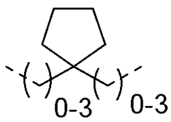, 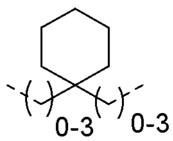, 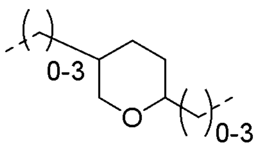, and 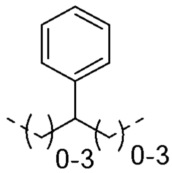.

In some embodiments of the present disclosure, $L_1$ and $L_2$ are each independently selected from a bond,

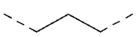, 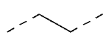, 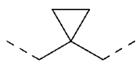, 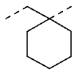, 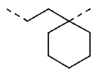, 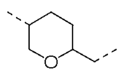, 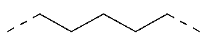, 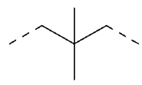,

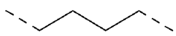, 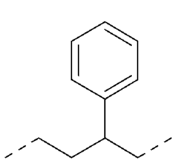, 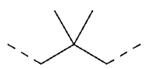, and 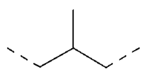.

In the present disclosure,

“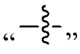” and “- - -”

have the same meaning and represent a linking bond.

In some embodiments of the present disclosure, the compound having the structure represented by formula (I-1) has a structure represented by formula (I-2):

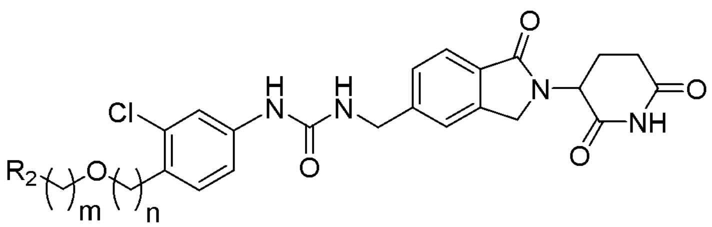

I-2

wherein $R_2$ is defined as in formula (I-1); and m and n are each independently selected from an integer of 0-6, such as 0, 1, 2, 3, 4, 5, or 6.

In some embodiments of the present disclosure, $R_2$ is selected from optionally substituted 3- to 16-membered

heterocyclyl, wherein heteroatoms of the heterocyclyl are selected from nitrogen.

In some embodiments of the present disclosure, $R_2$ is selected from the following optionally substituted substituents: 3- to 8-membered monocyclic heterocyclyl, 6- to 14-membered spiro heterocyclyl, 5- to 14-membered fused heterocyclyl, and 5- to 14-membered bridged heterocyclyl, wherein a heteroatom of the heterocyclyl is selected from nitrogen.

In some embodiments of the present disclosure, $R_2$ is selected from optionally substituted 5- to 6-membered monocyclic heterocyclyl.

In some embodiments of the present disclosure, $R_2$ is selected from the following optionally substituted substituents: azetidinyl, pyrrolidinyl, piperidinyl,

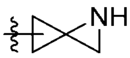, 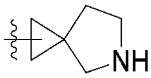, 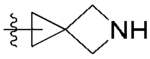, 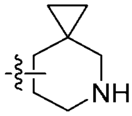, 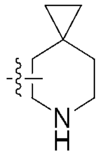,

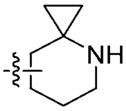, 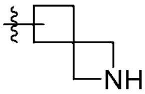, 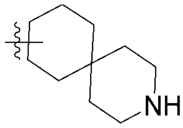, 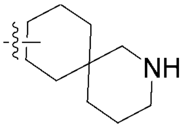, 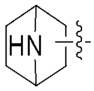, 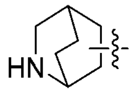, 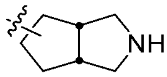,

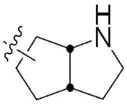, 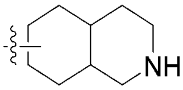, 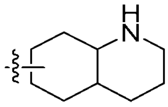, 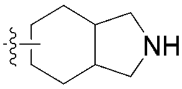, 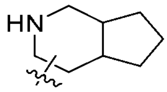, 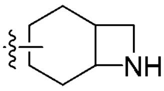,

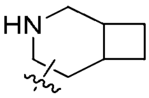, 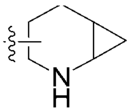, and 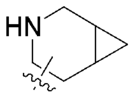.

In some embodiments of the present disclosure, $R_2$ is selected from the following optionally substituted substituents:

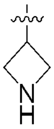, 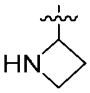, 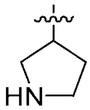, 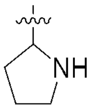, 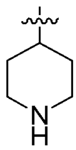, 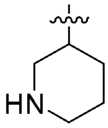, 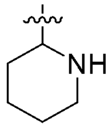, 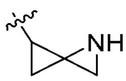, 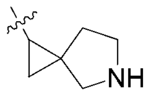,

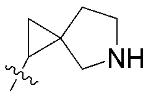, 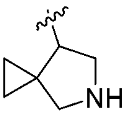, 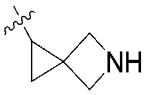, 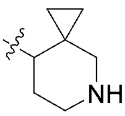, 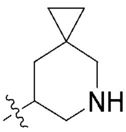, 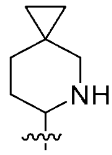, 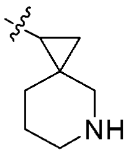, 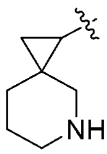,

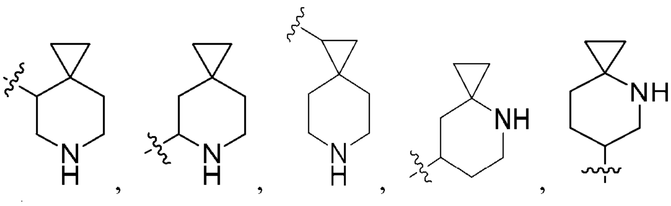, , , , 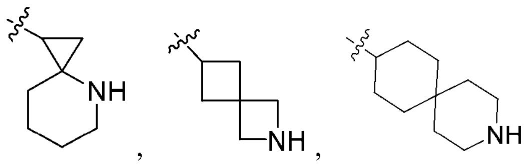, , ,

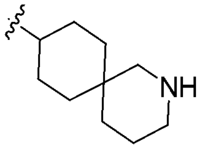, 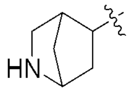, 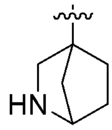, 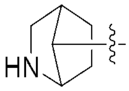, 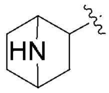, 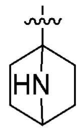, 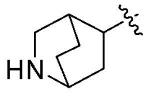, 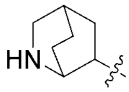,

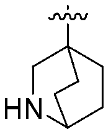, 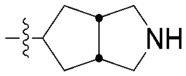, 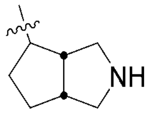, 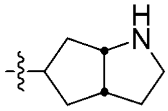, 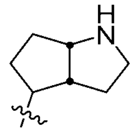, 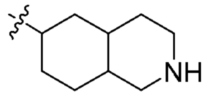,

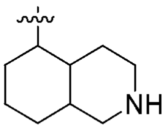, 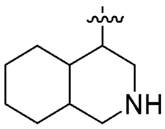, 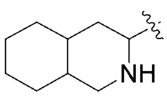, 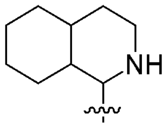, 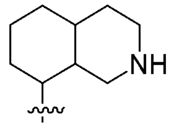, 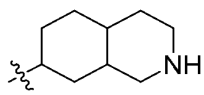,

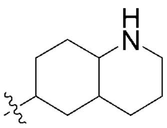, 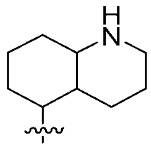, 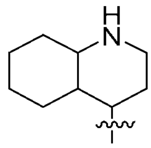, 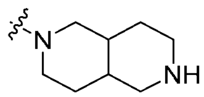, 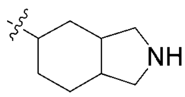, 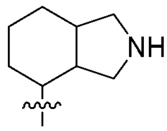,

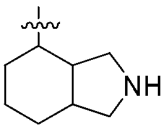, 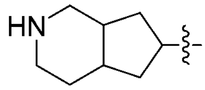, 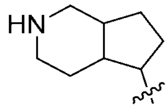, 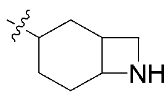, 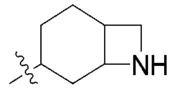, 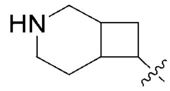,

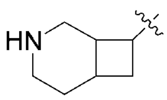, 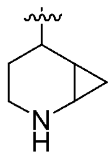, 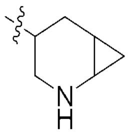, 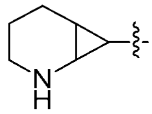, 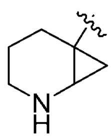, 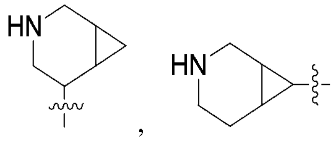, , and

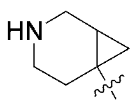.

In some embodiments of the present disclosure, the optionally substituted substituent is selected from H, $C_{1-3}$ alkyl (such as methyl, ethyl, n-propyl, and isopropyl), $C_{1-6}$ alkyloxycarbonyl (such as $C_{1-3}$ alkyloxycarbonyl, *tert*-butoxycarbonyl (Boc), methoxycarbonyl, ethoxycarbonyl, and propoxycarbonyl), benzyloxycarbonyl (Cbz), fluorenylmethoxycarbonyl (Fmoc), and allyloxycarbonyl (Alloc).

In some embodiments of the present disclosure, the compound having the structure represented by formula (I-1) has a structure represented by formula (I-3):

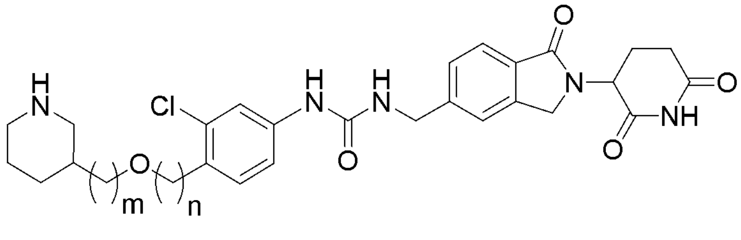

I-3

wherein m and n are each independently selected from an integer of 0-6, such as 0, 1, 2, 3, 4, 5, or 6.

In some embodiments of the present disclosure, the compound having the structure represented by formula (I-1) has a structure represented by formula (I-4):

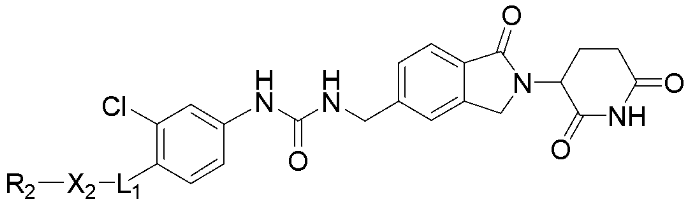

I-4

wherein $L_1$, $X_2$, and $R_2$ are defined as in formula (I-1).

In some embodiments of the present disclosure, $L_1$ is selected from

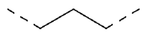, 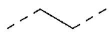, 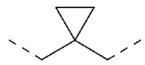,

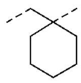, 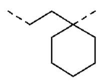, and 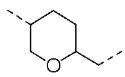.

In some embodiments of the present disclosure, $R_2$ is selected from the following optionally substituted substituents:

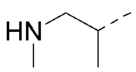, 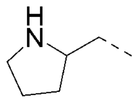, 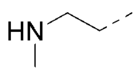, 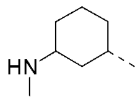, 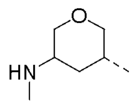, 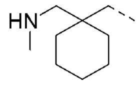, 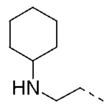, 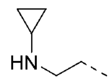,

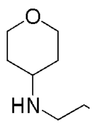, 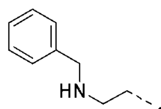, 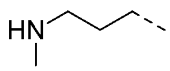, 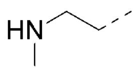, 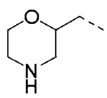, 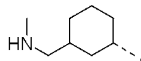, 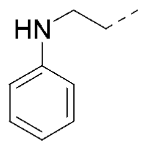, 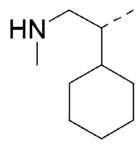,

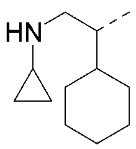, 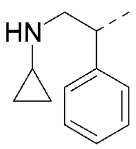, 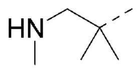, 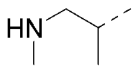, 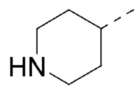, 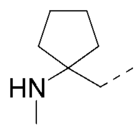, 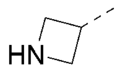, 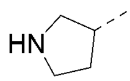,

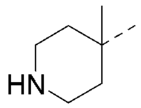, 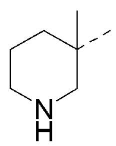, 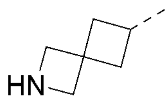, 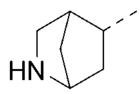, 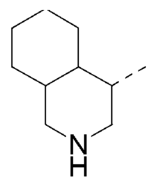, 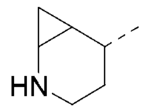, and 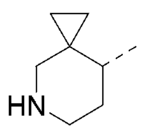.

In some embodiments of the present disclosure, the optionally substituted substituent is selected from $C_{1-6}$ alkyloxycarbonyl (such as $C_{1-3}$ alkyloxycarbonyl, tert-butoxycarbonyl (Boc), methoxycarbonyl, ethoxycarbonyl, and propoxycarbonyl), benzyloxycarbonyl (Cbz), fluorenylmethoxycarbonyl (Fmoc), and allyloxycarbonyl (Alloc). In some embodiments of the present disclosure, $X_2$ is selected from -O- and -S-.

In some embodiments of the present disclosure, the compound having the structure represented by formula (I-1) has a structure represented by formula (I-5):

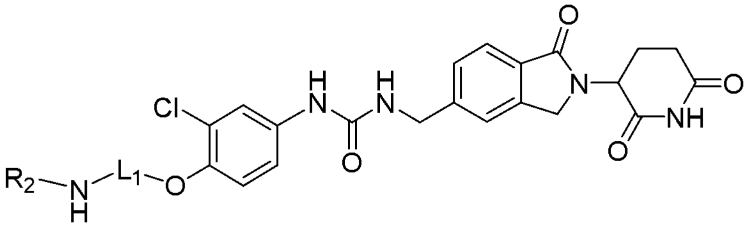

I-5

wherein $L_1$ and $R_2$ are defined as in formula (I-1).

In some embodiments of the present disclosure, $L_1$ is selected from

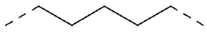, 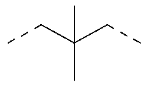,

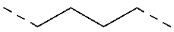, 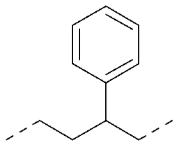, 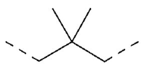, and 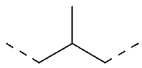.

In some embodiments of the present disclosure, $R_2$ is selected from

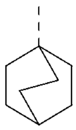, 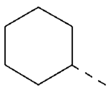, 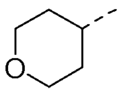, 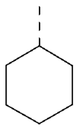,

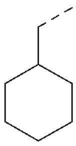, and 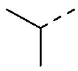.

In some embodiments of the present disclosure, the compound having the structure represented by formula (I-1) has a structure represented by formula (I-6):

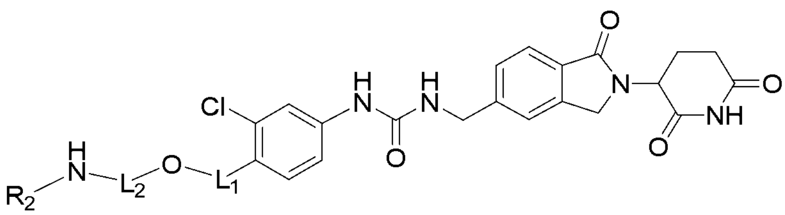

I-6

wherein $L_1$, $L_2$, and $R_2$ are defined as in formula (I-1).

In some embodiments of the present disclosure, $L_1$ and $L_2$ are each independently selected from

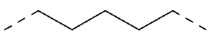,

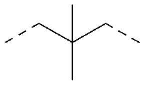, 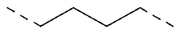, 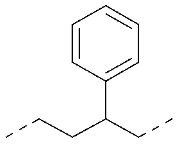, 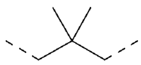, and 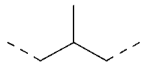.

In some embodiments of the present disclosure, $R_2$ is selected from

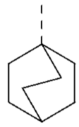, 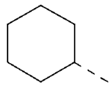, 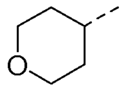, 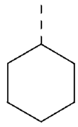,

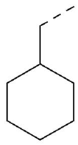, and 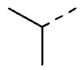.

In some embodiments of the present disclosure, $L_1$ is selected from methylene.

In some embodiments of the present disclosure, the compound having the structure represented by formula (I-1) has a structure represented by formula (I-7):

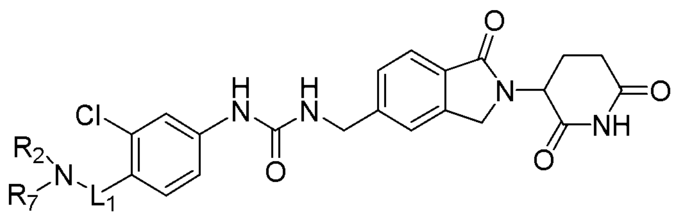

I-7

wherein $L_1$, $R_2$, and $R_7$ are defined as in formula (I-1).

In some embodiments of the present disclosure, $L_1$ is selected from -$C_{1-6}$ alkyl-.

In some embodiments of the present disclosure, $L_1$ is selected from methylene, ethylene, n-propylene, n-butylene, n-pentylene, and n-hexylene.

In some embodiments of the present disclosure, $R_2$ is selected from

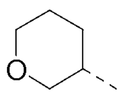, 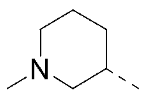, 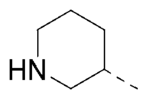, and

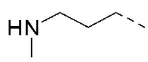.

In some embodiments of the present disclosure, $R_7$ is selected from H, methyl, and

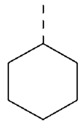.

In some embodiments of the present disclosure, $R_2$ and $R_7$, together with the N attached thereto, form

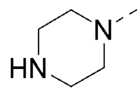.

In some embodiments of the present disclosure, the compound having the structure represented by formula (I-1) has a structure represented by formula (I-8):

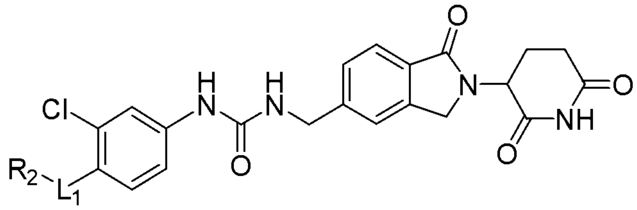

I-8

wherein $L_1$ and $R_2$ are defined as in formula (I-1).

In some embodiments of the present disclosure, $L_1$ is selected from $-C_{1-6}$ alkyl-.

In some embodiments of the present disclosure, $L_1$ is selected from methylene, ethylene, n-propylene, n-butylene, *n*-pentylene, and *n*-hexylene.

In some embodiments of the present disclosure, $R_2$ is selected from the following optionally substituted substituents:

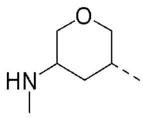, 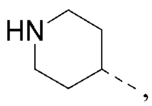, 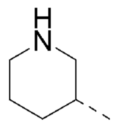, and 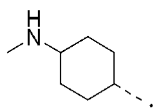.

In some embodiments of the present disclosure, the optionally substituted substituent is selected from $C_{1-6}$ alkyloxycarbonyl (such as $C_{1-3}$ alkyloxycarbonyl, tert-butoxycarbonyl (Boc), methoxycarbonyl, ethoxycarbonyl, and propoxycarbonyl), benzyloxycarbonyl (Cbz), fluorenylmethoxycarbonyl (Fmoc), and allyloxycarbonyl (Alloc). In some embodiments of the present disclosure, the compound having the structure represented by formula (I-1) has a structure represented by formula (I-9):

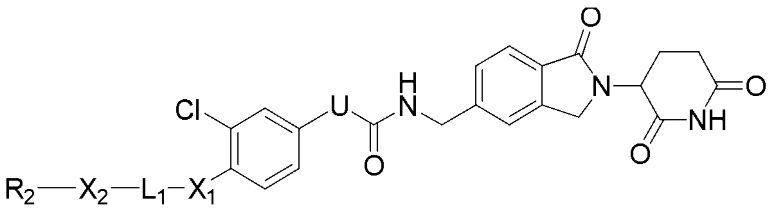

I-9

wherein $X_1$, $X_2$, $L_1$, $R_2$, and U are defined as in formula (I-1).

In some embodiments of the present disclosure, $X_1$ and $X_2$ are selected from -O-.

In some embodiments of the present disclosure, $L_1$ is selected from $-C_{1-6}$ alkyl-.

In some embodiments of the present disclosure, $L_1$ is selected from methylene, ethylene, n-propylene, n-butylene, n-pentylene, and n-hexylene.

In some embodiments of the present disclosure, $R_2$ is selected from optionally substituted piperidinyl.

In some embodiments of the present disclosure, $R_2$ is selected from the following optionally substituted substituents:

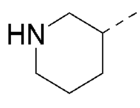, 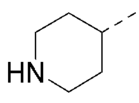, and 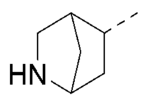.

In some embodiments of the present disclosure, the optionally substituted substituent is selected from $C_{1-6}$

alkyloxycarbonyl (such as $C_{1-3}$ alkyloxycarbonyl, *tert*-butoxycarbonyl (Boc), methoxycarbonyl, ethoxycarbonyl, and propoxycarbonyl), benzyloxycarbonyl (Cbz), fluorenylmethoxycarbonyl (Fmoc), and allyloxycarbonyl (Alloc). In some embodiments of the present disclosure, the compound having the structure represented by formula (I-1) has a structure represented by formula (I-10):

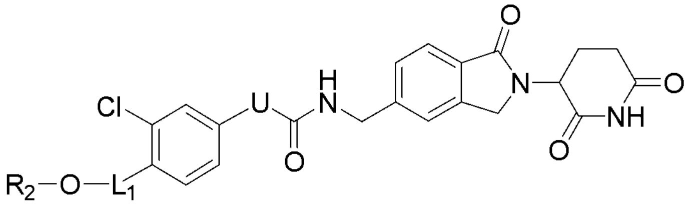

I-10

wherein $L_1$, $R_2$, and U are defined as in formula (I-1).

In some embodiments of the present disclosure, U is selected from -NH-.

In some embodiments of the present disclosure, $L_1$ is selected from $-C_{1-6}$ alkyl-.

In some embodiments of the present disclosure, $L_1$ is selected from methylene, ethylene, n-propylene, n-butylene, n-pentylene, and n-hexylene.

In some embodiments of the present disclosure, $R_2$ is selected from the following optionally substituted substituents: azetidinyl, pyrrolidinyl, piperidinyl,

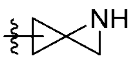 , 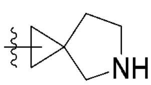 , 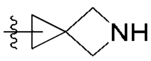 , 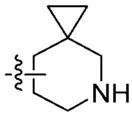 , 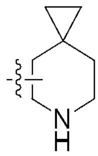 ,

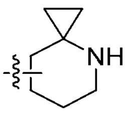 , 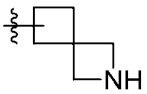 , 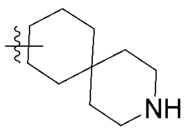 , 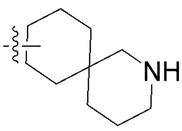 , 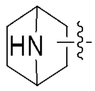 , 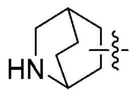 , 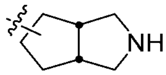 ,

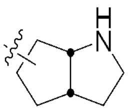 , 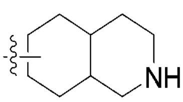 , 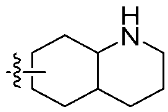 , 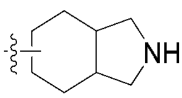 , 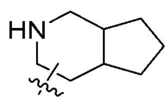 , 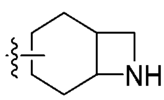 ,

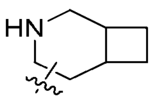 , 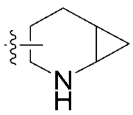 , and 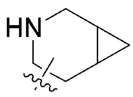 .

In some embodiments of the present disclosure, $R_2$ is selected from the following optionally substituted substituents:

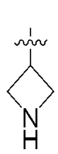 , 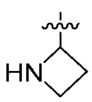 , 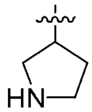 , 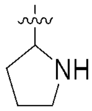 , 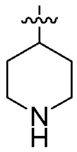 , 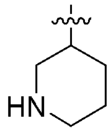 , 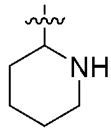 , 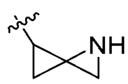 , 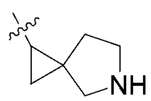 ,

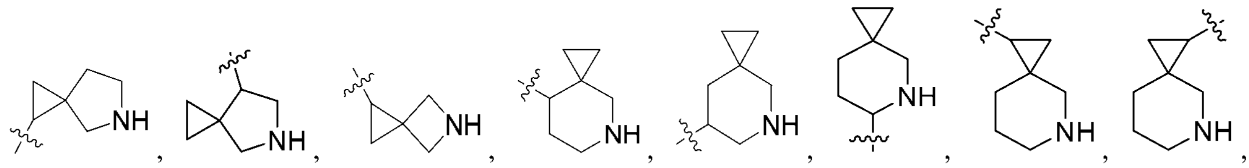

, , , , , , , ,

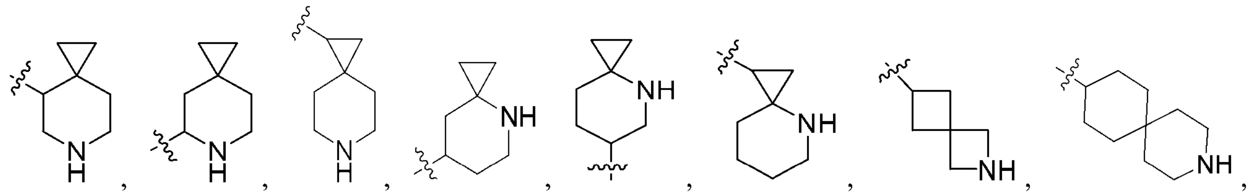

, , , , , , , ,

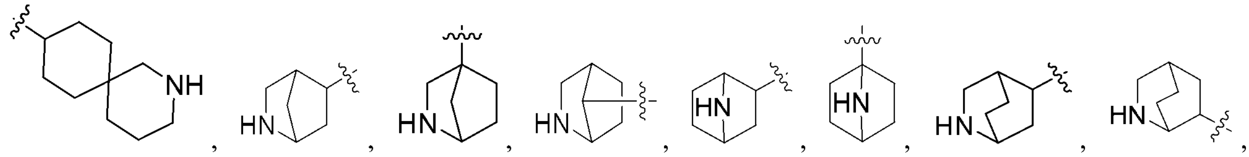

, , , , , , , ,

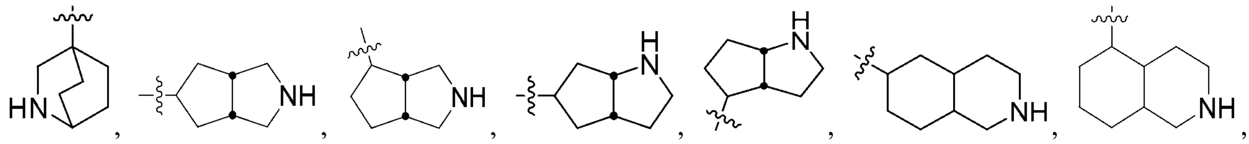

, , , , , , , ,

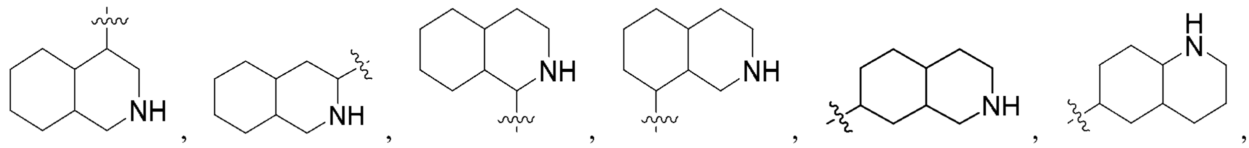

, , , , , ,

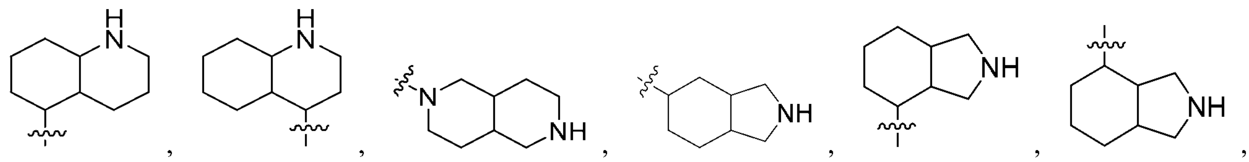

, , , , , ,

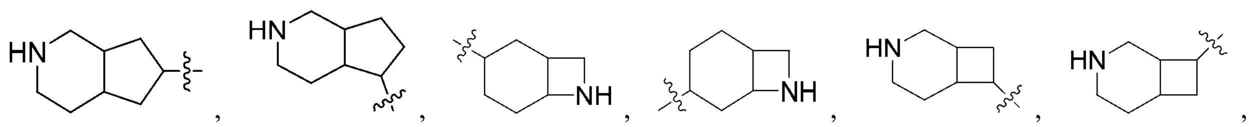

, , , , , ,

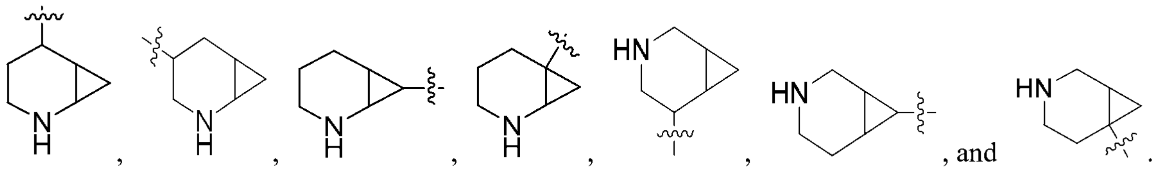

, , , , , , and .

In some embodiments of the present disclosure, the optionally substituted substituent is selected from H, $C_{1-3}$ alkyl (such as methyl, ethyl, *n*-propyl, and isopropyl), $C_{1-6}$ alkyloxycarbonyl (such as $C_{1-3}$ alkyloxycarbonyl, *tert*-butoxycarbonyl (Boc), methoxycarbonyl, ethoxycarbonyl, and propoxycarbonyl), benzyloxycarbonyl (Cbz), fluorenylmethoxycarbonyl (Fmoc), and allyloxycarbonyl (Alloc).

In some embodiments of the present disclosure, the compound having the structure represented by formula (I-1) has a structure represented by formula (I-11): wherein:

$R_{10}$ is selected from $C_{1-6}$ alkyloxycarbonyl (such as $C_{1-3}$ alkyloxycarbonyl, tert-butoxycarbonyl (Boc), methoxycarbonyl, ethoxycarbonyl, and propoxycarbonyl), benzyloxycarbonyl (Cbz), fluorenylmethoxycarbonyl (Fmoc), and allyloxycarbonyl (Alloc);

m and n are each independently selected from an integer of 0-6, such as 0, 1, 2, 3, 4, 5, or 6.

In some embodiments of the present disclosure, the compound having the structure represented by formula (I-1) has a structure represented by formula (I-12):

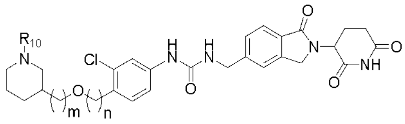

I-12a

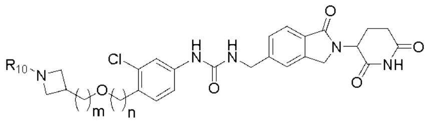

I-12b

wherein:

$R_{10}$ is selected from $C_{1-6}$ alkyloxycarbonyl (such as $C_{1-3}$ alkyloxycarbonyl, tert-butoxycarbonyl (Boc), methoxycarbonyl, ethoxycarbonyl, and propoxycarbonyl), benzyloxycarbonyl (Cbz), fluorenylmethoxycarbonyl (Fmoc), and allyloxycarbonyl (Alloc);

m and n are each independently selected from an integer of 0-6, such as 0, 1, 2, 3, 4, 5, or 6.

In the present disclosure, "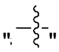" and "---" have the same meaning and represent a linking bond.

In some embodiments of the present disclosure, provided are compounds having the following structures, or tautomers, mesomers, racemates, enantiomers or diastereoisomers thereof, and pharmaceutically acceptable salts, hydrates or solvates thereof:

| | |
|---|---|
| 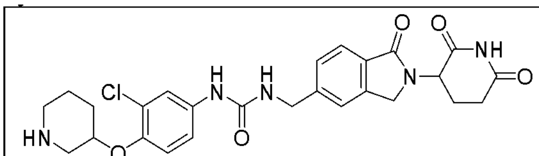 **B1** | 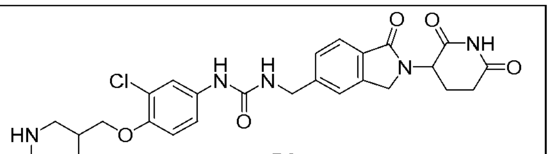 **B2** |
| 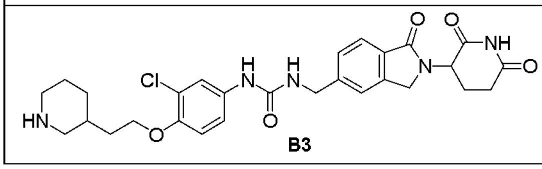 **B3** | 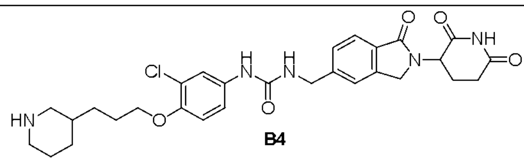 **B4** |

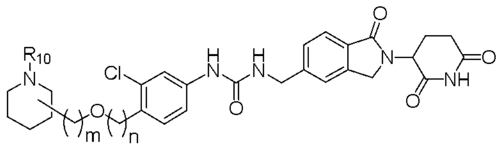

I-11a

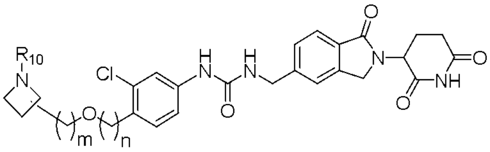

I-11b

| | |
|---|---|
| 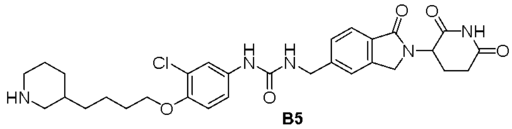 B5 | 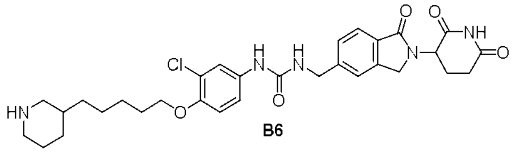 B6 |
| 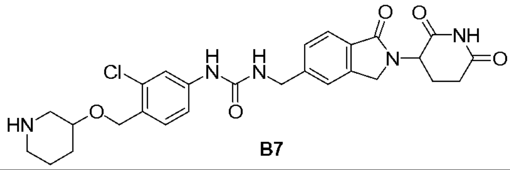 B7 | 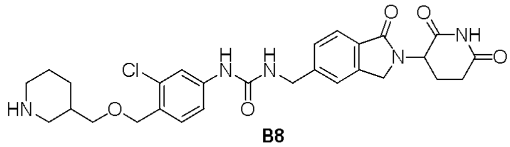 B8 |
| 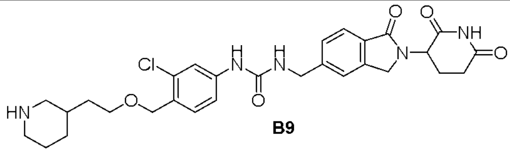 B9 | 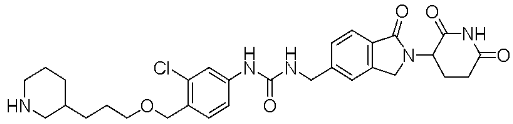 B10 |
| 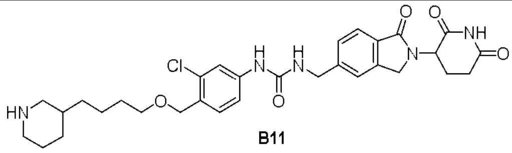 B11 | 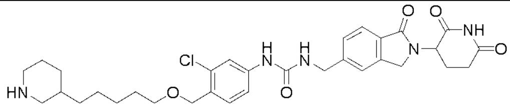 B12 |
| 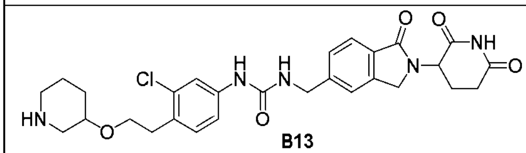 B13 | 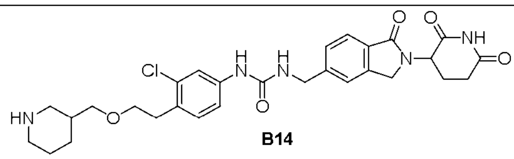 B14 |
| 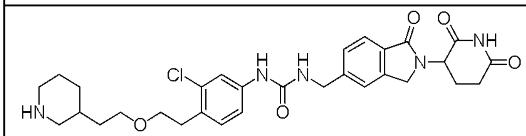 B15 | 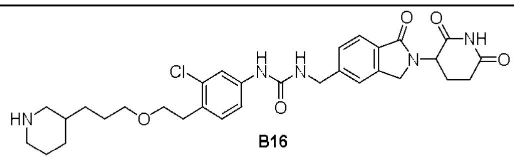 B16 |
| 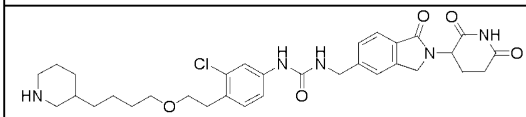 B17 | 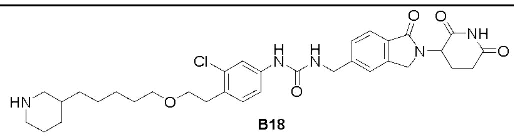 B18 |
| 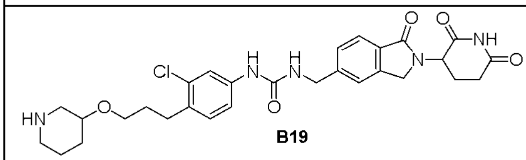 B19 | 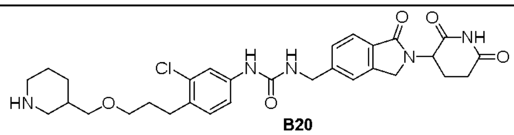 B20 |
| 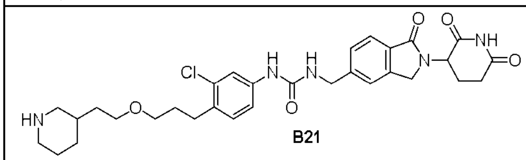 B21 | 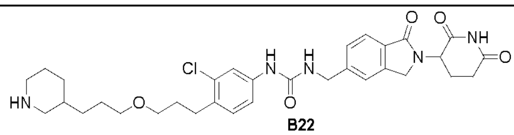 B22 |
| 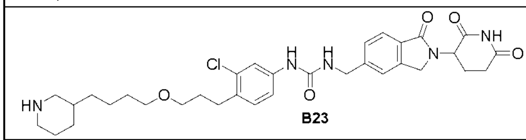 B23 | 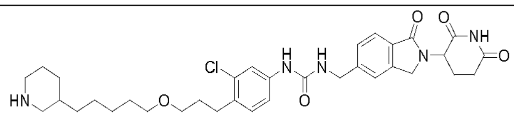 B24 |
| 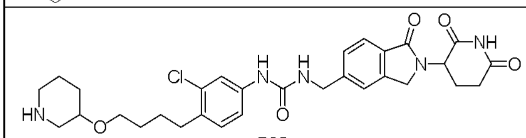 B25 | 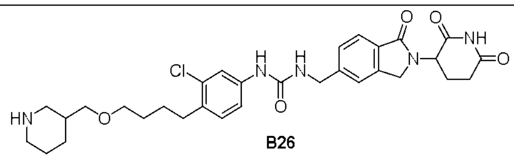 B26 |
| 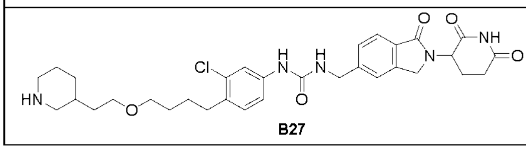 B27 | 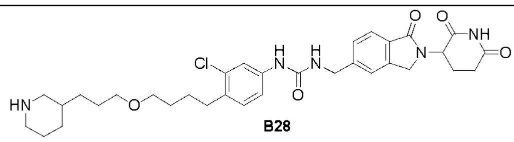 B28 |

| | |
|---|---|
| 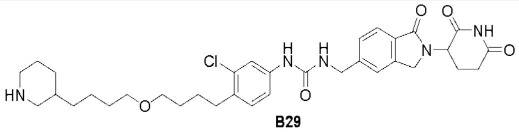 B29 | 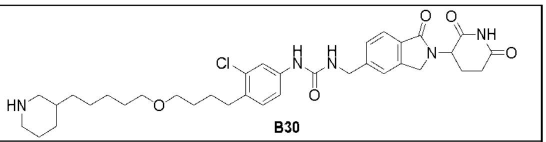 B30 |
| 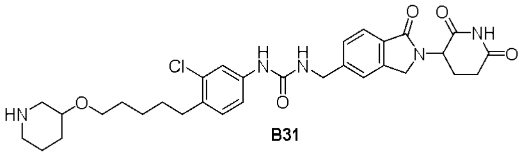 B31 | 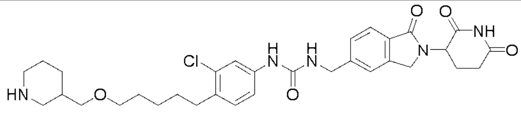 B32 |
| 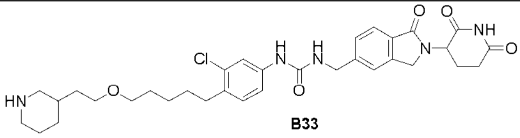 B33 | 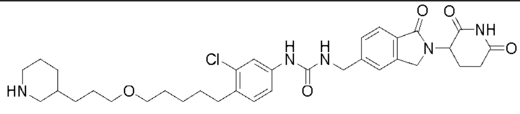 B34 |
| 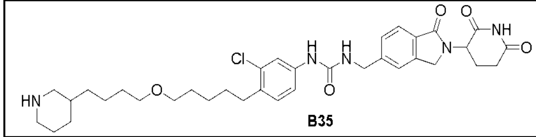 B35 | 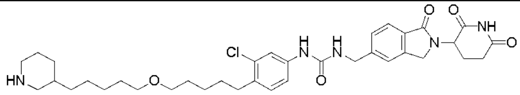 B36 |
| 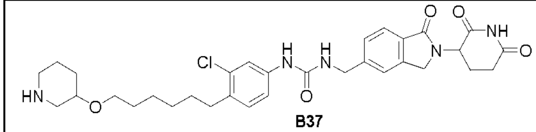 B37 | 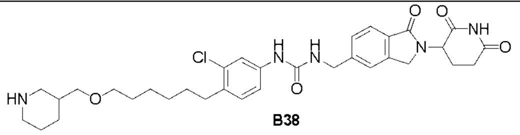 B38 |
| 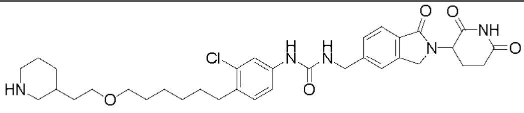 B39 | 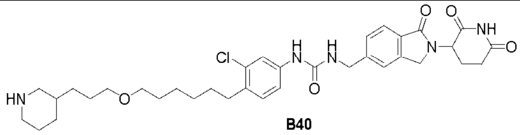 B40 |
| 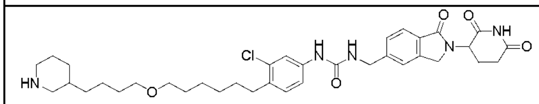 B41 | 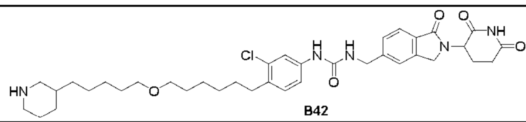 B42 |
| 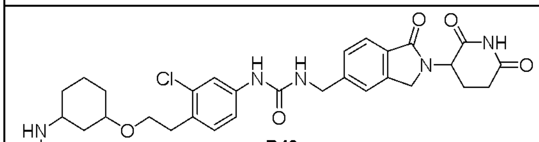 B43 | 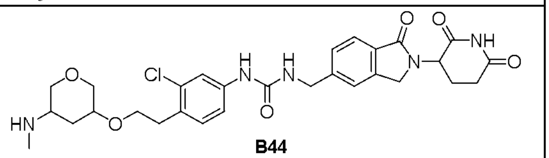 B44 |
| 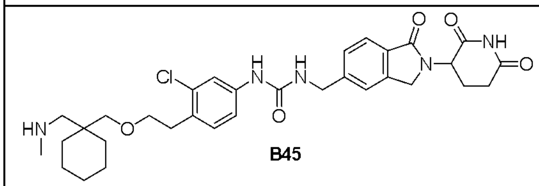 B45 | 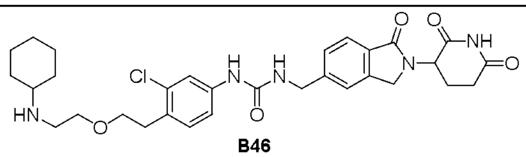 B46 |
| 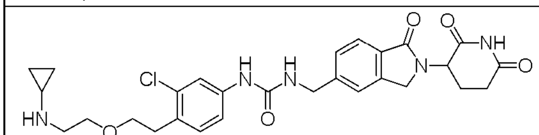 B47 | 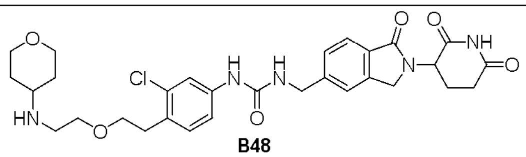 B48 |
| 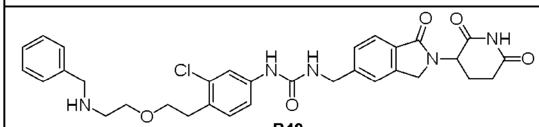 B49 | 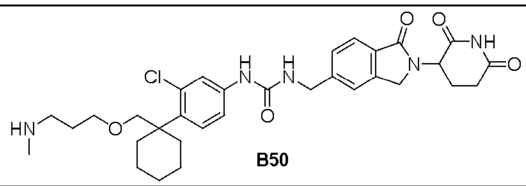 B50 |
| 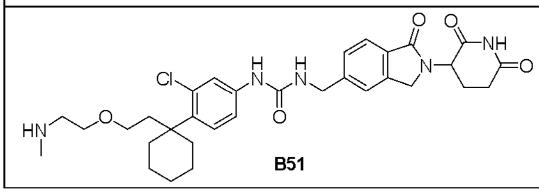 B51 | 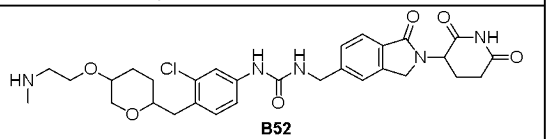 B52 |

| | |
|---|---|
| 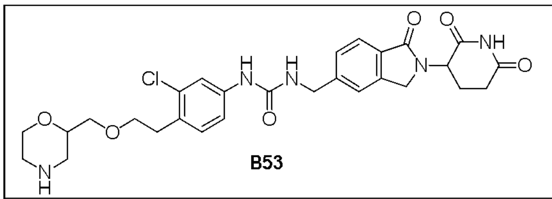 B53 | 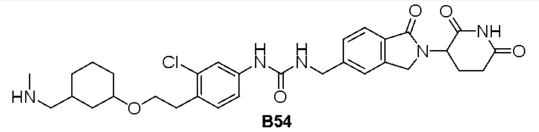 B54 |
| 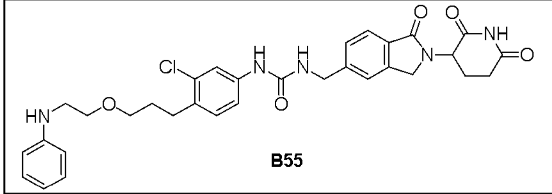 B55 | 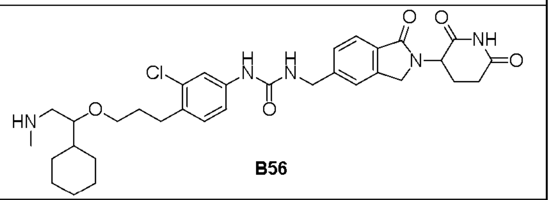 B56 |
| 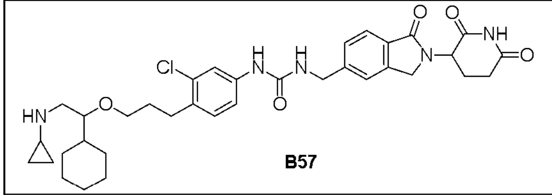 B57 | 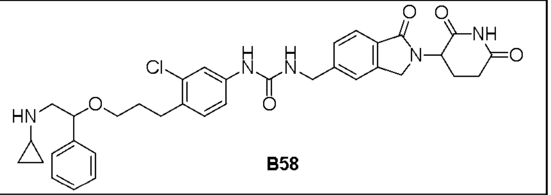 B58 |
| 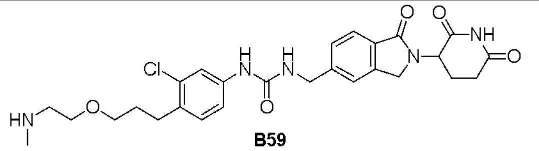 B59 | 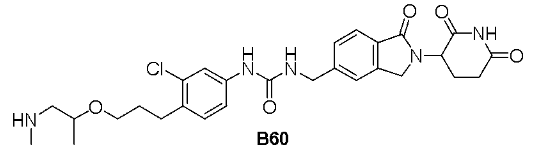 B60 |
| 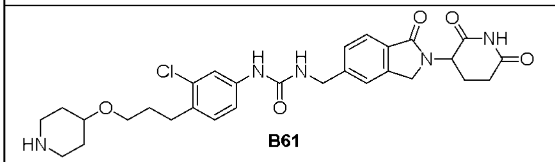 B61 | 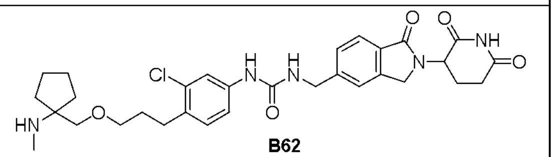 B62 |
| 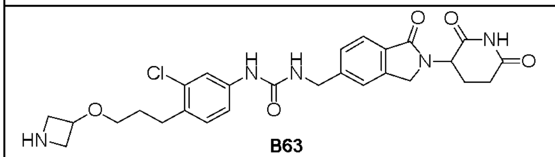 B63 | 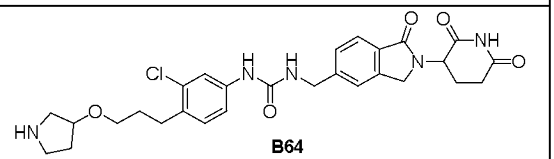 B64 |
| 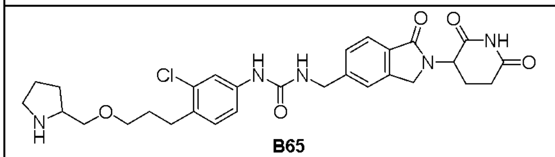 B65 | 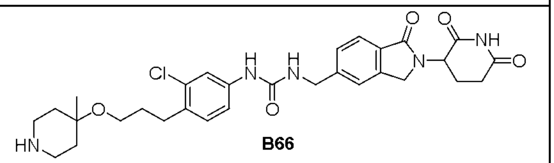 B66 |
| 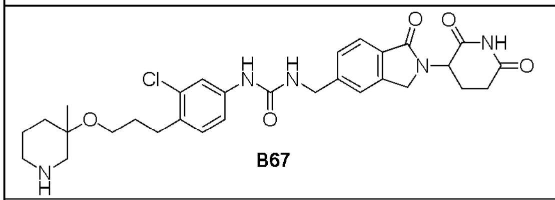 B67 | 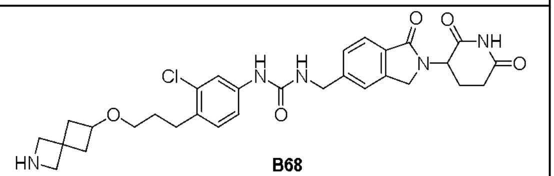 B68 |
| 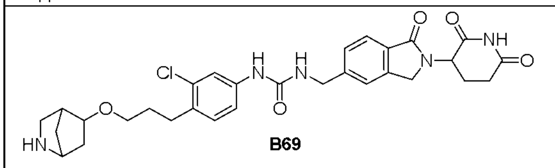 B69 | 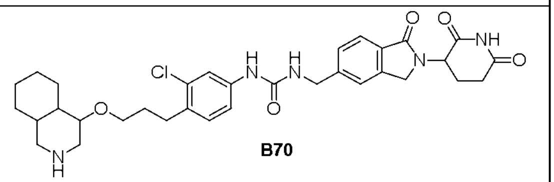 B70 |
| 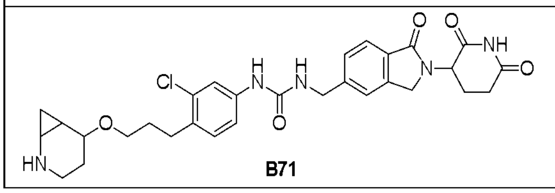 B71 | 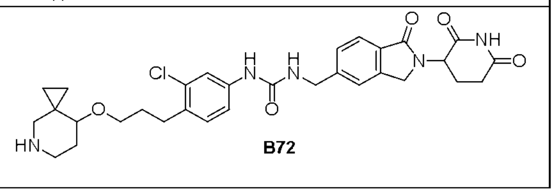 B72 |

| | |
|---|---|
| 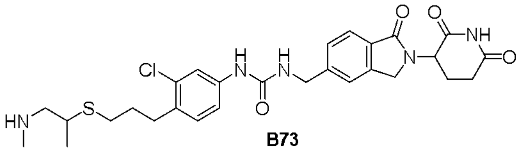 B73 | 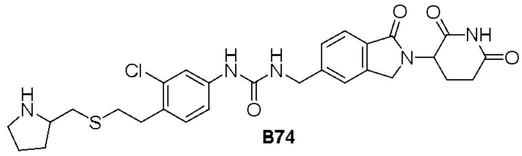 B74 |
| 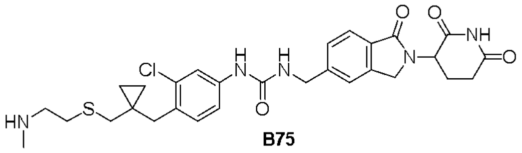 B75 | 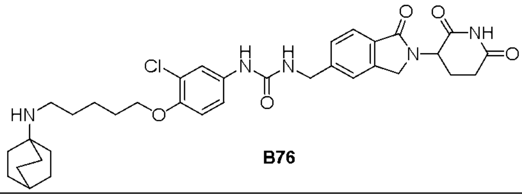 B76 |
| 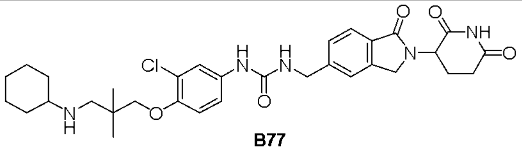 B77 | 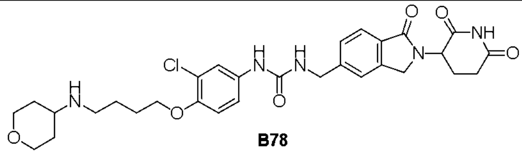 B78 |
| 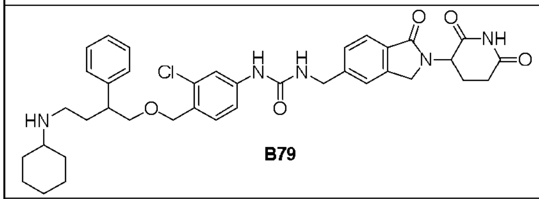 B79 | 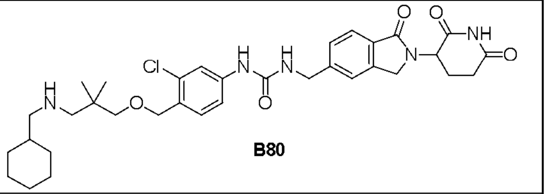 B80 |
| 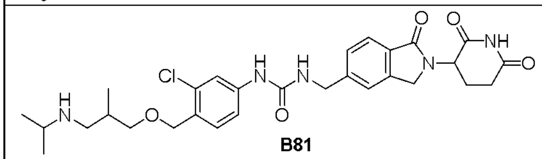 B81 | 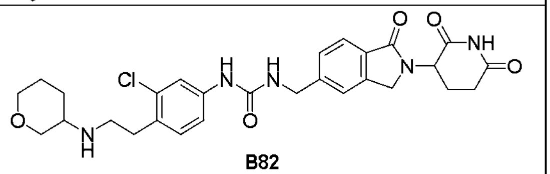 B82 |
| 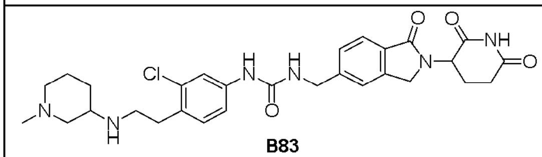 B83 | 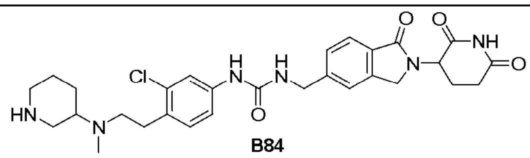 B84 |
| 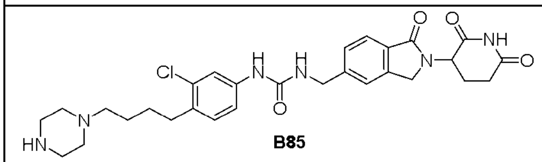 B85 | 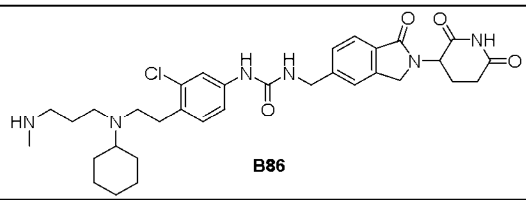 B86 |
| 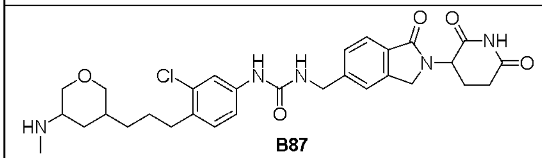 B87 | 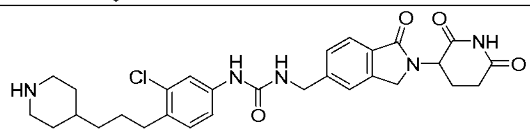 B88 |
| 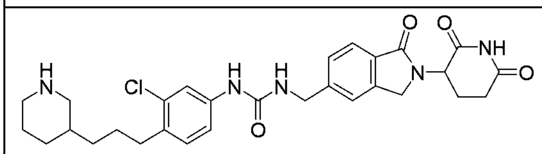 B89 | 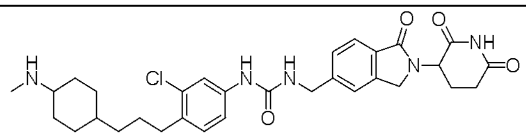 B90 |
| 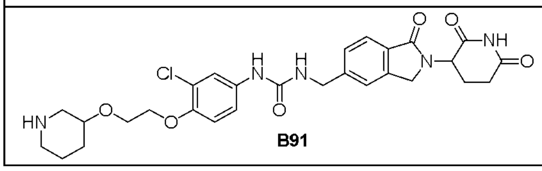 B91 | 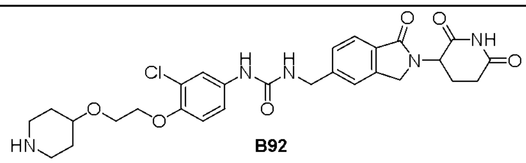 B92 |

| | |
|---|---|
| 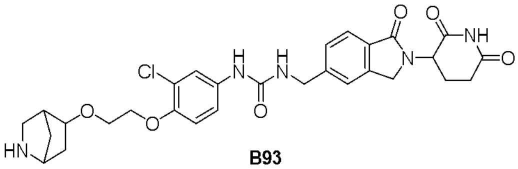 B93 | 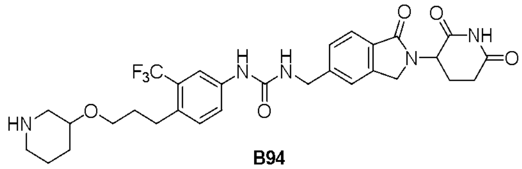 B94 |
| 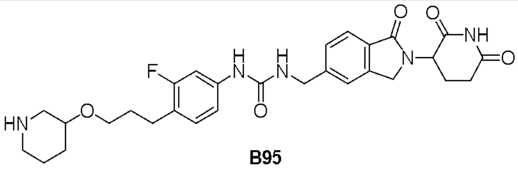 B95 | 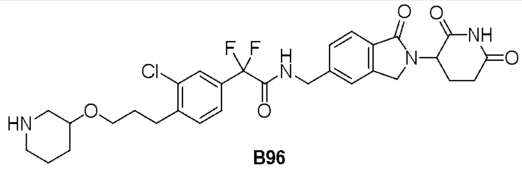 B96 |

In some embodiments, the present disclosure provides use of the compounds represented by formula (I-1) to formula (I-12) and the compounds shown in the above table, or the tautomers, the mesomers, the racemates, the enantiomers or the diastereoisomers thereof, and the pharmaceutically acceptable salts, the hydrates or the solvates thereof, in the preparation a medicament for treating cancer.

In some embodiments, the cancer includes liver cancer, kidney cancer, lung cancer (e.g., small cell lung cancer and non-small cell lung cancer), gastric cancer, esophageal cancer, urethral cancer, bladder cancer, colon cancer, rectal cancer, prostate cancer, breast cancer, ovarian cancer, pancreatic cancer, melanoma, hematological tumors or glioblastoma multiforme, lymphoma (e.g., Hodgkin lymphoma, non-Hodgkin lymphoma, or relapsed anaplastic large cell lymphoma), cervical cancer, uterine cancer, endometrial cancer, salivary gland cancer, glioma, neuroblastoma, sarcoma, colorectal cancer, leukemia (e.g., acute lymphoblastic leukemia, acute myeloid leukemia, acute promyelocytic leukemia, chronic myeloid leukemia, or chronic lymphocytic leukemia), bone cancer, skin cancer, thyroid cancer, etc. The cancer is preferably a cancer associated with abnormal expression of HER2, Claudin18.2, DLL3, or BCMA, and further preferably, the cancer associated with abnormal expression of HER2 includes lung cancer, breast cancer (e.g., ductal breast cancer), ovarian cancer, endometrial cancer, gastric cancer, and prostate cancer, the cancer associated with abnormal expression of DLL3 includes lung cancer (e.g., small cell lung cancer and non-small cell lung cancer), and the cancer associated with abnormal expression of BCMA includes lymphoma (multiple myeloma).

In some embodiments, the present disclosure provides use of the compounds represented by formula (I-1) to formula (I-12) and the compounds shown in the above table, or the tautomers, the mesomers, the racemates, the enantiomers or the diastereoisomers thereof, and the pharmaceutically acceptable salts, the hydrates or the solvates thereof, for the treatment of cancer.

In some embodiments, the cancer includes liver cancer, kidney cancer, lung cancer (e.g., small cell lung cancer and non-small cell lung cancer), gastric cancer, esophageal cancer, urethral cancer, bladder cancer, colon cancer, rectal cancer, prostate cancer, breast cancer, ovarian cancer, pancreatic cancer, melanoma, hematological tumors or glioblastoma multiforme, lymphoma (e.g., Hodgkin lymphoma, non-Hodgkin lymphoma, or relapsed anaplastic large cell lymphoma), cervical cancer, uterine cancer, endometrial cancer, salivary gland cancer, glioma, neuroblastoma, sarcoma, colorectal cancer, leukemia (e.g., acute lymphoblastic leukemia, acute myeloid leukemia, acute promyelocytic leukemia, chronic myeloid leukemia, or chronic lymphocytic leukemia), bone cancer, skin cancer, thyroid cancer, etc. The cancer is preferably a cancer associated with abnormal expression of HER2, Claudin18.2, DLL3, or BCMA, and further preferably, the cancer associated with abnormal expression of HER2 includes lung cancer, breast cancer (e.g., ductal breast cancer), ovarian cancer, endometrial cancer, gastric cancer, and prostate cancer, the cancer associated with abnormal expression of DLL3 includes lung cancer (e.g., small cell lung cancer and non-small cell lung cancer), and the cancer associated with abnormal expression of BCMA includes lymphoma (multiple myeloma).

In some embodiments, the present disclosure provides a pharmaceutical composition comprising the compounds having the structures represented by formula (I-1) to formula (I-12) and the structures shown in the above table, or the tautomers, the mesomers, the racemates, the enantiomers or the diastereoisomers thereof, and the pharmaceutically acceptable salts, the hydrates or the solvates thereof, and one or more pharmaceutically acceptable excipients.

In some embodiments, the present disclosure provides an antibody-drug conjugate of the following formula (II), and a pharmaceutically acceptable salt, a hydrate, a solvate, a stereoisomer or an isotopically labeled compound thereof:

$$Ab\text{-}(L\text{-}D)_d \qquad II$$

wherein:

Ab is selected from an antibody or an antigen-binding fragment;
L is a linker moiety, with one end attached to Ab and the other end attached to a bioactive molecule D;

D is a structure formed by attaching the compound represented by formula (I) to L;
d is selected from an integer or a decimal of 1-8, such as 1, 2, 3, 4, 5, 6, 7, or 8;
when d is a decimal, d refers to an average number of linker-drug molecules conjugated to each antibody unit.

In some embodiments of the present disclosure, D is a structure formed by attaching the compounds represented by formula (I-1) to formula (I-12) to L; further preferably, D is a structure formed by linking compounds B1-B96 described above to L, preferably, via a nitrogen atom, and further preferably, via a nitrogen atom on $R_2$ or attached to $R_2$, for example:

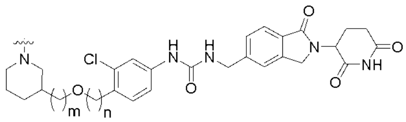

I-12a'

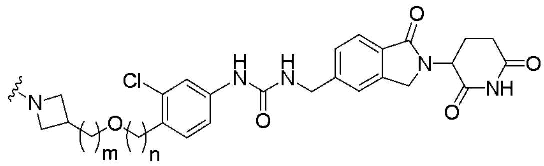

I-12b'

wherein the wavy line indicates the point of attachment of D to L.

In some embodiments, Ab is selected from an antibody targeting HER2, DLL3, Claudin18.2, or BCMA, or an antigen-binding fragment thereof.

In some embodiments, Ab is selected from trastuzumab (combination of heavy and light chains: SEQ ID NOs. 1 and 2) or pertuzumab (combination of heavy and light chains: SEQ ID NOs. 3 and 4), SYM003 (combination of heavy and light chains: SEQ ID NOs. 5 and 6), SYM004 (combination of heavy and light chains: SEQ ID NOs. 7 and 8), and belantamab (combination of heavy and light chains: SEQ ID NOs. 9 and 10).

In some embodiments, -L- is selected from -S-L'-T-C-, wherein S is an active functional group used for attachment to the antibody, L' is a spacer fragment or is absent, T is an optionally present functional group triggering cleavage of the linker, and C is an optionally present self-immolative spacer fragment.

In some embodiments, S is formed from any group Sr that reacts with the antibody or the antigen-binding fragment Ab, and Sr preferably comprises a sulfhydryl reactive group, an amino reactive group, a carboxyl reactive group, a disulfide bridging group, etc. For an antibody into which an unnatural amino acid is introduced, Sr may also comprise a click chemistry reactive group such as ketone, hydrazine or hydrazide, azide, or alkyne such as cyclotonic alkyne, cyclopropene or diene. A point of attachment of an antibody Ab includes any suitable amino acid residue or N297 glycan chain of a $CH_2$ domain, such as fucose, galactose, N-acetylgalactosamine (GalNAc), N-acetylglucosamine (GlcNAc), and sialic acid (SA) introduced by glycoengineering. The attachment reaction includes a chemical reaction or an enzymatic reaction, for example, transferring an amine-containing drug linker or a reactive spacer into a deglycosylated antibody using glutamine transaminase (MTGase).

For example, if Sr comprises a methylsulfonylpyrimidine group or a maleimide group, S comprises the following fragment attached to the antibody:

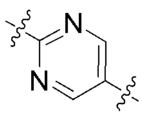
or
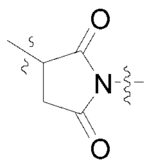

or a ring-opened form thereof:

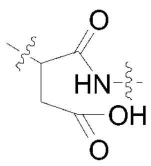 or 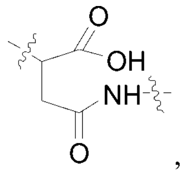 ,

wherein the wavy line on the left indicates the point of attachment with the antibody moiety, and the wavy line on the right indicates the point of attachment with the remainder of Sr or with L' (or with T if L' is absent).

In some embodiments, Sr further comprises any linker fragment $S_L$, wherein $S_L$ is optionally substituted $C_{1-10}$ alkylene, alkenylene or alkynylene, $C_{6-12}$ arylene or $C_{3-12}$ cycloalkylene, $C_{2-11}$ heteroarylene or $C_{2-11}$ heterocyclylene, or a combination thereof, and is optionally interrupted by O, CO, NH, or a combination thereof; preferably, $S_L$ comprises alkynyl or a polyethylene glycol fragment; further preferably, $S_L$ comprises cyclohexyl, phenyl, triazolyl, piperidinyl, or piperazinyl.

In some embodiments, L' comprises a hydrophilic modification fragment such as a polyethylene glycol fragment; in other embodiments, L' comprises an amino acid residue having a tertiary amine or quaternary ammonium group in the side chain.

In some embodiments, T is a peptide fragment, specifically a divalent peptide group comprising 1 to 8 (specifically 1, 2, 3, 4, 5, 6, 7, or 8) optionally substituted natural or non-natural, L- or D-amino acid residues, wherein each of the amino acid residues is the same or different, and is a residue of an amino acid independently selected from the following: alanine (Ala), cysteine (Cys), aspartic acid (Asp), glutamic acid (Glu), phenylalanine (Phe), glycine (Gly), histidine (His), isoleucine (Ile), lysine (Lys), leucine (Leu), methionine (Met), asparagine (Asn), proline (Pro), glutamine (Gln), arginine (Arg), serine (Ser), threonine (Thr), valine (Val), tryptophan (Trp), tyrosine (Tyr), citrulline (Cit), norvaline (Nva), norleucine (Nle), selenocysteine (Sec), pyrrolysine (Pyl), homoserine, homocysteine, desmethylpyrrolysine, and analogs of the amino acids described above; e.g., -ValCit-, -CitVal-, - AlaAla-, -AlaCit-, -CitAla-, -AsnCit-, -CitAsn-, -CitCit-, -ValGlu-, -GluVal-, -SerCit-, -CitSer-, -LysCit-, -CitLys-, -AspCit-, -CitAsp-, -AlaVal-, -ValAla-, -PheAla-, -AlaPhe-, -PheLys-, -LysPhe-, -ValLys-, -LysVal-, -AlaLys-, - LysAla-, -PheCit-, -CitPhe-, -LeuCit-, -CitLeu-, -IleCit-, -CitIle-, -PheArg-, -ArgPhe-, -CitTrp-, -TrpCit-, - AlaAlaAla-, -PhePheLys-, -LysPhePhe-, -DPhePheLys-, -DLysPhePhe-, -GlyPheLys-, -LysPheGly-, - GlyPhe-LeuGly-, -GlyLeuPheGly-, -GluValCit-, -AlaLeuAlaLeu-, -GlyGlyGly-, -GlyGlyGlyGly-, - GlyPheValGly-, -GlyValPheGly-, -GlyGlyPheGly-, or -GlyGlyValGly-.

In a further embodiment, L' is located at both ends of the peptide fragment T or between any two amino acids or replaces any one amino acid.

In some embodiments, S, L', T, C, and D are linked via any functional groups, preferably via amide bonds, wherein the N atom in the amide bond is optionally substituted with methyl.

In some embodiments, Sr is selected from Sr1-Sr11:

| | | | |
|---|---|---|---|
| Sr1 | 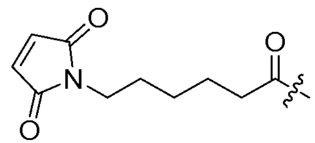 | Sr7 | 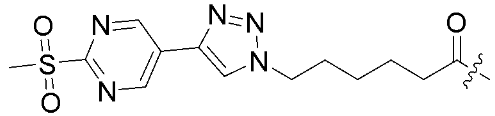 |
| Sr2 | 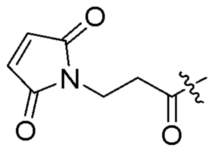 | Sr8 | 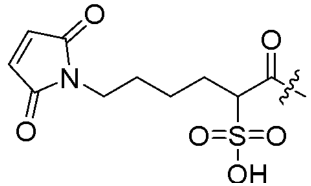 |
| Sr3 | 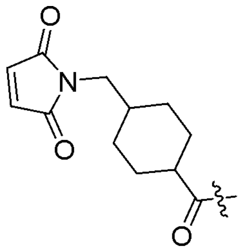 | Sr9 | 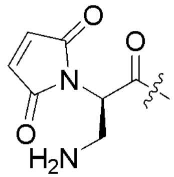 |
| Sr4 | 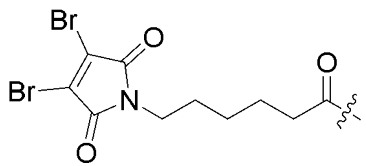 | Sr10 | 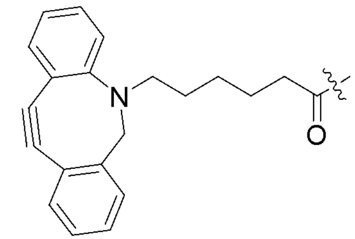 |
| Sr5 | 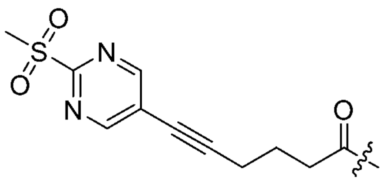 | Sr11 | 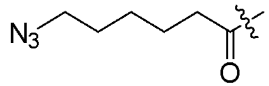 |

(continued)

| | |
|---|---|
| Sr6 | 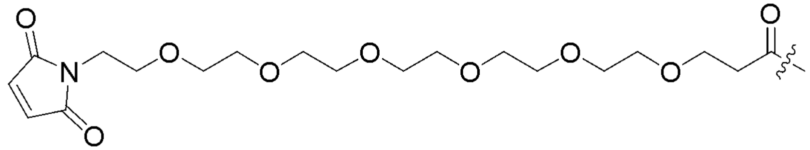 |

In some embodiments, L' is selected from L'1-L'7:

| | |
|---|---|
| L'1 | 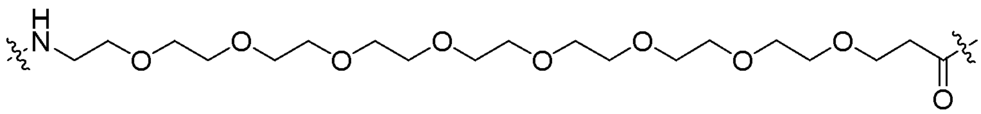 |
| L'2 | 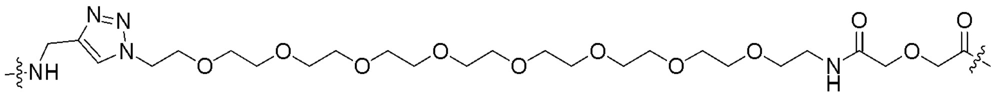 |
| L'3 | 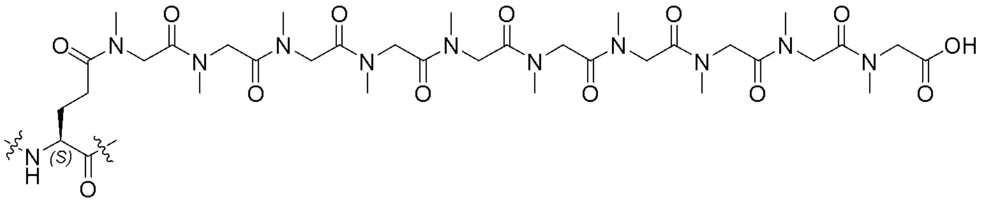 |
| L'4 | 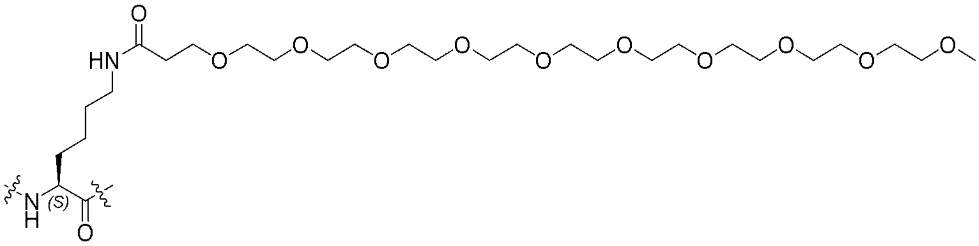 |
| L'5 | 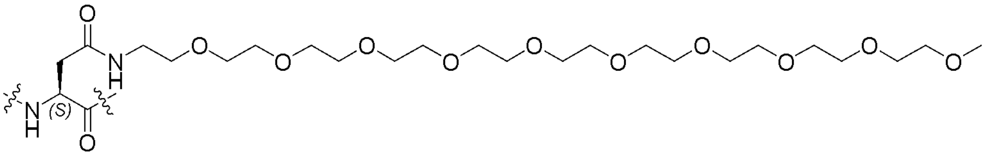 |
| L'6 | 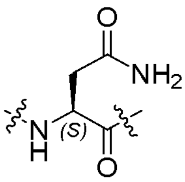 |
| L'7 | 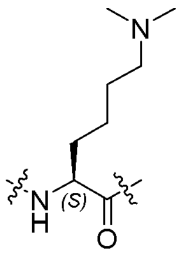 |

In some embodiments, T is selected from T1-T11:

| | | | |
|---|---|---|---|
| T1 | 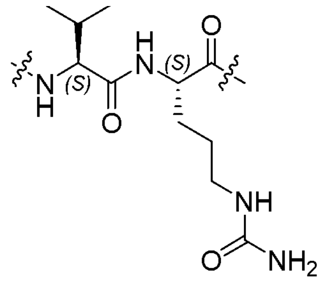 | T7 | 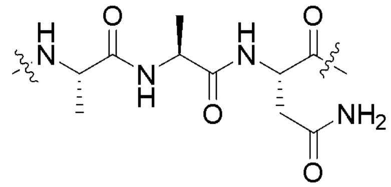 |
| T2 | 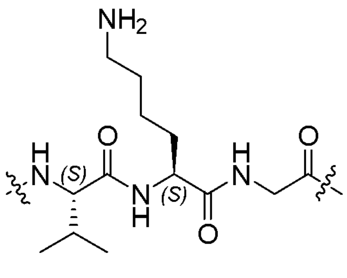 | T8 | 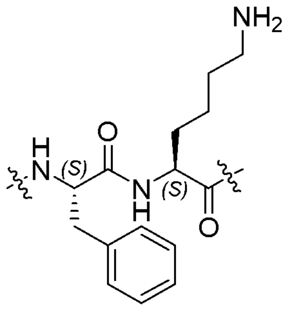 |
| T3 | 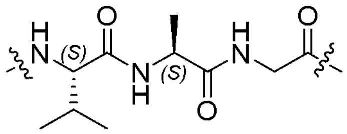 | T9 | 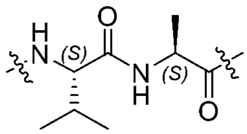 |
| T4 | 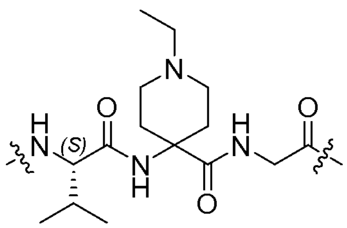 | T10 | 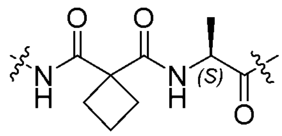 |
| T5 | 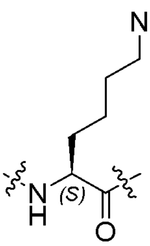 | T11 | 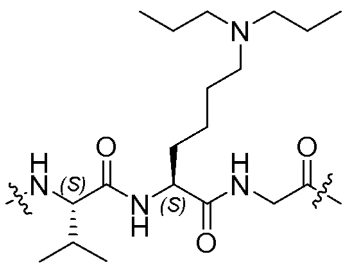 |
| T6 | 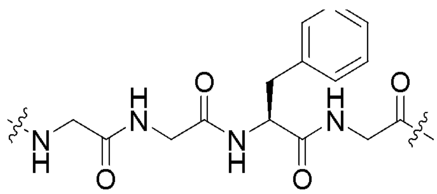 | | |

In some embodiments, C is selected from C1 and C2:

| | | | |
|---|---|---|---|
| C1 | 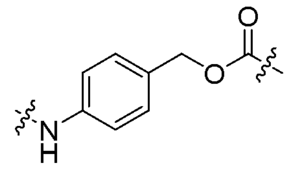 | C2 | 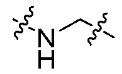 |

In some embodiments of the present disclosure, formula (II) has a structure represented by formula (II-a1) or formula (II-a2):

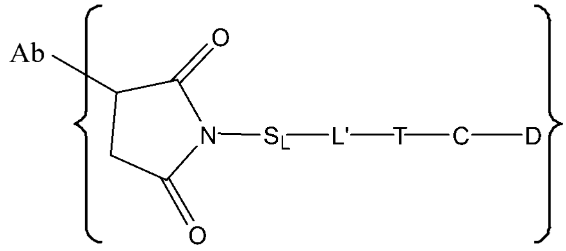

(II-a1)

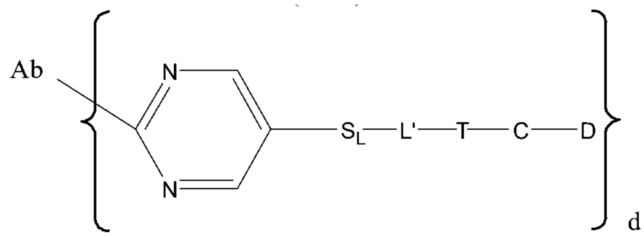

(II-a2)

wherein Ab, $S_L$, L', C, D, and d have the same meanings as above.

In some embodiments of the present disclosure, formula (II) has structures represented by formula (II-b1) to formula (II-b4):

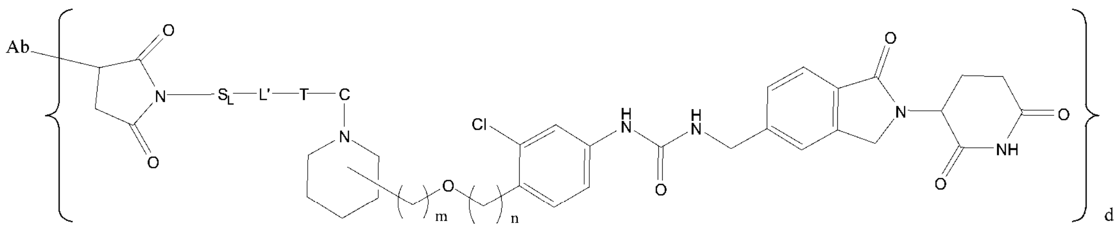

(II-b1)

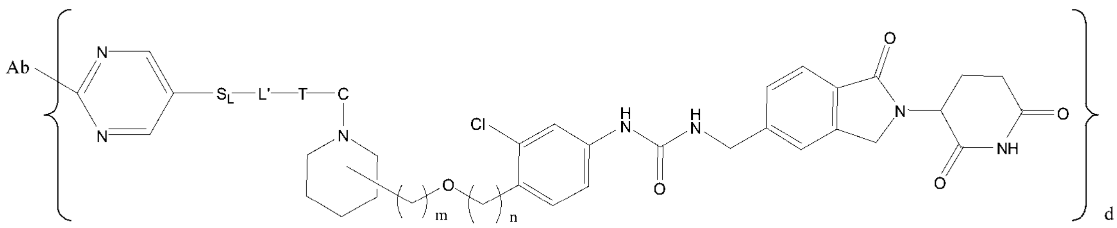

(II-b2)

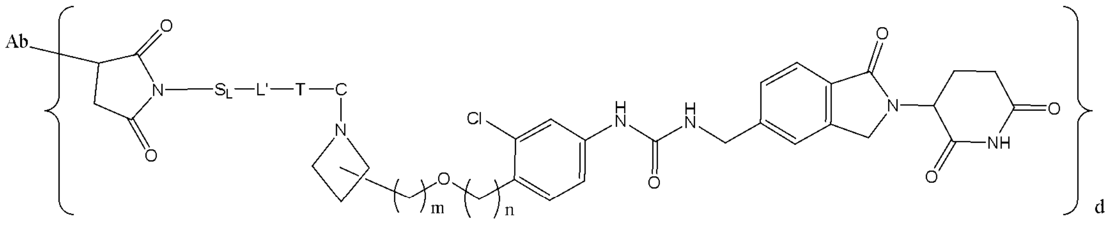

(II-b3)

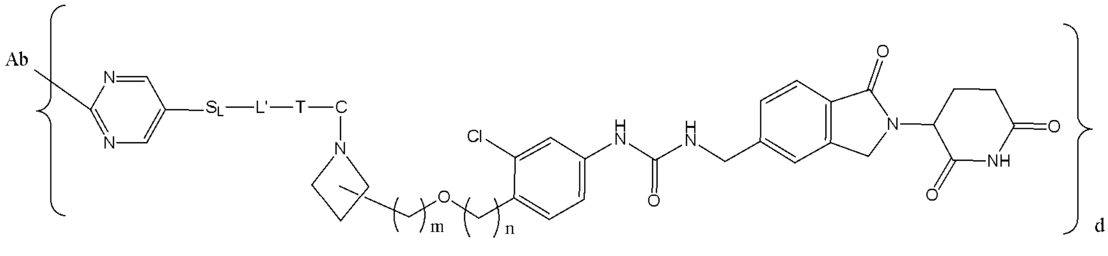

(II-b4)

wherein L' is absent, T is a peptide fragment, C is a self-immolative spacer fragment, and the remaining groups have the same meaning as above.

In some embodiments of the present disclosure, formula (II) has a structure represented by formulas (II-c1) to (II-c5):

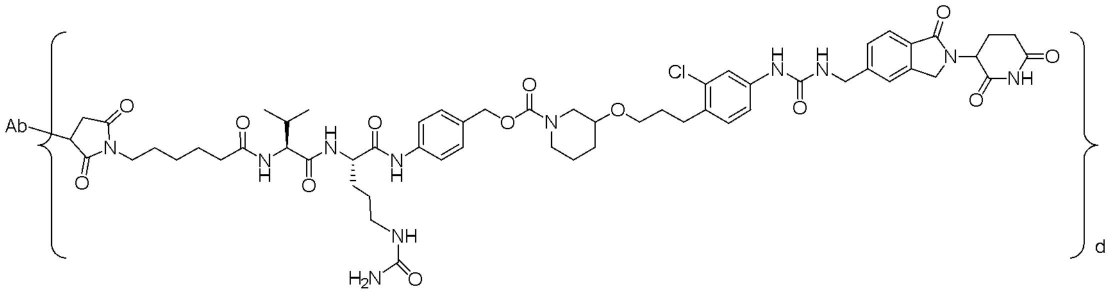

II-c1

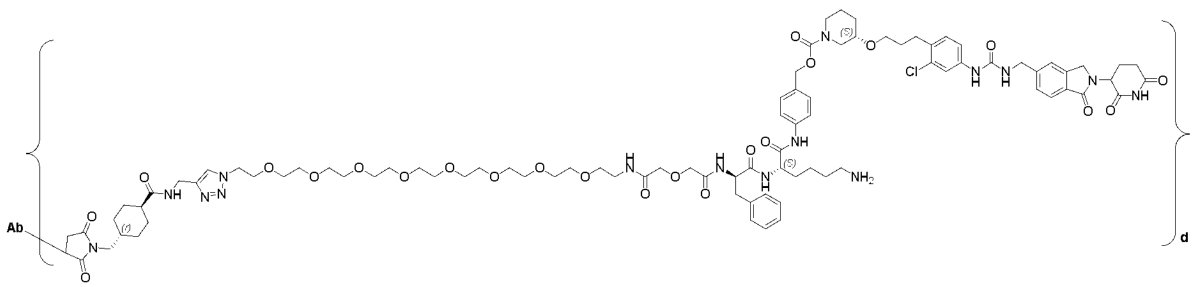

II-c2

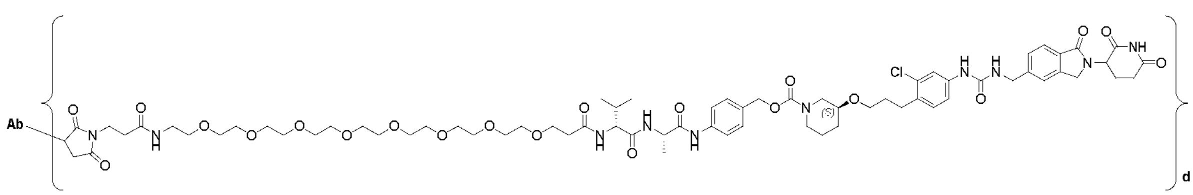

II-c3

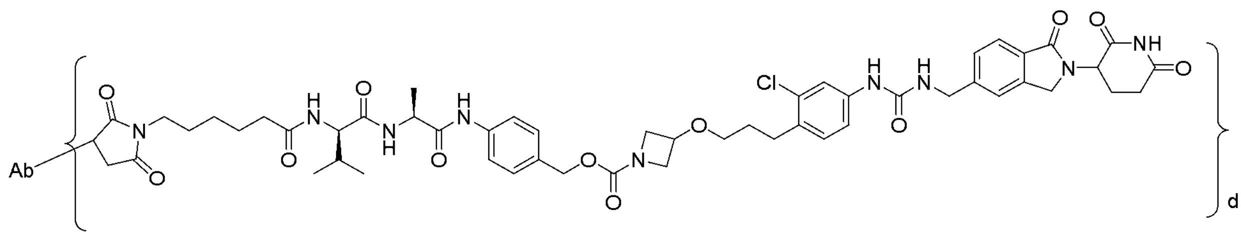

II-c4

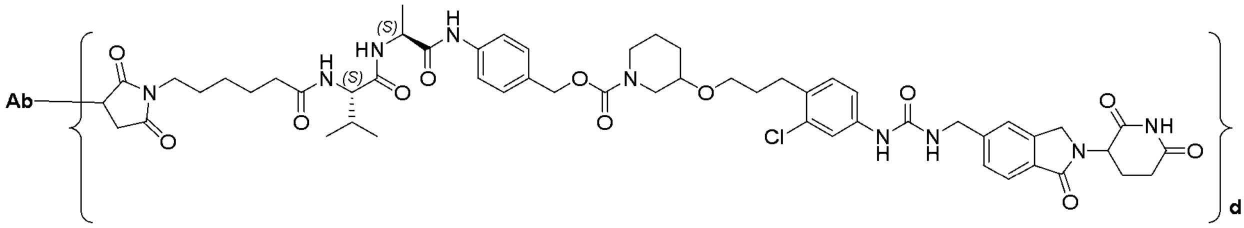

II-c5

In some embodiments of the present disclosure, Ab is selected from trastuzumab (combination of heavy and light chains: SEQ ID NOs. 1 and 2) or pertuzumab (combination of heavy and light chains: SEQ ID NOs. 3 and 4), SYM003 (combination of heavy and light chains: SEQ ID NOs. 5 and 6), SYM004 (combination of heavy and light chains: SEQ ID NOs. 7 and 8), and belantamab (combination of heavy and light chains: SEQ ID NOs. 9 and 10); d is selected from an integer or a decimal of 1-8, such as 1, 2, 3, 4, 5, 6, 7, or 8;

when d is a decimal, d refers to an average number of linker-drug molecules conjugated to each antibody unit.

The present disclosure further provides a compound of the following formula (III):

Sr-L'-T-C-D (III)

wherein Sr, L', T, C, and D have the same definitions as above.

In some embodiments, the present disclosure provides compounds of the following formulas (III-a1) and (III-a2):

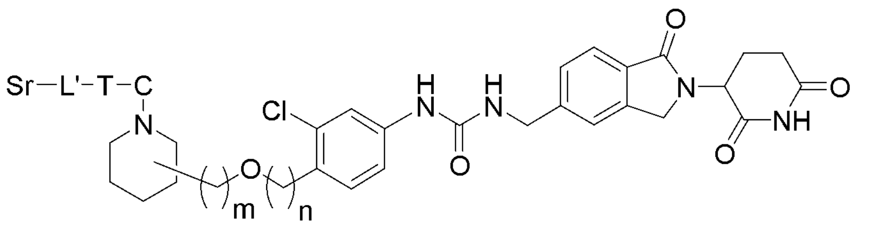

III-a1

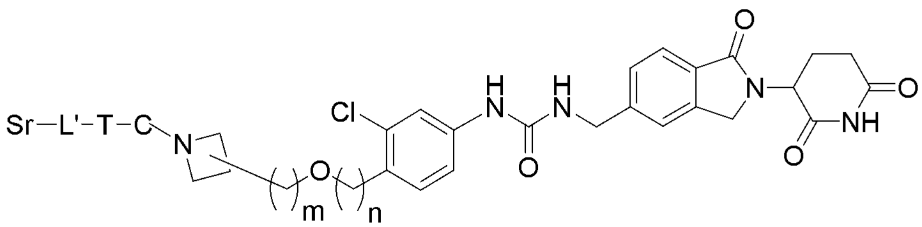

III-a2

wherein Sr, L', T, C, m, and n have the same definitions as above; preferably, Sr comprises a maleimide group or a methylsulfonylpyrimidine group, L' is absent, T is a peptide fragment, and C is

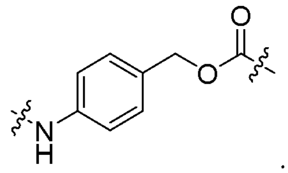

In some embodiments, the present disclosure provides compounds of the following formulas (III-b1) to (III-b5):

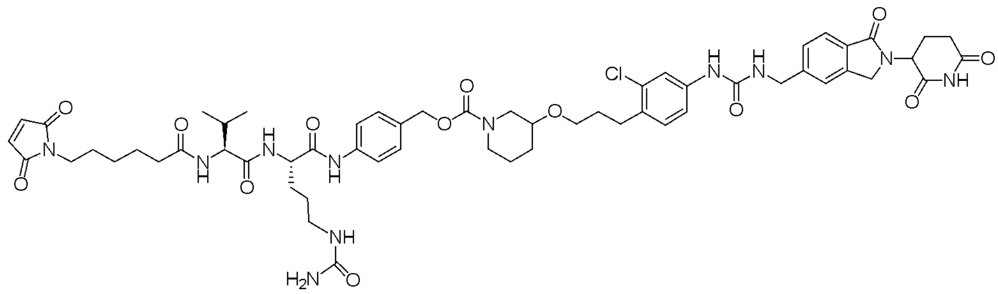

III-b1

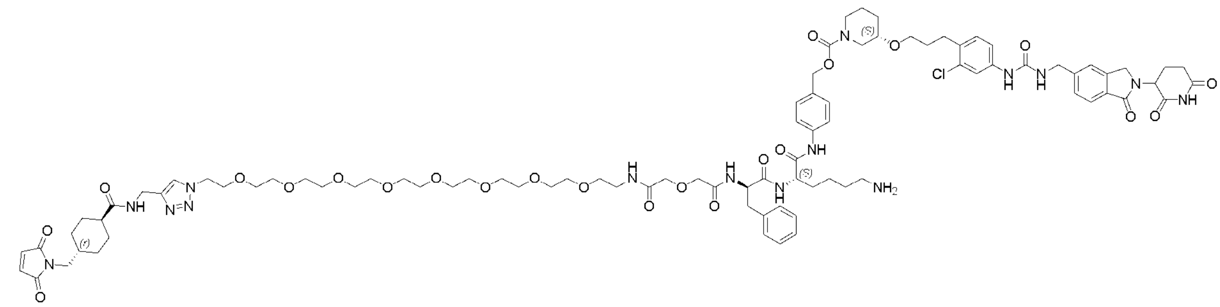

III-b2

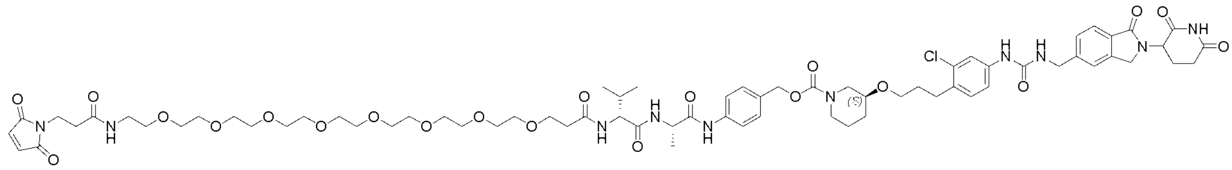

III-b3

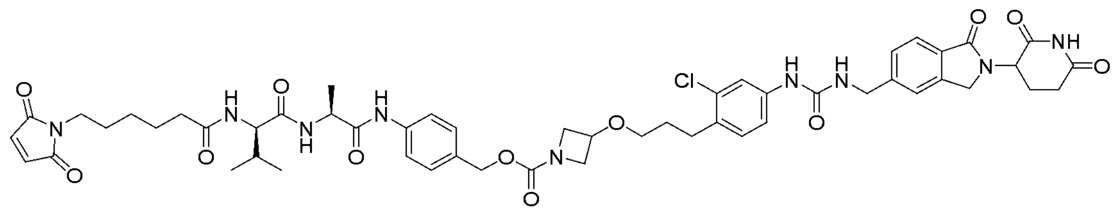

III-b4

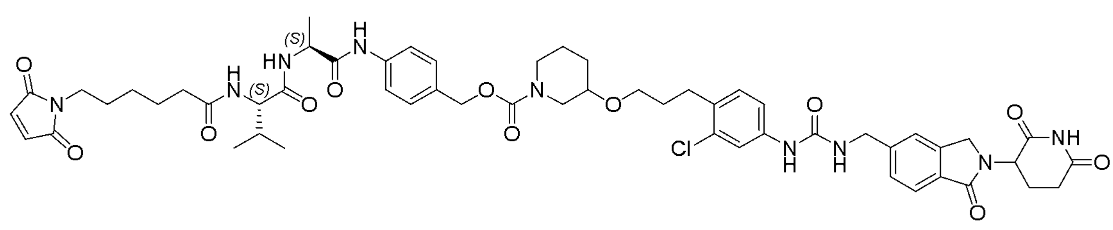

III-b5

The present disclosure further provides use of the compound of formula I in the preparation of an antibody-drug conjugate (ADC).

In the present disclosure, the antibody is attached to the compound of formula (III) by conventional conjugation methods in the art, including lysine conjugation, reductive disulfide bond conjugation between heavy and light chains, and site-specific conjugation (Beck A, Reichert JM. Antibody-drug conjugates: Present and future. MAbs, 2014, 6: 15-17; McCombs J R, Owen S C. Antibody drug conjugates: design and selection of linker, payload and conjugation chemistry. The AAPS journal, 2015, 17: 339-351). The present disclosure preferably provides conjugation via reductive disulfide bonds between the light and heavy chains, i.e., linkage via a reaction with thiol groups (sulfur atoms of cysteine residues) formed by reduction of one or more of disulfide bond sites between the light and heavy chains (two sites between the heavy chains and two sites between the heavy and light chains).

The present disclosure further provides a pharmaceutical composition comprising the antibody-drug conjugate, and the pharmaceutically acceptable salt, the hydrate, the solvate, the stereoisomer or the isotopically labeled compound thereof described herein. The pharmaceutical composition further comprises a pharmaceutically acceptable excipient and carrier.

The present disclosure further provides use of the antibody-drug conjugate, and the pharmaceutically acceptable salt, the hydrate, the solvate, the stereoisomer or the isotopically labeled compound thereof described herein in the preparation of a medicament for treating cancer.

In some embodiments, the cancer includes liver cancer, kidney cancer, lung cancer (e.g., small cell lung cancer and non-small cell lung cancer), gastric cancer, esophageal cancer, urethral cancer, bladder cancer, colon cancer, rectal cancer, prostate cancer, breast cancer, ovarian cancer, pancreatic cancer, melanoma, hematological tumors or glioblastoma multiforme, lymphoma (e.g., Hodgkin lymphoma, non-Hodgkin lymphoma, or relapsed anaplastic large cell lymphoma), cervical cancer, uterine cancer, endometrial cancer, salivary gland cancer, glioma, neuroblastoma, sarcoma, colorectal cancer, leukemia (e.g., acute lymphoblastic leukemia, acute myeloid leukemia, acute promyelocytic leukemia, chronic myeloid leukemia, or chronic lymphocytic leukemia), bone cancer, skin cancer, thyroid cancer, etc. The cancer is preferably a cancer associated with abnormal expression of HER2, Claudin18.2, DLL3, or BCMA. Further preferably, the cancer associated with abnormal expression of HER2 includes lung cancer, breast cancer (e.g., ductal breast carcinoma), ovarian cancer, endometrial cancer, gastric cancer, and prostate cancer, the cancer associated with abnormal expression of DLL3 includes lung cancer (e.g., small cell lung cancer and non-small cell lung cancer), and the cancer associated with abnormal expression of BCMA includes lymphoma (multiple myeloma).

In some embodiments, the present disclosure provides use of the antibody-drug conjugate, and the pharmaceutically acceptable salt, the hydrate, the solvate, the stereoisomer or the isotopically labeled compound thereof described herein for treating cancer.

In some embodiments, the cancer includes liver cancer, kidney cancer, lung cancer (e.g., small cell lung cancer and non-small cell lung cancer), gastric cancer, esophageal cancer, urethral cancer, bladder cancer, colon cancer, rectal cancer, prostate cancer, breast cancer, ovarian cancer, pancreatic cancer, melanoma, hematological tumors or glioblastoma multiforme, lymphoma (e.g., Hodgkin lymphoma, non-Hodgkin lymphoma, or relapsed anaplastic large cell lymphoma), cervical cancer, uterine cancer, endometrial cancer, salivary gland cancer, glioma, neuroblastoma, sarcoma, colorectal cancer, leukemia (e.g., acute lymphoblastic leukemia, acute myeloid leukemia, acute promyelocytic leukemia, chronic myeloid leukemia, or chronic lymphocytic leukemia), bone cancer, skin cancer, thyroid cancer, etc. The cancer is preferably a cancer associated with abnormal expression of HER2, Claudin18.2, DLL3, or BCMA. Further preferably, the cancer associated with abnormal expression of HER2 includes lung cancer, breast cancer (e.g., ductal breast carcinoma), ovarian cancer, endometrial cancer, gastric cancer, and prostate cancer, the cancer associated with abnormal expression of DLL3 includes lung cancer (e.g., small cell lung cancer and non-small cell lung cancer), and the cancer associated with abnormal expression of BCMA includes lymphoma (multiple myeloma).

Compared with the compounds in the prior art, such as Smol006 from Orum Therapeutics, the domide molecular glue compound of the present disclosure has better effects, such as better cell viability and better tumor inhibitory effects. The compound of the present disclosure can be used for preparing an antibody-drug conjugate (ADC), and the ADC prepared also has better effects, such as better cell viability and better tumor inhibitory effects.

## Abbreviations and Definitions

Unless otherwise stated, the following terms used in the present application have the following meanings.

When a trade name is used in the present application, the trade name includes the product formula, generic drug, and active pharmaceutical ingredient of the product under the trade name, unless otherwise indicated in the context. Unless otherwise specified, the term "antibody-drug conjugate" means that an antibody (e.g., a monoclonal antibody) or an antibody fragment is attached to a biologically active cytotoxin drug via a stable chemical linker compound.

Unless otherwise specified, the term "linker-drug compound" refers to a partial structure of the "antibody-drug conjugate" consisting of the linker compound and the drug compound.

The linker-drug compound of the present disclosure is attached to the antibody by conventional conjugation methods in the art, including lysine conjugation, reductive disulfide bond conjugation between heavy and light chains, and site-specific conjugation (Beck A, Reichert JM. Antibody-drug conjugates: Present and future. MAbs, 2014, 6: 15-17; McCombs J R, Owen S C. Antibody drug conjugates: design and selection of linker, payload and conjugation chemistry. The AAPS journal, 2015, 17: 339-351). The present disclosure preferably provides conjugation via reductive disulfide bonds between the light and heavy chains, i.e., linkage via a reaction with thiol groups (sulfur atoms of cysteine residues) formed by reduction of one or more of disulfide bond sites between the light and heavy chains (two sites between the heavy chains and two sites between the heavy and light chains).

The term "tautomer" refers to isomers having different energies that are interconvertible by a lower energy barrier. If a tautomer is possible (e.g., in solution), the chemical equilibrium of the tautomer can be achieved. For example, a proton tautomer (also known as a prototropic tautomer) includes tautomers that undergo interconversion by proton migration, such as keto-enol isomerism and imine-enamine isomerism. A valence tautomer includes tautomers that undergo interconversion by recombination of bonding electrons.

The compound of the present disclosure can be present in an isotopically labeled or enriched form, containing one or more atoms whose atomic mass or mass number is different from that of the largest amount of atoms found in nature. The isotope may be a radioactive or non-radioactive isotope. Isotopes commonly used as isotopic labels include hydrogen isotopes: $^{2}H$ and $^{3}H$; carbon isotopes: $^{13}C$ and $^{14}C$; chlorine isotopes: $^{35}Cl$ and $^{37}Cl$; fluorine isotope: $^{18}F$; iodine isotopes: $^{123}I$ and $^{125}I$; nitrogen isotopes: $^{13}N$ and $^{15}N$; oxygen isotopes: $^{15}O$, $^{17}O$, and $^{18}O$; and sulfur isotope: $^{35}S$. These isotopically labeled compounds can be used for research on the distribution of pharmaceutical molecules in tissues. Particularly, $^{2}H$ and $^{13}C$ are more widely used due to their ease of labeling and ease of detection. The substitution with certain heavy isotopes, such as deuterium ($^{2}H$), can enhance the stability of metabolism and prolong the half-life to achieve the purpose of reducing the dose and provide therapeutic advantages. Isotopically labeled compounds are generally synthesized starting from labeled starting materials and synthesized using known synthetic techniques in the same way as non-isotopically labeled compounds. The present application includes various deuterated forms. Each available hydrogen atom attached to carbon atoms may be independently replaced with a deuterium atom. In addition to commercially available deuterated molecular building blocks, deuterated products can be prepared using commercially available deuterated starting materials or synthesized by conventional techniques with deuterated reagents. Non-limiting examples of deuterated reagents include: deuterated water, deuterated acetone, deuterated methanol, deuterated acetonitrile, deuterated borane, deuterated sodium borohydride, deuterated lithium aluminum hydride, etc.

The term "solvate" means a physical association of the compound of the present disclosure with one or more solvent molecules (whether organic or inorganic). The physical association includes hydrogen bonding. In certain cases, the solvate can be isolated, for example, when one or more solvent molecules are incorporated into the crystal lattice of a crystalline solid. The solvent molecules in the solvate may be present in a regular arrangement and/or a disordered arrangement. The solvate may contain a stoichiometric or non-stoichiometric amount of solvent molecules. The "solvate" encompasses both solution phase and isolatable solvates. Exemplary solvates include, but are not limited to, hydrates, ethanolates, methanolates, and isopropanolates. Solvation methods are well known in the art.

The term "heteroatom" refers to nitrogen, oxygen, sulfur, and halogen atoms.

The term "alkyl" refers to a monovalent saturated aliphatic hydrocarbyl group, i.e., a linear or branched group containing 1-20 carbon atoms, preferably containing 1-10 carbon atoms (i.e., C1-10 alkyl), further preferably containing 1-8 carbon atoms (C1-8 alkyl), and more preferably containing 1-6 carbon atoms (i.e., C1-6 alkyl). For example, "C1-6 alkyl" means that the group is alkyl and the number of carbon atoms on the carbon chain is between 1 and 6 (specifically 1, 2, 3, 4, 5, or 6), and examples include, but are not limited to, methyl, ethyl, n-propyl, isopropyl, n-butyl, isobutyl, *tert*-butyl, *sec*-butyl, n-pentyl, neopentyl, 1,1-dimethylpropyl, 1,2-dimethylpropyl, 2,2-dimethylpropyl, 1-ethylpropyl, 2-methylbutyl, 3-methylbutyl, n-hexyl, etc.

The term "-alkyl-" or "alkylene" refers to saturated linear or branched chain divalent hydrocarbyl. For example, C1-C8 alkylene refers to linear or branched alkylene having 1-8 carbon atoms.

The term "alkoxy" refers to -O-alkyl, wherein the alkyl is defined as above, i.e., the alkyl contains 1-20 carbon atoms, preferably 1-10 carbon atoms, more preferably 1-8 carbon atoms, and further more preferably 1-6 (specifically 1, 2, 3, 4, 5, or 6) carbon atoms. Representative examples of the alkoxy include, but are not limited to, methoxy, ethoxy, propoxy, isopropoxy, butoxy, 1-methylpropoxy, 2-methylpropoxy, tert-butoxy, pentyloxy, 1-methylbutoxy, 2-methylbutoxy, 3-methylbutoxy, 1,1-dimethylpropoxy, 1,2-dimethylpropoxy, 2,2-dimethylpropoxy, 1-ethylpropoxy, etc.

The term "halogen" or "halo" refers to F, Cl, Br, and I.

The term "haloalkyl" means that one, two, or more hydrogen atoms or all hydrogen atoms in the alkyl defined as above are substituted with halogen. Representative examples of the haloalkyl include $CCl_3$, $CHCl_2$, $CH_2Cl$, $CF_3$, $CHF_2$, $CH_2F$, $CBr_3$, $CHBr_2$, $CH_2Br$, $CI_3$, $CHI_2$, $CH_2I$, $CH_2CF_3$, $CF_2CF_3$, etc.

The term "aryl" or "aromatic ring group" refers to a monocyclic, bicyclic, and tricyclic aromatic carbocyclic system containing 6-16 carbon atoms, or 6-14 carbon atoms, or 6-12 carbon atoms, or 6-10 carbon atoms, preferably 6-10 carbon atoms, and the term "aryl" are used interchangeably with the term "aromatic ring". Examples of the aryl group may include,

but are not limited to, phenyl, naphthyl, anthryl, phenanthryl, pyrenyl, or the like.

The term "heteroaryl" or "heteroaromatic ring group" refers to an aromatic monocyclic or polycyclic ring system containing a 5- to 14-membered structure, or preferably a 5- to 10-membered structure or a 5- to 8-membered structure, and more preferably a 5- to 6-membered structure, wherein 1, 2, 3 or more ring atoms are heteroatoms and the remaining atoms are carbon, the heteroatoms are independently selected from O, N, and S, and the number of the heteroatoms is preferably 1, 2, or 3. Examples of the heteroaryl include, but are not limited to, furanyl, thienyl, oxazolyl, thiazolyl, isoxazolyl, oxadiazolyl, thiadiazolyl, pyrrolyl, pyrazolyl, imidazolyl, triazolyl, tetrazolyl, pyridinyl, pyrimidinyl, pyrazinyl, pyridazinyl, thiadiazolyl, triazinyl, phthalazinyl, quinolinyl, isoquinolinyl, pteridinyl, purinyl, indolyl, isoindolyl, indazolyl, benzofuranyl, benzothienyl, benzopyridinyl, benzopyrimidinyl, benpyrazinyl, benzimidazolyl, benzophthalizinyl, pyrrolo [2,3-b]pyridinyl, imidazo[1,2-*a*]pyridinyl, pyrazolo[1,5-*a*]pyridinyl, pyrazolo[1,5-*a*]pyrimidinyl, imidazo[1,2-b]pyridazinyl, [1,2,4]triazolo[4,3-b]pyridazinyl, [1,2,4]triazolo[1,5-*a*]pyrimidinyl, [1,2,4]triazolo[1,5-*a*]pyridinyl, etc.

The term "cycloalkyl" refers to a carbocyclic ring that is fully saturated and may exist as a monocyclic, bridged cyclic, spiro cyclic, or fused cyclic structure. Preferably, the cycloalkyl contains 3-16 carbon atoms (i.e., C3-16 cycloalkyl); more preferably, the cycloalkyl contains 3-12 carbon atoms (C3-12 cycloalkyl); further preferably, the cycloalkyl contains 3-8 carbon atoms (C3-8 cycloalkyl), 3-6 carbon atoms (C3-6 cycloalkyl), or 5-6 carbon atoms (C5-6 cycloalkyl). Examples of the cycloalkyl include, but are not limited to, cyclopropyl, cyclobutyl, cyclopentyl, cyclohexyl, cycloheptyl, methylcyclopropyl, 2-ethyl-cyclopentyl, dimethylcyclobutyl, norbornyl (bicyclo[2.2.1]heptyl), bicyclo[2.2.2]octyl, adamantyl, etc.

The term "bridged cyclyl" refers to a class of polycyclic aliphatic hydrocarbyl groups in which two carbocyclic rings share two or more carbon atoms. The bridged cyclyl ring contains 4-20 carbon atoms, preferably 4-10 carbon atoms (containing 4, 5, 6, 7, 8, 9, or 10 carbon atoms). Non-limiting examples of the bridged ring include the following:

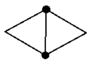 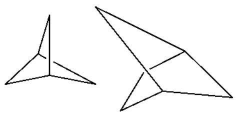 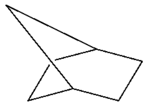 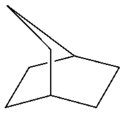

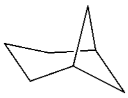 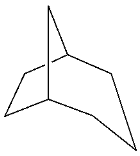 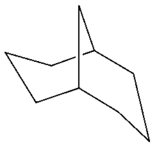 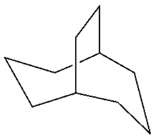

The term "spirocyclyl" refers to alicyclic hydrocarbyl in which two carbocyclic rings in the molecule share one carbon atom. The spirocyclyl ring contains 5-20 carbon atoms, preferably 5-11 carbon atoms (containing 3, 4, 5, 6, 7, 8, 9, 10, or 11 carbon atoms). Non-limiting examples of the spiro ring include the following:

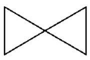 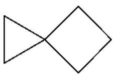 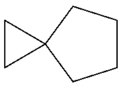 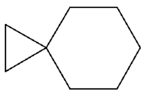 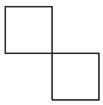 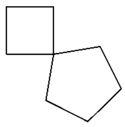

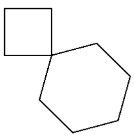 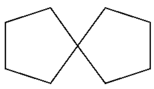 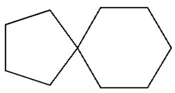 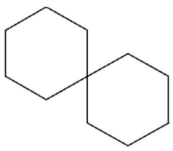

The term "fused cyclyl" refers to alicyclic hydrocarbyl in which two carbocyclic rings in the molecule share two carbon atoms. The fused cyclyl ring contains 5-20 carbon atoms, preferably 5-11 carbon atoms (containing 3, 4, 5, 6, 7, 8, 9, 10, or 11 carbon atoms). Non-limiting examples of the fused ring include the following:

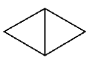 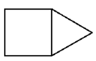 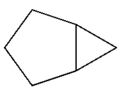 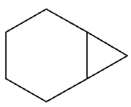 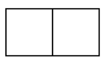 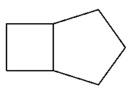

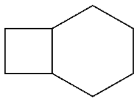 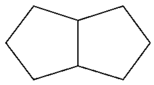 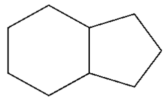 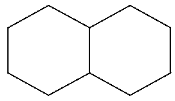 .

The term "-cycloalkyl-" or "cycloalkylene" refers to "cycloalkyl" to which two substituents are attached.

The term "heterocyclyl" or "heterocyclic ring" refers to a saturated or partially unsaturated monocyclic or polycyclic non-aromatic substituent having ring carbon atoms and 1-4 ring heteroatoms, which contains 3-20 ring atoms, wherein 1, 2, 3, or more ring atoms are selected from N, O, and S, and the remaining ring atoms are C. Preferably, the heterocyclyl contains 3-12 ring atoms (3- to 12-membered heterocyclyl), further preferably 3-10 ring atoms (3-to 10-membered heterocyclyl), or 3-8 ring atoms (3- to 8-membered heterocyclyl), or 3-6 ring atoms (3- to 6-membered heterocyclyl), or 4-6 ring atoms (4- to 6-membered heterocyclyl), or 5-6 ring atoms (5- to 6-membered heterocyclyl). The number of the heteroatoms is preferably 1-4, more preferably 1-3 (i.e., 1, 2, or 3). Examples of the monocyclic heterocyclyl include pyrrolidinyl, imidazolidinyl, tetrahydrofuranyl, dihydropyrrolyl, piperidinyl, piperazinyl, pyranyl, etc. Polycyclic heterocyclyl includes spiroheterocyclyl, fused heterocyclyl, and bridged heterocyclyl. "Heterocyclyl" may be monocyclic ("monocyclic heterocyclyl") or a fused ("fused heterocyclyl" or "heterofused cyclyl"), bridged ("heterobridged cyclyl" or "bridged heterocyclyl"), or spiro-fused ("heterospiro cyclyl" or "spiro heterocyclyl") ring system, such as a bicyclic system ("bicyclic heterocyclyl"), and may be saturated or may be partially unsaturated. The bicyclic heterocyclyl system may contain one or more heteroatoms in one or both rings. "Heterocyclyl" further includes ring systems in which the heterocyclyl ring, as defined above, is fused with one or more carbocyclyl groups, wherein the point of attachment is either on the carbocyclyl or heterocyclyl ring, or "heterocyclyl" further includes ring systems in which the heterocyclyl ring, as defined above, is fused with one or more aryl or heteroaryl groups, or the cycloalkyl ring, as defined above, is fused with one or more heteroaryl groups, wherein the point of attachment is either on the heterocyclyl or cycloalkyl ring. In such cases, the number of members of the heterocyclyl ring system is the number of atoms in the fused ring system. In certain embodiments, each example of the heterocyclyl is independently and optionally substituted, e.g., unsubstituted ("unsubstituted heterocyclyl") or substituted with one or more substituents ("substituted heterocyclyl"). Exemplary 3-membered heterocyclyl groups containing one heteroatom include, but are not limited to, aziridinyl, oxiranyl, and thiorenyl. Exemplary 4-membered heterocyclyl groups containing one heteroatom include, but are not limited to, azetidinyl, oxetanyl, and thietanyl. Exemplary 5-membered heterocyclyl groups containing one heteroatom include, but are not limited to, tetrahydrofuranyl, dihydrofuranyl, tetrahydrothiophenyl, dihydrothiophenyl, pyrrolidinyl, dihydropyrrolyl, and pyrrolyl-2,5-dione. Exemplary 5-membered heterocyclyl groups containing two heteroatoms include, but are not limited to, dioxolanyl, oxathiolanyl, dithiolanyl, and oxazolidin-2-one. Exemplary 5-membered heterocyclyl groups containing three heteroatoms include, but are not limited to, triazolinyl, oxadiazolinyl, and thiadiazolinyl. Exemplary 6-membered heterocyclyl groups containing one heteroatom include, but are not limited to, piperidinyl, tetrahydropyranyl, dihydropyridinyl, and thianyl. Exemplary 6-membered heterocyclyl groups containing two heteroatoms include, but are not limited to, piperazinyl, morpholinyl, dithianyl, and dioxanyl. Exemplary 6-membered heterocyclyl groups containing three heteroatoms include, but are not limited to, triazinanyl, oxadiazinanyl, thiadiazinanyl, oxathiazinanyl, and dioxazinanyl. Exemplary 7-membered heterocyclyl groups containing one heteroatom include, but are not limited to, azepanyl, oxepanyl, and thiepanyl. Exemplary 8-membered heterocyclyl groups containing one heteroatom include, but are not limited to, azocanyl, oxecanyl, and thiocanyl. Exemplary 5-membered heterocyclyl groups fused to a $C_6$ aryl ring (also referred to herein as a 5,6-bicyclic heterocyclic ring) include, but are not limited to, indolinyl, isoindolinyl, dihydrobenzofuranyl, dihydrobenzothienyl, benzoxazolonyl, etc. Exemplary 6-membered heterocyclyl groups fused to an aryl ring (also referred to herein as a 6,6-bicyclic heterocyclic ring) include, but are not limited to, tetrahydroquinolinyl, tetrahydroisoquinolinyl, etc.

Unless otherwise specified, "heterocycloalkyl" refers to a saturated monocyclic "heterocyclyl" or "heterocyclic ring" defined as above, with ring atoms defined as above, i.e., the heterocycloalkyl contains 3-20 ring atoms ("3- to 20-membered heterocycloalkyl") and 1-4 (1, 2, 3, or 4), preferably 1-3 (1, 2, or 3), heteroatoms, wherein the heteroatoms are each independently selected from N, O, and S. The heterocycloalkyl preferably contains 3-12 ring atoms ("3- to 12-membered heterocycloalkyl"), further preferably 3-10 ring atoms ("3- to 10-membered heterocycloalkyl"), still further preferably 3-8 ring atoms ("3- to 8-membered heterocycloalkyl"), still further preferably 4-7 ring atoms ("4- to 7-membered heterocycloalkyl"), still further preferably 5-10 ring atoms ("5- to 10-membered heterocycloalkyl"), and still further preferably 5-6 ring atoms ("5- to 6-membered heterocycloalkyl"). In certain embodiments, each example of the heterocycloalkyl is independently and optionally substituted, e.g., unsubstituted ("unsubstituted heterocycloalkyl") or substituted with one or more substituents ("substituted heterocycloalkyl"). Some exemplary "heterocycloalkyl" groups have been provided in the section of the "heterocyclyl" or "heterocyclic ring" above. The heterocycloalkyl groups further include, but are not limited to, aziridinyl, oxiranyl, thiorenyl, azetidinyl, oxetanyl, thietanyl, tetrahydrofuranyl, oxanyl, piperidinyl, piperazinyl, morpholinyl, thiomorpholinyl, oxathianyl, oxazolidinyl, dioxanyl, dithianyl, thiazolidinyl, pyrrolidinyl, pyrazolidinyl, imidazolinidinyl, etc.

Non-limiting examples of "spiroheterocyclyl" include:

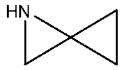
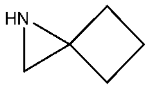
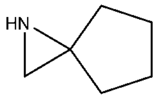
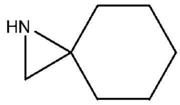
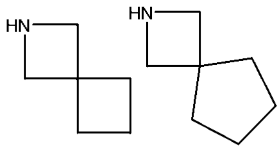
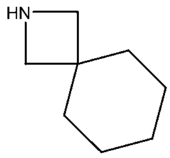
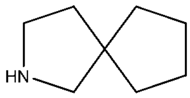
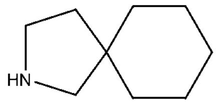
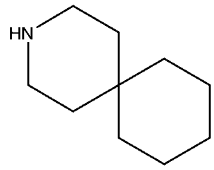
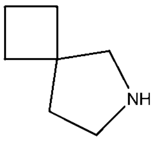
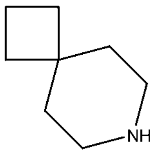
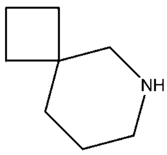

Non-limiting examples of "fused heterocyclyl" include:

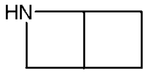
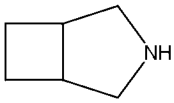
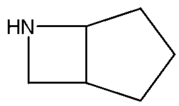
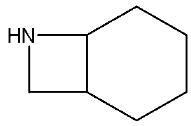
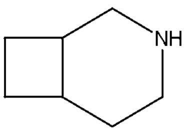
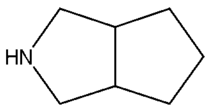
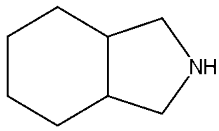
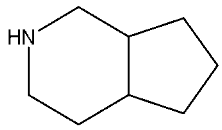
.

The term "-heterocyclyl-" or "heterocyclylene" refers to "heterocyclyl" to which two substituents are attached. The term "derivative" refers to a compound formed by substituting an atom or a group of atoms in the molecule of the parent compound with another atom or group of atoms, which is referred to as a derivative of the parent compound.

The term "pharmaceutically acceptable salt" refers to a pharmaceutically acceptable organic or inorganic salt of a compound (e.g., a drug, a drug-linker, or an antibody-linker-drug conjugate). The compound may contain at least one amino, imino, hydroxyl, or carboxyl group, and thus may form addition salts with the corresponding acids or bases. Exemplary salts include, but are not limited to: sulfate, trifluoroacetate, citrate, acetate, oxalate, hydrochloride, hydrobromide, hydroiodide, nitrate, bisulfate, phosphate, acidic phosphate ($-H_2PO_4$), phosphite, isonicotinate, lactate, salicylate, acidic citrate, tartrate, oleate, tannate, pantothenate, bitartrate, ascorbate, formate, benzoate, glutamate, methanesulfonate, ethanesulfonate, benzenesulfonate, p-toluenesulfonate, potassium salts, sodium salts, ammonium salts, calcium salts, etc. In addition, the pharmaceutically acceptable salt has more than one charged atom in the structure. Examples in which multiple charged atoms are part of the pharmaceutically acceptable salt can have multiple counter atoms. For example, the pharmaceutically acceptable salt has one or more charged atoms and/or one or more counter atoms.

The term "tumor" refers to a neoplasm formed by clonal abnormal proliferation of a certain cell in a local tissue due to the loss of normal regulation of its growth at the genetic level under the action of various carcinogenic factors in the body.

**DETAILED DESCRIPTION**

The present application is further illustrated by examples, which, however, are not intended to limit the scope of the present application. Experimental procedures without specified conditions in the following examples are generally conducted according to conventional conditions or according to conditions recommended by the manufacturer. Unless otherwise stated, all percentages, proportions, ratios, or parts are by weight.

Example 1: Synthesis of Compound Smol006

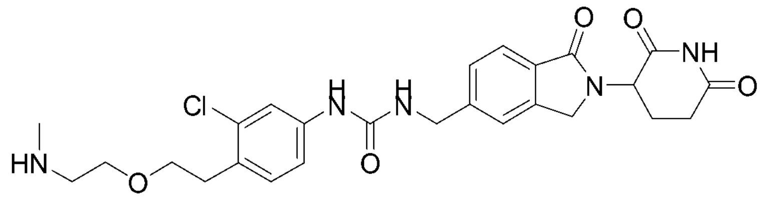

Smol006 was obtained with reference to the synthesis method for the same compound in the patent WO2021198965A1. LC-MS (ESI): $(M+H)^+$ calculated 528.2, found 528.3.

Example 2: Synthesis of Compounds B19-B24

| Structural formula | m | n | N-1 Compound | Compound |
|---|---|---|---|---|
| | 0 | 3 | A19 | B19 |
| 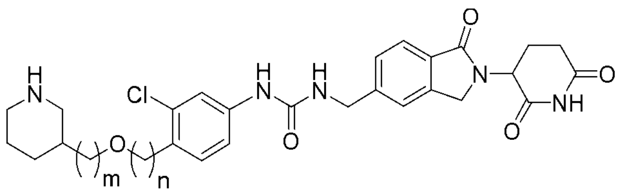 | 1 | 3 | A20 | B20 |
| | 2 | 3 | A21 | B21 |
| | 3 | 3 | A22 | B22 |
| | 4 | 3 | A23 | B23 |
| | 5 | 3 | A24 | B24 |

The synthesis of compound B19 (m = 0, n = 3) was taken as an example

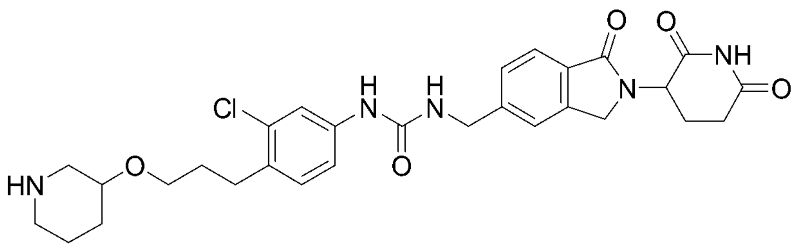

Synthesis of intermediate

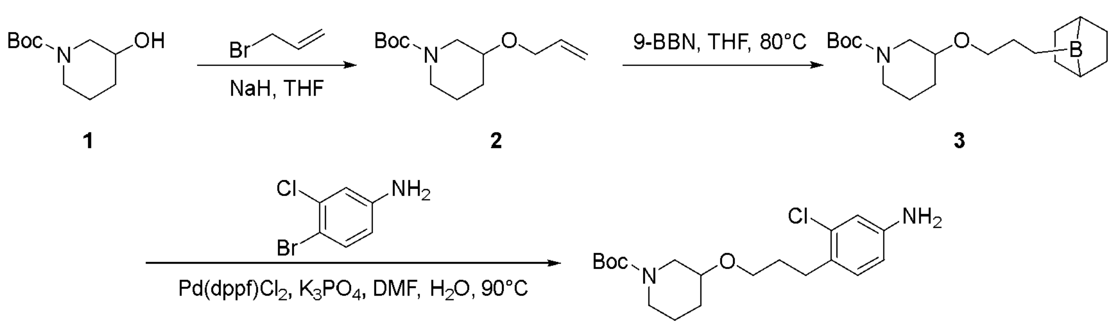


1 2 3

4

Compound 1 (49.686 mmol) was dissolved in THF (120 mL), and sodium hydride (99.372 mmol, 60%) was added

in an ice bath under nitrogen atmosphere. The mixture was stirred for half an hour. Allyl bromide (99.372 mmol) was added to the reaction system under nitrogen atmosphere. After two hours, the reaction progress was detected by TLC. After the reaction was completed, a saturated ammonium chloride solution was added in an ice bath to quench the reaction. The reaction solution was extracted with methyl tert-butyl ether and a saturated sodium chloride solution. The organic phases were combined, dried over anhydrous sodium sulfate, and then filtered. The filtrate was concentrated under reduced pressure, and the residue was purified by silica gel column chromatography to give compound 2 (33.15 mmol, yield: 66%). LC-MS (ESI): $(M+H-Boc)^+$ calculated 142.2, found 142.3.

A solution of 9-BBN in tetrahydrofuran (79.56 mL, 39.7788 mmol, 0.5 mmol/mL) was added to a solution of compound 2 (33.149 mmol) in THF in an ice bath under nitrogen atmosphere. The mixture was heated to 80 °C and reacted. After two hours, the reaction progress was monitored by TLC. After the reaction was completed, the reaction solution was cooled to room temperature. The reaction solution containing compound 3 was directly used in the next step.

DMF (100 mL) and a solution of potassium phosphate (41.436 mmol) in water (30 mL) were added to the solution of compound 3 under nitrogen atmosphere. The mixture was stirred for 10 min, and 3-chloro-4-bromoaniline (33.149 mmol) and $Pd(dppf)Cl_2$ (3.3149 mmol) were added under nitrogen atmosphere. The resulting mixture was reacted at 90 °C for three hours under nitrogen atmosphere. The reaction progress was monitored by TLC and LCMS. After the starting materials were completely consumed, the reaction solution was cooled to room temperature and extracted with ethyl acetate and a saturated ammonium chloride solution. Insoluble substances in the system were removed by filtration, and then the organic phase was extracted with a saturated sodium chloride solution. The organic phase was collected, dried over anhydrous sodium sulfate, and then filtered. The filtrate was concentrated under reduced pressure, and the residue was purified twice by silica gel column chromatography to give compound 4 (13.82 mmol, yield: 42%). LC-MS (ESI): $(M+H-Boc)^+$ calculated 269.1, found 269.1.

General Synthetic Method for Target Compound

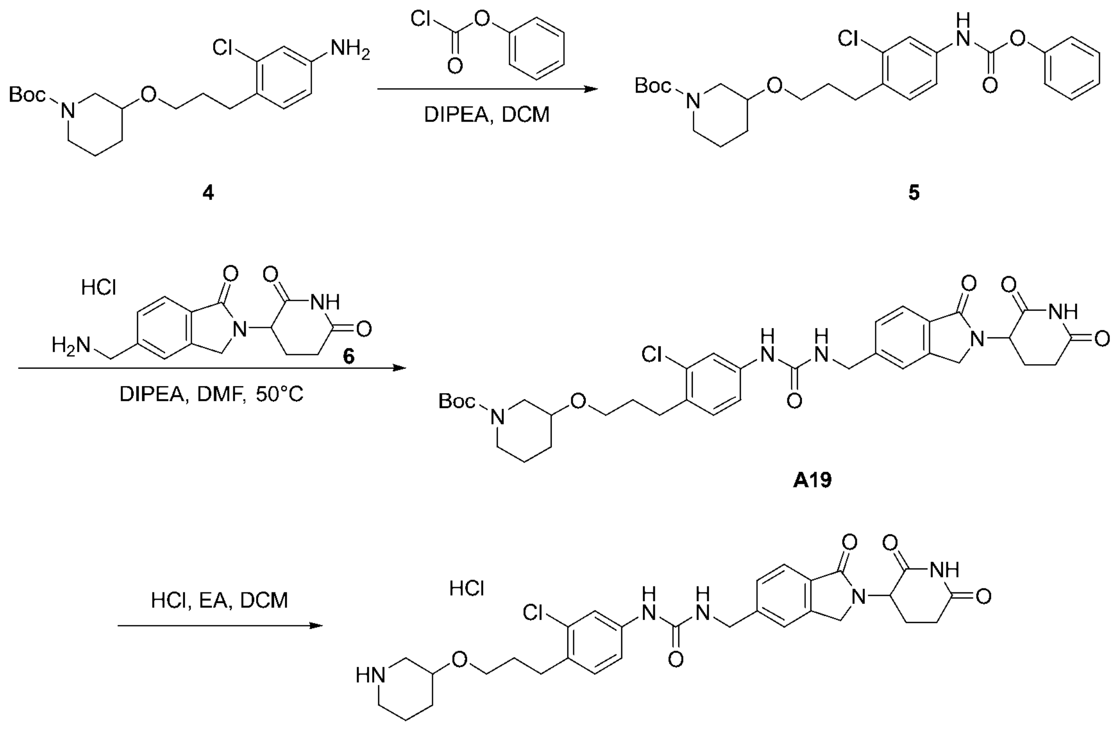


4

5

6

A19

B19

Compound 4 (13.5538 mmol) was dissolved in dichloromethane (90 mL), and DIPEA (3.5 g, 27.1076 mmol) and phenyl chloroformate (16.264 mmol) were sequentially added to the solution. The mixture was stirred for reaction at room temperature. After 30 min, the reaction progress was monitored by TLC. After the reaction was completed, the reaction solution was concentrated under reduced pressure, and the residue was purified by silica gel column chromatography to give compound 5 (12.07 mmol, yield: 89%). LC-MS (ESI): $(M+H-Boc)^+$ calculated 389.2, found 389.2.

Compound 5 (10.225 mmol) was dissolved in DMF (80 mL), and then compound 6 (11.247 mmol) was added. After the solid was dispersed in the solution by ultrasonication, DIPEA (40.9 mmol) was added. The mixture was heated to

50 °C and reacted. After 3 h, the reaction progress was monitored by TLC and LCMS. After the reaction was completed, the reaction solution was extracted with ethyl acetate and a saturated sodium chloride solution. The organic phase was collected, dried over anhydrous sodium sulfate, and then filtered. The filtrate was concentrated under reduced pressure, and the residue was purified by silica gel column chromatography to give compound A19 (9.637 mmol, yield: 94%). LC-MS (ESI): $(M+H-Boc)^+$ calculated 568.2, found 568.4. HRMS (ESI): $(M+Na)^+$ calculated 690.2665, found 690.2607. $^1H$ NMR (400 MHz, DMSO-$d_6$) δ 10.98 (s, 1H), 8.75 (s, 1H), 7.69 (d, *J* = 7.8 Hz, 1H), 7.67 - 7.63 (m, 1H), 7.51 (s, 1H), 7.44 (dd, *J* = 7.9, 1.5 Hz, 1H), 7.19 - 7.11 (m, 2H), 6.79 (t, *J* = 6.0 Hz, 1H), 5.11 (dd, *J* = 13.3, 5.1 Hz, 1H), 4.50 - 4.37 (m, 3H), 4.31 (d, *J* = 17.3 Hz, 1H), 3.70 - 3.33 (m, 4H), 3.29 - 3.21 (m, 2H), 3.16 - 2.97 (m, 1H), 2.96 - 2.87 (m, 1H), 2.69 - 2.56 (m, 3H), 2.38 (qd, *J* = 13.1, 4.4 Hz, 1H), 2.04 - 1.95 (m, 1H), 1.86 - 1.68 (m, 3H), 1.67 - 1.58 (m, 1H), 1.54 - 1.42 (m, 1H), 1.37 (s, 9H), 1.30 (t, *J* = 5.8 Hz, 1H). Compound A19 (2.7986 mmol) was dissolved in dichloromethane (19 mL), and a solution of hydrogen chloride in ethyl acetate (44 mmol, 11 mL, 4 mmol/mL) was added dropwise to the solution in an ice bath for reaction. A large amount of solid was observed to form as the solution was added dropwise. Two hours later, the reaction progress was monitored by TLC and LCMS. After the reaction was completed, the reaction solution was concentrated under reduced pressure at a low temperature to remove the solvent, and the residue was dried in vacuum to give compound B19 (2.647 mmol, yield: 94%). LC-MS (ESI): $(M+H)^+$ calculated 568.2, found 568.3. HRMS (ESI): $(M+H)^+$ calculated 568.2321, found 568.2340. $^1H$ NMR (400 MHz, DMSO-$d_6$) δ 10.98 (s, 1H), 9.16 (s, 1H), 9.09 - 8.88 (m, 1H), 8.67 - 8.38 (m, 1H), 7.69 (d, *J* = 7.8 Hz, 1H), 7.65 (d, *J* = 1.9 Hz, 1H), 7.51 (s, 1H), 7.44 (dd, *J* = 7.8, 1.4 Hz, 1H), 7.23 - 7.14 (m, 2H), 7.05 (t, *J* = 6.1 Hz, 1H), 5.11 (dd, *J* = 13.3, 5.1 Hz, 1H), 4.50 - 4.36 (m, 3H), 4.31 (d, *J* = 17.3 Hz, 1H), 3.61 (dt, *J* = 6.5, 3.4 Hz, 1H), 3.56 - 3.47 (m, 1H), 3.42 - 3.34 (m, 1H), 3.19 - 3.10 (m, 1H), 3.00 - 2.86 (m, 4H), 2.71 - 2.63 (m, 2H), 2.63 - 2.54 (m, 1H), 2.38 (qd, *J* = 13.1, 4.3 Hz, 1H), 2.04 - 1.99 (m, 1H), 1.87 - 1.69 (m, 4H), 1.69 - 1.52 (m, 2H).

Compounds A20-A24 and B20-B24 were prepared by replacing the starting material compound 1 with the corresponding Boc-protected piperidin-3-ylalkyl alcohols, using the same preparation method as that for compounds A19 and B19.

| N-1 Compound | MS: $(M+H)^+$ calculated | MS: $(M+H)^+$ found | Compound | MS: $(M+H)^+$ calculated | MS: $(M+H)^+$ found |
|---|---|---|---|---|---|
| A20 | 682.2 | 682.4 | B20 | 582.2 | 582.5 |
| A21 | 696.3 | 696.5 | B21 | 596.3 | 596.5 |
| A22 | 710.3 | 710.6 | B22 | 610.3 | 610.5 |
| A23 | 724.3 | 724.6 | B23 | 624.3 | 624.6 |
| A24 | 738.3 | 738.6 | B24 | 638.3 | 638.5 |

Example 3: Synthesis of Compounds B1-B6

| Structural formula | m | N | N-1 Compound | Compound |
|---|---|---|---|---|
| 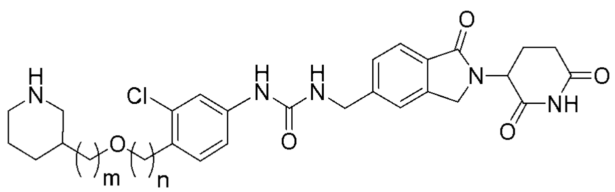 | 0 | 0 | A1 | B1 |
| | 1 | 0 | A2 | B2 |
| | 2 | 0 | A3 | B3 |
| | 3 | 0 | A4 | B4 |
| | 4 | 0 | A5 | B5 |
| | 5 | 0 | A6 | B6 |

The synthesis of compound B2 (m = 1, n = 0) was taken as an example

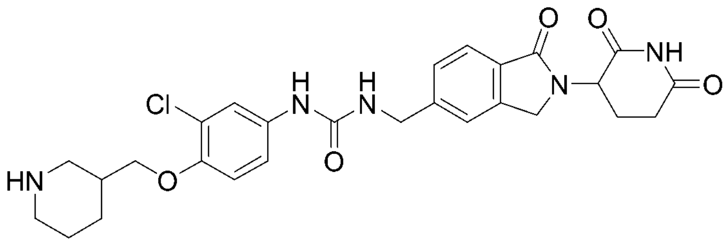

Synthesis of intermediate

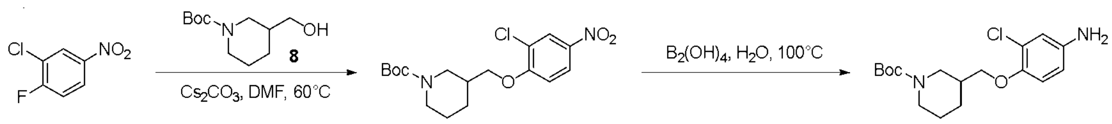


7 9 10

Compound 7 (4 mmol) and compound 8 (6 mmol) were dissolved in DMF (20 mL), and cesium carbonate (8 mmol) was added. The mixture was stirred at 60 °C overnight under nitrogen atmosphere. The reaction progress was monitored by TLC and LCMS. After the reaction was completed, a saturated ammonium chloride solution was added to quench the reaction, and the reaction solution was extracted with ethyl acetate and a saturated sodium chloride solution. The organic phase was collected, dried over anhydrous sodium sulfate, and then filtered. The filtrate was concentrated under reduced pressure, and the residue was purified by silica gel column chromatography to give compound 9 (3.44 mmol, yield: 86%). LC-MS (ESI): $(M+H)^+$ calculated 371.1, found 371.2.

Compound 9 (3 mmol) was dispersed in water (30 mL), and $B_2(OH)_4$ (15 mmol) was added. The mixture was heated to 100 °C and stirred overnight under nitrogen atmosphere. The reaction progress was monitored by TLC and LCMS. After the reaction was completed, the reaction solution was extracted with ethyl acetate and a saturated sodium chloride solution. The organic phase was collected, dried over anhydrous sodium sulfate, and then filtered. The filtrate was concentrated under reduced pressure, and the residue was purified by silica gel column chromatography to give compound 10 (2.34 mmol, yield: 78%). LC-MS (ESI): $(M+H)^+$ calculated 341.2, found 341.2.

Referring to the method in Example 2, compound A2 was obtained by replacing compound 4 with compound 10. LC-MS (ESI): $(M+H)^+$ calculated 640.2, found 640.4.

Referring to the method in Example 2, compound B2 was obtained by replacing compound 4 with compound 10. LC-MS (ESI): $(M+H)^+$ calculated 540.2, found 540.5.

Compounds A1 and A3-A6 as well as B1 and B3-B6 were prepared by replacing the starting material compound 8 with the corresponding Boc-protected piperidin-3-ylalkyl alcohols, using the same preparation method as that for compounds A2 and B2.

| N-1 Compound | MS: $(M+H)^+$ calculated | MS: $(M+H)^+$ found | Compound | MS: $(M+H)^+$ calculated | MS: $(M+H)^+$ found |
|---|---|---|---|---|---|
| A1 | 626.2 | 626.3 | B1 | 526.2 | 526.3 |
| A3 | 654.2 | 654.4 | B3 | 554.2 | 554.4 |
| A4 | 668.2 | 668.5 | B4 | 568.2 | 568.5 |
| A5 | 682.2 | 682.5 | B5 | 582.2 | 582.4 |
| A6 | 696.3 | 696.6 | B6 | 596.3 | 596.5 |

Example 4: Synthesis of Compounds B7-B12

| Structural formula | m | n | N-1 Compound | Compound |
|---|---|---|---|---|
| 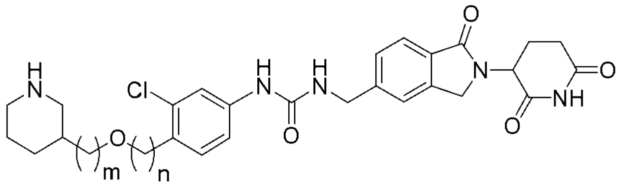 | 0 | 1 | A7 | B7 |
| | 1 | 1 | A8 | B8 |
| | 2 | 1 | A9 | B9 |
| | 3 | 1 | A10 | B10 |
| | 4 | 1 | A11 | B11 |
| | 5 | 1 | A12 | B12 |

The synthesis of compound B9 (m = 2, n = 1) was taken as an example

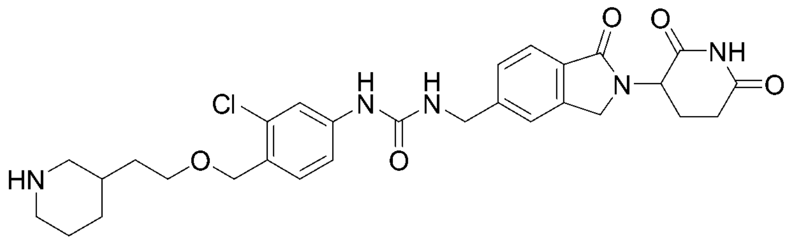

Synthesis of intermediate

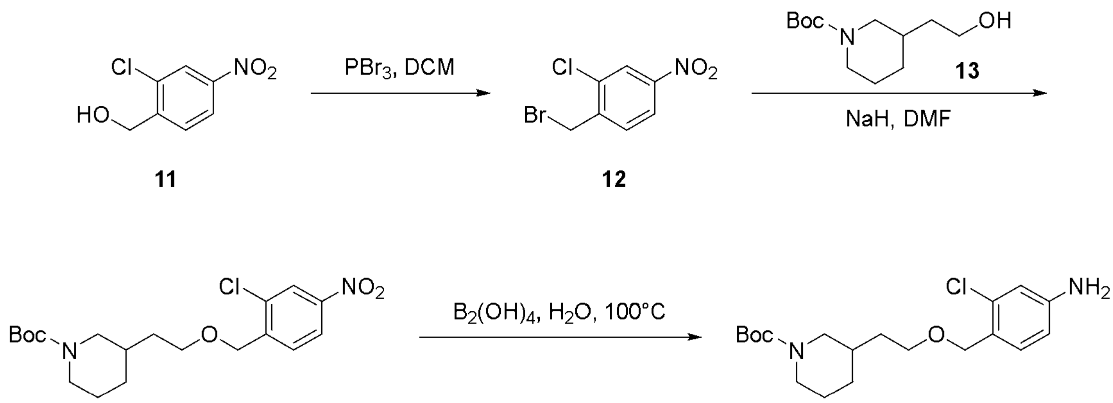


11 12 13 14 15

Compound 11 (4 mmol) was dissolved in DCM (10 mL), and phosphorus tribromide (8 mmol) was added under nitrogen atmosphere. The mixture was stirred for reaction. After 2 h, the reaction was monitored by TLC and LCMS. After the reaction was completed, the reaction was quenched with water, and extracted with ethyl acetate and a saturated sodium chloride solution. The organic phases were combined, dried over anhydrous sodium sulfate, and then filtered. The filtrate was concentrated under reduced pressure, and the residue was purified by silica gel column chromatography to give compound 12 (2.84 mmol, yield: 71%).

Compound 13 (5.0 mmol) was dissolved in DMF (10 mL), and sodium hydride (5 mmol, 60%) was added in an ice bath under nitrogen atmosphere. The mixture was stirred for reaction for half an hour. A solution of compound 12 (2.5 mmol) in DMF (5 mL) was added to the reaction system. The mixture was stirred for reaction for 3 h. The reaction was detected by TLC and LCMS. After the reaction was completed, a saturated ammonium chloride solution was added to quench the reaction, and the reaction solution was extracted with ethyl acetate and a saturated sodium chloride solution. The organic phases were combined, dried over anhydrous sodium sulfate, and then filtered. The filtrate was concentrated under reduced pressure, and the residue was purified by silica gel column chromatography to give compound 14 (2.15 mmol, yield: 86%). LC-MS (ESI): $(M+H)^+$ calculated 399.2, found 399.3.

Compound 14 (2 mmol) was dispersed in water (5 mL), and $B_2(OH)_4$ (10 mmol) was added. The mixture was heated to 100 °C and stirred overnight under nitrogen atmosphere. The reaction progress was detected by TLC and LCMS. After the reaction was completed, the reaction solution was extracted with ethyl acetate and a saturated sodium chloride

solution. The organic phase was collected, dried over anhydrous sodium sulfate, and then filtered. The filtrate was concentrated under reduced pressure, and the residue was purified by silica gel column chromatography to give compound 15 (1.74 mmol, yield: 87%). LC-MS (ESI): $(M+H)^+$ calculated 369.2, found 369.3.

Referring to the method in Example 2, compound A9 was obtained by replacing compound 4 with compound 15. LC-MS (ESI): $(M+H)^+$ calculated 668.2, found 668.5.

Referring to the method in Example 2, compound B9 was obtained by replacing compound 4 with compound 15. LC-MS (ESI): $(M+H)^+$ calculated 568.2, found 568.6.

Compounds A7-A8, A10-A12, B7-B8, and B10-B12 were prepared by replacing the starting material compound 13 with the corresponding Boc-protected piperidin-3-ylalkyl alcohols, using the same preparation method as that for compounds A9 and B9.

| N-1 Compound | MS: $(M+H)^+$ calculated | MS: $(M+H)^+$ found | Compound | MS: $(M+H)^+$ calculated | MS: $(M+H)^+$ found |
|---|---|---|---|---|---|
| A7 | 640.2 | 640.4 | B7 | 540.2 | 540.4 |
| A8 | 654.2 | 654.5 | B8 | 554.2 | 554.4 |
| A10 | 682.2 | 682.4 | B10 | 582.2 | 582.5 |
| A11 | 696.3 | 696.6 | B11 | 596.3 | 596.5 |
| A12 | 710.3 | 710.6 | B12 | 610.3 | 610.5 |

Example 5: Synthesis of Compounds B13-B18

| Structural formula | m | n | N-1 Compound | Compound |
|---|---|---|---|---|
| 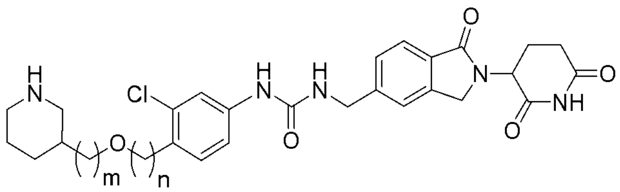 | 0 | 2 | A13 | B13 |
| | 1 | 2 | A14 | B14 |
| | 2 | 2 | A15 | B15 |
| | 3 | 2 | A16 | B16 |
| | 4 | 2 | A17 | B17 |
| | 5 | 2 | A18 | B18 |

The synthesis of compound B13 (m = 0, n = 2) was taken as an example

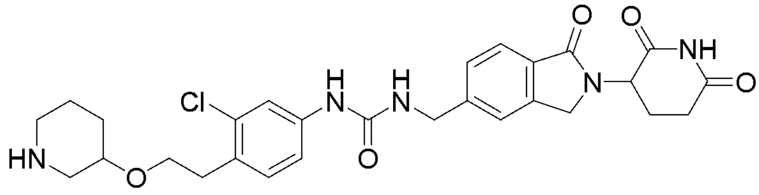

Synthesis of intermediate

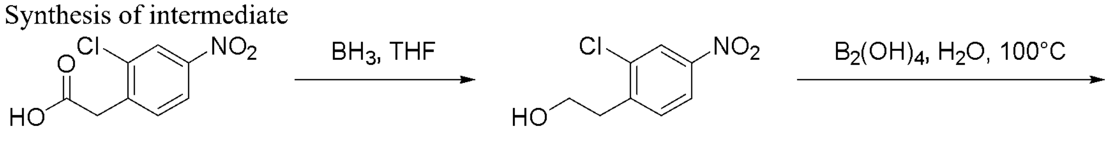


16 17

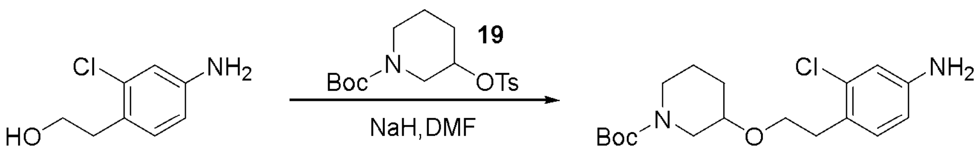


18 20

Compound 16 (4.638 mmol) was dissolved in THF (10 mL), and a borane-tetrahydrofuran solution (13.5 mL, 1.0 mmol/mL, 13.5 mmol) was added in an ice bath under nitrogen atmosphere. The mixture was stirred for reaction overnight. The reaction progress was detected by TLC and LCMS. After the reaction was completed, the reaction solution was heated to room temperature, and methanol was added dropwise in an ice bath to quench the reaction. The reaction solution was extracted with ethyl acetate and a saturated sodium chloride solution. The organic phase was collected, dried over anhydrous sodium sulfate, and then filtered. The filtrate was concentrated under reduced pressure, and the residue was purified by silica gel column chromatography to give compound 17 (4.524 mmol). LC-MS (ESI): $(M+H)^+$ calculated 202.0, found 202.1.

Compound 17 (1 mmol) was dispersed in water (5 mL), and $B_2(OH)_4$ (5 mmol) was added. The mixture was stirred for reaction at 100 °C under nitrogen atmosphere. The reaction progress was detected by TLC and LCMS. After the reaction was completed, the reaction solution was cooled to room temperature and extracted with ethyl acetate and a saturated sodium chloride solution. The organic phase was collected, dried over anhydrous sodium sulfate, and then filtered. The filtrate was concentrated under reduced pressure, and the residue was purified by silica gel column chromatography to give compound 18 (0.92 mmol, yield: 92%). LC-MS (ESI): $(M+H)^+$ calculated 172.0, found 172.1.

Compound 18 (0.5 mmol) was dissolved in DMF (5 mL), and sodium hydride (0.5 mmol, 60%) was added in an ice bath under nitrogen atmosphere. The mixture was stirred for reaction for half an hour, and then a solution of compound 19 (0.5 mmol) in DMF (2 mL) was added. The mixture was stirred for reaction overnight. The reaction progress was detected by TLC and LCMS. After the reaction was completed, a saturated ammonium chloride solution was added to quench the reaction, and the reaction solution was extracted with ethyl acetate and a saturated sodium chloride solution. The organic phases were combined, dried over anhydrous sodium sulfate, and then filtered. The filtrate was concentrated under reduced pressure, and the residue was purified by silica gel column chromatography to give compound 20 (0.16 mmol, yield: 32%). LC-MS (ESI): $(M+H)^+$ calculated 355.2, found 355.2.

Referring to the method in Example 2, compound A13 was obtained by replacing compound 4 with compound 20. LC-MS (ESI): $(M+H)^+$ calculated 654.2, found 654.5.

Referring to the method in Example 2, compound B13 was obtained by replacing compound 4 with compound 20. LC-MS (ESI): $(M+H)^+$ calculated 554.2, found 554.4.

Compounds A14-A18 and B14-B18 were prepared by replacing the starting material compound 13 with the corresponding piperidin-3-ylalkyl alcohols protected by Boc or Ts, using the same preparation method as that for compounds A13 and B13.

| N-1 Compound | MS: $(M+H)^+$ calculated | MS: $(M+H)^+$ found | Compound | MS: $(M+H)^+$ calculated | MS: $(M+H)^+$ found |
|---|---|---|---|---|---|
| A14 | 668.2 | 668.5 | B14 | 568.2 | 568.5 |
| A15 | 682.2 | 682.5 | B15 | 582.2 | 582.4 |

(continued)

| N-1 Compound | MS: (M+H)+ calculated | MS: (M+H)+ found | Compound | MS: (M+H)+ calculated | MS: (M+H)+ found |
|---|---|---|---|---|---|
| A16 | 696.3 | 696.5 | B16 | 596.3 | 596.5 |
| A17 | 710.3 | 710.6 | B17 | 610.3 | 610.5 |
| A18 | 724.3 | 724.6 | B18 | 624.3 | 624.5 |

Example 6: Synthesis of Compounds B25-B42

| Structural formula | m | n | N-1 Compound | Compound |
|---|---|---|---|---|
| 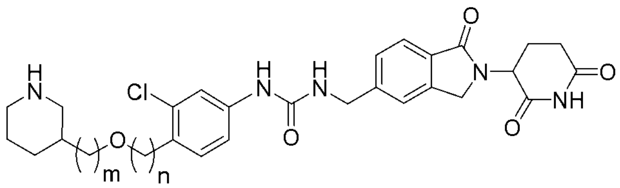 | 0 | 4 | A25 | B25 |
| | 1 | 4 | A26 | B26 |
| | 2 | 4 | A27 | B27 |
| | 3 | 4 | A28 | B28 |
| | 4 | 4 | A29 | B29 |
| | 5 | 4 | A30 | B30 |
| | 0 | 5 | A31 | B31 |
| | 1 | 5 | A32 | B32 |
| | 2 | 5 | A33 | B33 |
| | 3 | 5 | A34 | B34 |
| | 4 | 5 | A35 | B35 |
| | 5 | 5 | A36 | B36 |
| | 0 | 6 | A37 | B37 |
| | 1 | 6 | A38 | B38 |
| | 2 | 6 | A39 | B39 |
| | 3 | 6 | A40 | B40 |
| | 4 | 6 | A41 | B41 |
| | 5 | 6 | A42 | B42 |

The synthesis of compound B30 (m = 5, n = 4) was taken as an example

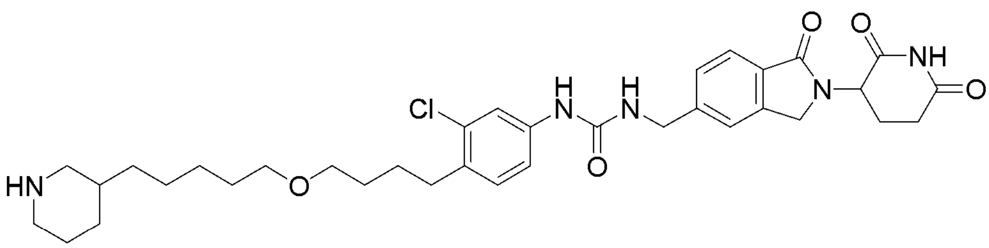

Synthesis of intermediate

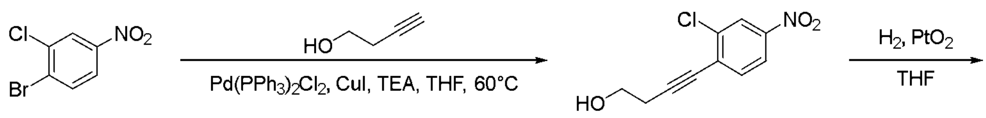


21 22

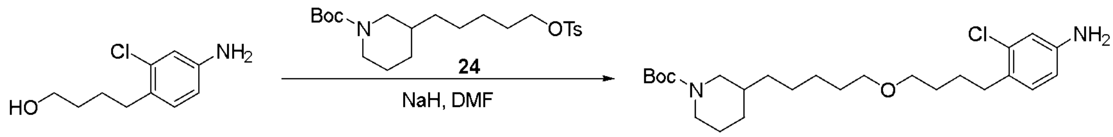


23 25

Compound 21 (10 mmol), copper(I) iodide (1 mmol), triethylamine (5 mL), and $Pd(PPh_3)_2Cl_2$ (0.5 mmol) were dissolved in THF (20 mL), and 3-butyn-1-ol (0.757 mL, 10 mmol) was added to the reaction system under nitrogen atmosphere. The mixture was heated to 60 °C and stirred for reaction. After 4 h of reaction, the reaction progress was detected by TLC and LCMS. After the reaction was completed, the solid in the reaction system was removed by filtration. The filtrate was concentrated under reduced pressure, and the residue was extracted with ethyl acetate and a saturated sodium chloride solution. The organic phases were combined, dried over anhydrous sodium sulfate, and then filtered. The filtrate was concentrated under reduced pressure, and the residue was purified by silica gel column chromatography to give compound 22 (8.9 mmol, yield: 89%). LC-MS (ESI): $(M+H)^+$ calculated 226.0, found 226.0.

Compound 22 (8 mmol) was dissolved in THF (20 mL), and platinum dioxide (0.125 g) was added under nitrogen atmosphere. The mixture was purged with hydrogen and then stirred for reaction. The reaction progress was detected by TLC and LCMS. After the reaction was completed, celite was added, and the solid was removed by filtration.

The filtrate was concentrated under reduced pressure, and the residue was purified by silica gel column chromatography to give compound 23 (7.36 mmol, yield: 92%). LC-MS (ESI): $(M+H)^+$ calculated 200.1, found 200.1.

Compound 23 (5 mmol) was dissolved in DMF (50 mL), and sodium hydride (5 mmol, 60%) was added in an ice bath under nitrogen atmosphere. The mixture was stirred for reaction for half an hour, and then a solution of compound 24 (5 mmol) in DMF (10 mL) was added. The mixture was stirred for reaction overnight. The reaction progress was detected by TLC and LCMS. After the reaction was completed, a saturated ammonium chloride solution was added to quench the reaction, and the reaction solution was extracted with ethyl acetate and a saturated sodium chloride solution. The organic phases were combined, dried over anhydrous sodium sulfate, and then filtered. The filtrate was concentrated under reduced pressure, and the residue was purified by silica gel column chromatography to give compound 25 (1.49 mmol, yield: 30%). LC-MS (ESI): $(M+H)^+$ calculated 453.3, found 453.5.

Referring to the method in Example 2, compound A30 was obtained by replacing compound 4 with compound 25. LC-MS (ESI): $(M+H)^+$ calculated 752.3, found 752.6.

Referring to the method in Example 2, compound B30 was obtained by replacing compound 4 with compound 25. LC-MS (ESI): $(M+H)^+$ calculated 652.3, found 652.6.

Compounds A25-A29 and A31-A42 as well as B25-B29 and B31-B42 were prepared by replacing the starting materials 3-butyn-1-ol and compound 24 with 4-pentyn-1-ol or 6-hexyn-1-ol and the corresponding piperidin-3-yl alkyl alcohols protected by Boc and Ts, respectively, using the same preparation method as that for compounds A30 and B30.

| N-1 Compound | MS: $(M+H)^+$ calculated | MS: $(M+H)^+$ found | Compound | MS: $(M+H)^+$ calculated | MS: $(M+H)^+$ found |
|---|---|---|---|---|---|
| A25 | 682.2 | 682.5 | B25 | 582.2 | 582.4 |
| A26 | 696.3 | 696.5 | B26 | 596.3 | 596.5 |
| A27 | 710.3 | 710.5 | B27 | 610.3 | 610.5 |
| A28 | 724.3 | 724.6 | B28 | 624.3 | 624.6 |
| A29 | 738.3 | 738.6 | B29 | 638.3 | 638.6 |
| A31 | 696.3 | 696.5 | B31 | 596.3 | 596.5 |
| A32 | 710.3 | 710.6 | B32 | 610.3 | 610.5 |
| A33 | 724.3 | 724.5 | B33 | 624.3 | 624.5 |
| A34 | 738.3 | 738.5 | B34 | 638.3 | 638.6 |

(continued)

| N-1 Compound | MS: $(M+H)^+$ calculated | MS: $(M+H)^+$ found | Compound | MS: $(M+H)^+$ calculated | MS: $(M+H)^+$ found |
|---|---|---|---|---|---|
| A35 | 752.3 | 752.6 | B35 | 652.3 | 652.6 |
| A36 | 766.3 | 766.6 | B36 | 666.3 | 666.6 |
| A37 | 710.3 | 710.6 | B37 | 610.3 | 610.5 |
| A38 | 724.3 | 724.5 | B38 | 624.3 | 624.5 |
| A39 | 738.3 | 738.5 | B39 | 638.3 | 638.6 |
| A40 | 752.3 | 752.6 | B40 | 652.3 | 652.6 |
| A41 | 766.3 | 766.8 | B41 | 666.3 | 666.6 |
| A42 | 780.4 | 780.7 | B42 | 680.4 | 680.7 |

Example 7: Synthesis of Compounds B43-B54

| Structural formula | $R_2$ | $L_1$ | N-1 Compo und | Compo und |
|---|---|---|---|---|
| 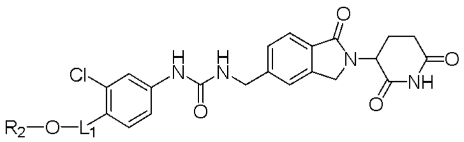 | 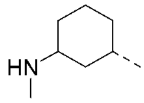 | 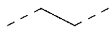 | A43 | B43 |
| | 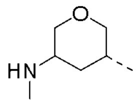 | 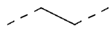 | A44 | B44 |
| | 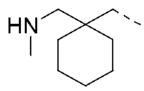 | 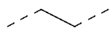 | A45 | B45 |
| | 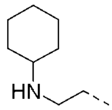 | 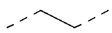 | A46 | B46 |
| | 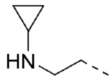 | 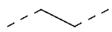 | A47 | B47 |
| | 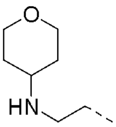 | 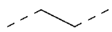 | A48 | B48 |
| | 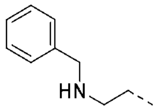 | 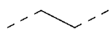 | A49 | B49 |
| | 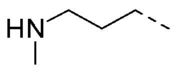 | 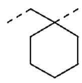 | A50 | B50 |
| | 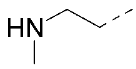 | 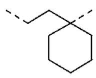 | A51 | B51 |
| | 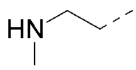 | 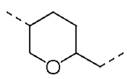 | A52 | B52 |
| | 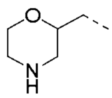 | 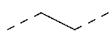 | A53 | B53 |
| | 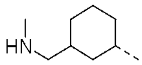 | 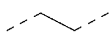 | A54 | B54 |

The synthesis of compound B48 was taken as an example

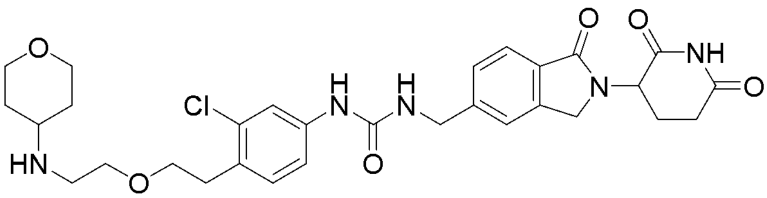

Synthesis of intermediate

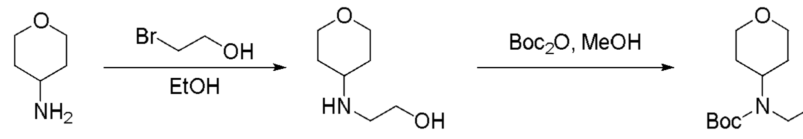


26 27 28

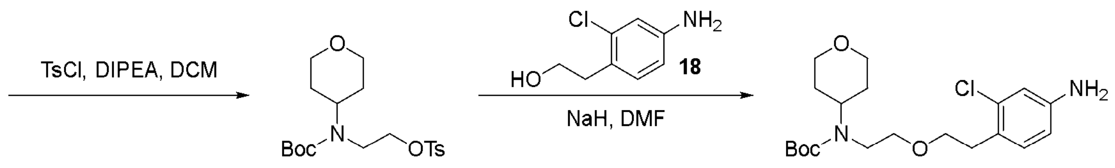


29 30

Compound 26 (20 mmol) was dissolved in dichloromethane (50 mL), and bromoethanol (21 mmol) was added to the reaction system. The mixture was reacted at 45 °C overnight under nitrogen atmosphere. The reaction was detected by TLC and LCMS. After the reaction was completed and the reaction solution was cooled to room temperature, ethyl acetate (200 mL) was added to the reaction system, and the mixture was stirred for two hours. Then the reaction system was stirred in an ice bath for 3 h . The solid was collected by filtration and washed with ethyl acetate to give hydrobromide of compound 27 (17.41 mmol, yield: 87%). LC-MS (ESI): $(M+H)^+$ calculated 146.1, found 146.1.

The hydrobromide of compound 27 (17.41 mmol) was dissolved in methanol (40 mL), and TEA (18 mmol) and $Boc_2O$ (18 mmol) were added. The mixture was stirred for reaction. The reaction progress was detected by TLC and LCMS. After the reaction was completed, the reaction solution was extracted with ethyl acetate and a saturated sodium chloride solution. The organic phases were combined, dried over anhydrous sodium sulfate, and then filtered. The filtrate was concentrated under reduced pressure, and the residue was purified by silica gel column chromatography to give compound 28 (16.89 mmol, yield: 97%). LC-MS (ESI): $(M+H)^+$ calculated 246.2, found 246.4.

Compound 28 (16.89 mmol) was dissolved in DCM (50 mL), and DIPEA (34 mmol) and TsCl (34 mmol) were added to the reaction system. The mixture was stirred for reaction overnight. The reaction progress was detected by TLC and LCMS. After the reaction was completed, the reaction solution was extracted with ethyl acetate and a saturated sodium chloride solution. The organic phases were combined, dried over anhydrous sodium sulfate, and then filtered. The filtrate was concentrated under reduced pressure, and the residue was purified by silica gel column chromatography to give compound 29 (14.69 mmol, yield: 87%). LC-MS (ESI): $(M+H)^+$ calculated 400.2, found 400.3.

Compound 18 (2 mmol) was dissolved in DMF (10 mL), and sodium hydride (2 mmol, 60%) was added in an ice bath under nitrogen atmosphere. The mixture was stirred for reaction for half an hour, and then a solution of compound 29 (2 mmol) in DMF (2 mL) was added. The mixture was stirred for reaction overnight. The reaction progress was detected by TLC and LCMS. After the reaction was completed, a saturated ammonium chloride solution was added to quench the reaction, and the reaction solution was extracted with ethyl acetate and a saturated sodium chloride solution. The organic phases were combined, dried over anhydrous sodium sulfate, and then filtered. The filtrate was concentrated under reduced pressure, and the residue was purified by silica gel column chromatography to give compound 30 (0.46 mmol, yield: 23%). LC-MS (ESI): $(M+H)^+$ calculated 399.2, found 399.4.

Referring to the method in Example 2, compound A48 was obtained by replacing compound 4 with compound 30. LC-MS (ESI): $(M+H)^+$ calculated 698.2, found 698.4.

Referring to the method in Example 2, compound B48 was obtained by replacing compound 4 with compound 30. LC-MS (ESI): $(M+H)^+$ calculated 598.2, found 598.4.

Referring to the synthetic steps and methods of compounds A48 and B48, A43-A47, A49-A54, B43-B47, and B49-B54 were obtained by replacing compound 26 and compound 18 with different primary amines and alcohols.

| N-1 Compound | MS: $(M+H)^+$ calculated | MS: $(M+H)^+$ found | Compound | MS: $(M+H)^+$ calculated | MS: $(M+H)^+$ found |
|---|---|---|---|---|---|
| A43 | 682.2 | 682.3 | B43 | 582.2 | 582.3 |
| A44 | 684.2 | 684.4 | B44 | 584.2 | 584.4 |

(continued)

| N-1 Compound | MS: (M+H)+ calculated | MS: (M+H)+ found | Compound | MS: (M+H)+ calculated | MS: (M+H)+ found |
|---|---|---|---|---|---|
| A45 | 710.3 | 710.5 | B45 | 610.3 | 610.5 |
| A46 | 696.3 | 696.5 | B46 | 596.3 | 596.5 |
| A47 | 654.2 | 654.5 | B47 | 554.2 | 554.5 |
| A49 | 704.2 | 704.4 | B49 | 604.2 | 604.4 |
| A50 | 710.3 | 710.5 | B50 | 610.3 | 610.5 |
| A51 | 710.3 | 710.6 | B51 | 610.3 | 610.6 |
| A52 | 698.2 | 698.5 | B52 | 598.2 | 598.5 |
| A53 | 670.2 | 670.4 | B53 | 570.2 | 570.4 |
| A54 | 696.3 | 696.6 | B54 | 596.3 | 596.6 |

Example 8: Synthesis of Compounds B55-B72

| Structural formula | $R_2$ | N-1 Compound | Compound |
|---|---|---|---|
| 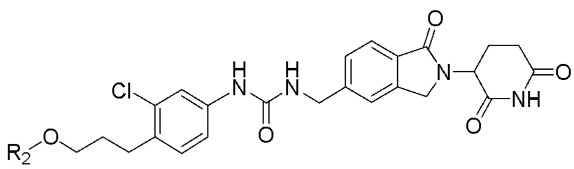 | 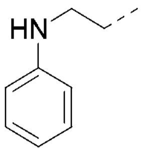 | A55 | B55 |
| | 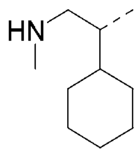 | A56 | B56 |
| | 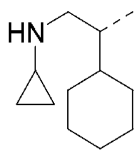 | A57 | B57 |
| | 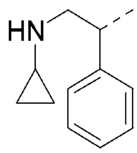 | A58 | B58 |
| | 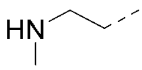 | A59 | B59 |
| | 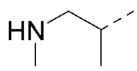 | A60 | B60 |

(continued)

| Structural formula | $R_2$ | N-1 Compound | Compound |
|---|---|---|---|
| | 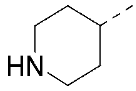 | A61 | B61 |
| | 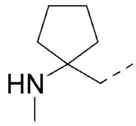 | A62 | B62 |
| | 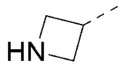 | A63 | B63 |
| | 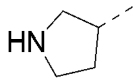 | A64 | B64 |
| | 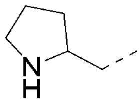 | A65 | B65 |
| | 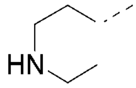 | A66 | B66 |
| | 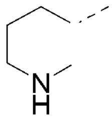 | A67 | B67 |
| | 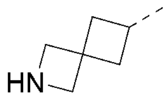 | A68 | B68 |
| | 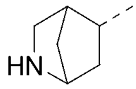 | A69 | B69 |
| | 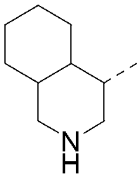 | A70 | B70 |
| | 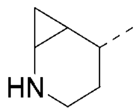 | A71 | B71 |
| | 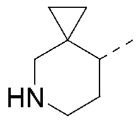 | A72 | B72 |

The synthesis of compound B59 was taken as an example

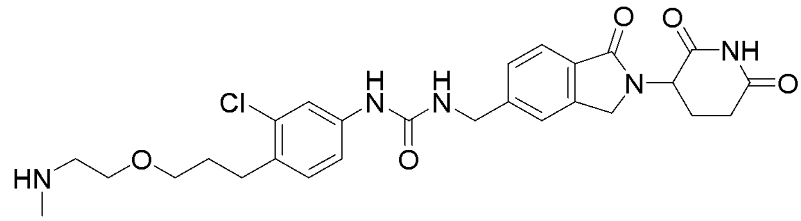

Synthesis of intermediate:

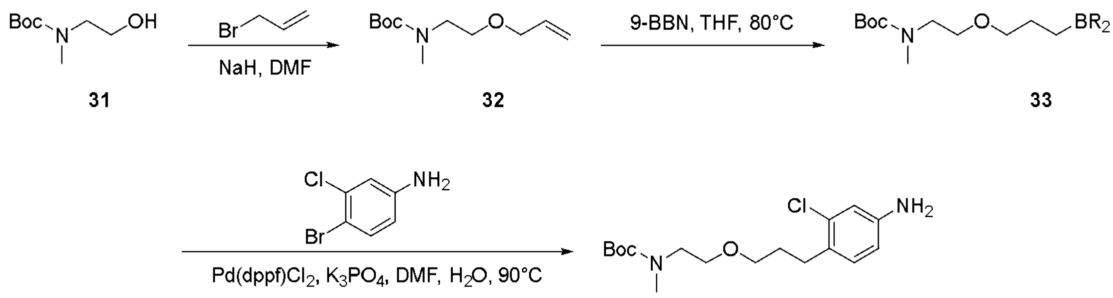


31 32 33

34

Compound 31 (5 mmol) was dissolved in DMF (20 mL), and sodium hydride (10 mmol, 60%) was added in an ice bath under nitrogen atmosphere. The mixture was stirred for half an hour. Allyl bromide (10 mmol) was added to the reaction system under nitrogen atmosphere. After two hours, the reaction progress was detected by TLC. After the reaction was completed, a saturated ammonium chloride solution was added in an ice bath to quench the reaction. The reaction solution was extracted with methyl tert-butyl ether and a saturated sodium chloride solution. The organic phases were combined, dried over anhydrous sodium sulfate, and then filtered. The filtrate was concentrated under reduced pressure, and the residue was purified by silica gel column chromatography to give compound 32 (4.3 mmol, yield: 86%). LC-MS (ESI): $(M+H)^+$ calculated 216.2, found 216.4.

A solution of 9-BBN (3.0 mL, 1.5 mmol, 0.5 mmol/mL) in tetrahydrofuran was added to a solution of compound 32 (1.0 mmol) in tetrahydrofuran in an ice bath under nitrogen atmosphere. The mixture was heated to 80 °C and reacted. After the mixture was reacted overnight, the reaction progress was detected by TLC. After the reaction was completed, the reaction solution was cooled to room temperature, and the reaction solution containing compound 33 was directly used in the next step.

DMF (10 mL) and a solution of potassium phosphate (1.25 mmol) in water (3 mL) were added to a solution of 33 under nitrogen atmosphere. The mixture was stirred for 10 min, and then 3-chloro-4-bromoaniline (1.0 mmol) and Pd(dppf)$Cl_2$ (0.1 mmol) were added under nitrogen atmosphere. The mixture was heated to 90 °C and reacted overnight. The reaction progress was detected by TLC and LCMS. After the starting materials were completely consumed, the reaction solution was cooled to room temperature and extracted with ethyl acetate and a saturated ammonium chloride solution. Insoluble substances in the system were removed by filtration, and then the organic phase was extracted with a saturated sodium chloride solution. The organic phase was collected, dried over anhydrous sodium sulfate, and then filtered. The filtrate was concentrated under reduced pressure, and the residue was purified by silica gel column chromatography to give compound 34 (0.43 mmol, yield: 43%). LC-MS (ESI): $(M+H)^+$ calculated 342.2, found 342.4.

Referring to the method in Example 2, compound A59 was obtained by replacing compound 4 with compound 34. LC-MS (ESI): $(M+H)^+$ calculated 642.2, found 642.4. HRMS (ESI): $(M+Na)^+$ calculated 664.2508, found 664.2509. $^1H$ NMR (400 MHz, DMSO-$d_6$) δ 10.96 (s, 1H), 8.77 (s, 1H), 7.69 (d, *J* = 7.8 Hz, 1H), 7.65 (d, *J* = 1.7 Hz, 1H), 7.51 (s, 1H), 7.47 - 7.41 (m, 1H), 7.20 - 7.10 (m, 2H), 6.80 (t, *J* = 6.0 Hz, 1H), 5.10 (dd, *J* = 13.3, 5.0 Hz, 1H), 4.50 - 4.37 (m, 3H), 4.31 (d, *J* = 17.3 Hz, 1H), 3.45 (t, *J* = 5.6 Hz, 2H), 3.38 (t, *J*= 6.2 Hz, 2H), 3.31 - 3.29 (m, 2H), 2.98 - 2.86 (m, 1H), 2.86 - 2.74 (m, 3H), 2.69 - 2.55 (m, 3H), 2.44 - 2.32 (m, 1H), 2.05 - 1.94 (m, 1H), 1.80 - 1.66 (m, 2H), 1.38 (s, 9H).

Referring to the method in Example 2, compound B59 was obtained by replacing compound 4 with compound 34. LC-MS (ESI): $(M+H)^+$ calculated 542.2, found 542.4. HRMS (ESI): $(M+H)^+$ calculated 542.2165, found 542.2174. $^1H$ NMR (400 MHz, DMSO-$d_6$) δ 10.98 (s, 1H), 9.35 (s, 1H), 7.68 (d, *J* = 7.8 Hz, 1H), 7.66 (d, *J* = 1.8 Hz, 1H), 7.52 - 7.51 (m, 1H), 7.45 - 7.44 (m, 1H), 7.20 - 7.15 (m, 3H), 5.10 (dd, *J* = 13.3, 5.1 Hz, 1H), 4.50 - 4.36 (m, 3H), 4.31 (d, *J* = 17.3 Hz, 1H), 3.63 (t, *J* = 5.2 Hz, 2H), 3.44 (t, *J* = 6.4 Hz, 2H), 3.07 (t, *J*= 5.2 Hz, 2H), 2.98 - 2.83 (m, 1H), 2.71 - 2.63 (m, 2H), 2.63 - 2.56 (m, 1H), 2.55 (s, 3H), 2.44 - 2.32 (m, 1H), 2.07 - 1.93 (m, 1H), 1.84 - 1.76 (m, 2H).

Referring to the synthetic steps and methods of compounds A59 and B59, A55-A72 and B55-B72 were obtained by replacing compound 31 with different alcohols.

| N-1 Compound | MS: $(M+H)^+$ calculated | MS: $(M+H)^+$ found | Compound | MS: $(M+H)^+$ calculated | MS: $(M+H)^+$ found |
|---|---|---|---|---|---|
| A55 | 704.2 | 704.5 | B55 | 604.2 | 604.5 |
| A56 | 724.3 | 724.6 | B56 | 624.3 | 624.4 |
| A57 | 750.3 | 750.6 | B57 | 650.3 | 650.5 |
| A58 | 744.3 | 744.5 | B58 | 644.3 | 644.5 |
| A60 | 656.2 | 656.5 | B60 | 556.2 | 556.5 |
| A61 | 668.2 | 668.5 | B61 | 568.2 | 568.4 |
| A62 | 696.3 | 696.6 | B62 | 596.3 | 596.6 |
| A63 | 640.2 | 640.4 | B63 | 540.2 | 540.4 |
| A64 | 654.2 | 654.3 | B64 | 554.2 | 554.4 |
| A65 | 668.2 | 668.6 | B65 | 568.2 | 568.4 |
| A66 | 682.2 | 682.4 | B66 | 582.2 | 582.5 |
| A67 | 682.2 | 682.5 | B67 | 582.2 | 582.5 |
| A68 | 680.2 | 680.5 | B68 | 580.2 | 580.4 |
| A69 | 680.2 | 680.5 | B69 | 580.2 | 580.4 |
| A70 | 722.3 | 722.6 | B70 | 622.3 | 622.6 |
| A71 | 680.2 | 680.5 | B71 | 580.2 | 580.5 |
| A72 | 694.2 | 694.4 | B72 | 594.2 | 594.5 |

Example 9: Synthesis of Compounds B73-B75

| Structural formula | $R_2$ | $L_1$ | N-1 Compound | Compound |
|---|---|---|---|---|
| 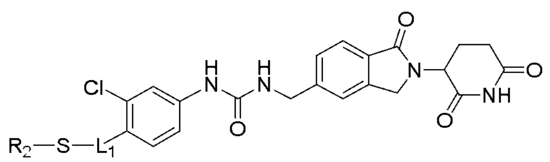 | 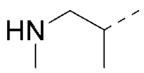 | 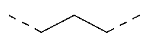 | A73 | B73 |
| | 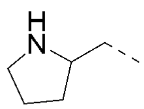 | 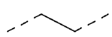 | A74 | B74 |
| | 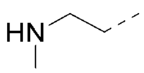 | 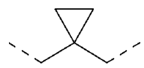 | A75 | B75 |

The synthesis of compound B75 was taken as an example

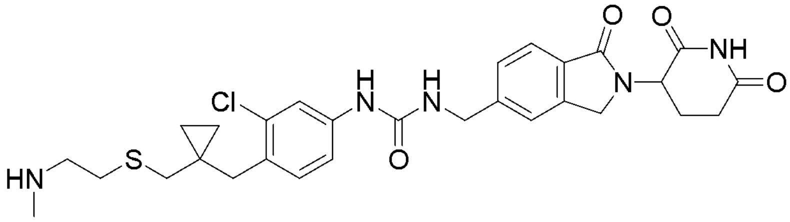

Synthesis of intermediate

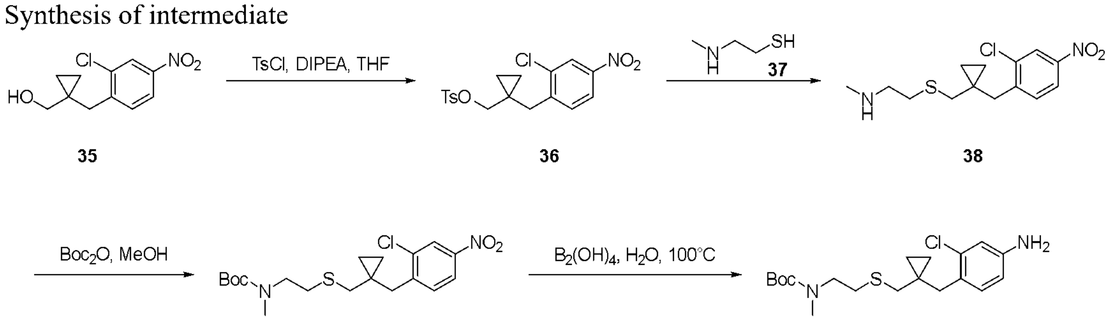


35 36 38

39 40

Compound 35 (5 mmol) was dissolved in THF (20 mL), and DIPEA (10 mmol) and TsCl (10 mmol) were added. The mixture was stirred for reaction overnight. The reaction progress was detected by TLC and LCMS. After the reaction was completed, the reaction solution was extracted with ethyl acetate and a saturated sodium chloride solution. The organic phases were combined, dried over anhydrous sodium sulfate, and then filtered. The filtrate was concentrated under reduced pressure, and the residue was purified by silica gel column chromatography to give compound 36 (3.7 mmol, yield: 74%). LC-MS (ESI): $(M+H)^+$ calculated 396.1, found 396.4.

Compound 36 (1 mmol) was dissolved in methanol (10 mL), and compound 37 (1.1 mmol) was added. The mixture was stirred for reaction. The reaction progress was detected by TLC and LCMS. After the reaction was completed, the reaction solution containing compound 38 was directly used in the next step without treatment. LC-MS (ESI): $(M+H)^+$ calculated 315.1, found 315.3.

$Boc_2O$ (2.0 mmol) was added to the reaction solution of compound 38. The mixture was stirred for reaction. After 2 h, the reaction progress was detected by TLC and LCMS. After the reaction was completed, the reaction solution was concentrated under reduced pressure, and the residue was purified by silica gel column chromatography to give compound 39 (0.84 mmol, yield: 84%). LC-MS (ESI): $(M+H)^+$ calculated 415.1, found 415.3.

Compound 39 (0.5 mmol) was dispersed in water (5 mL), and $B_2(OH)_4$ (5 mmol) was added. The mixture was heated to 100 °C and stirred overnight under nitrogen atmosphere. The reaction progress was detected by TLC and LCMS. After the reaction was completed, the reaction solution was extracted with ethyl acetate and a saturated sodium chloride solution. The organic phase was collected, dried over anhydrous sodium sulfate, and then filtered. The filtrate was concentrated under reduced pressure, and the residue was purified by silica gel column chromatography to give compound 40 (0.355 mmol, yield: 71%). LC-MS (ESI): $(M+H)^+$ calculated 385.2, found 385.3.

Referring to the method in Example 2, compound A75 was obtained by replacing compound 4 with compound 40. LC-MS (ESI): $(M+H)^+$ calculated 684.2, found 684.5.

Referring to the method in Example 2, compound B75 was obtained by replacing compound 4 with compound 40. LC-MS (ESI): $(M+H)^+$ calculated 584.2, found 584.5.

Referring to the synthetic steps and methods for compounds A75 and B75, A73-A74 and B73-B74 were obtained by replacing compound 35 and compound 37 with different alcohols and thiols, respectively.

| N-1 Compound | MS: $(M+H)^+$ calculated | MS: $(M+H)^+$ found | Compound | MS: $(M+H)^+$ calculated | MS: $(M+H)^+$ found |
|---|---|---|---|---|---|
| A73 | 672.2 | 672.4 | B73 | 572.2 | 572.4 |
| A74 | 670.2 | 670.5 | B74 | 570.2 | 570.5 |

Example 10: Synthesis of Compounds B76-B78

| Structural formula | $R_2$ | $L_1$ | N-1 Compound | Compound |
|---|---|---|---|---|
| 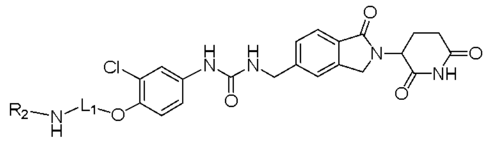 | 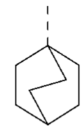 | 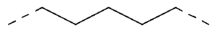 | A76 | B76 |
| | 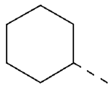 | 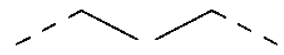 | A77 | B77 |
| | 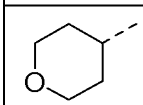 | 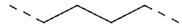 | A78 | B78 |

The synthesis of compound B76 was taken as an example

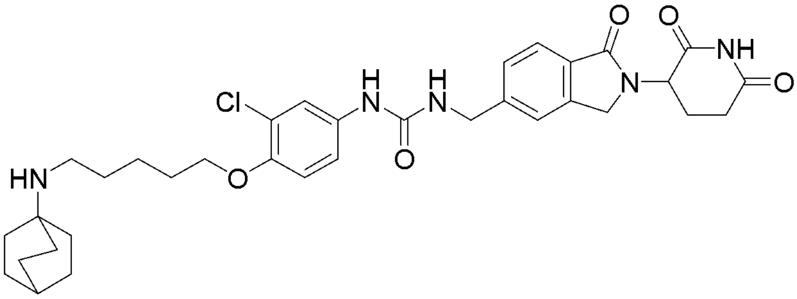

Synthesis of intermediate

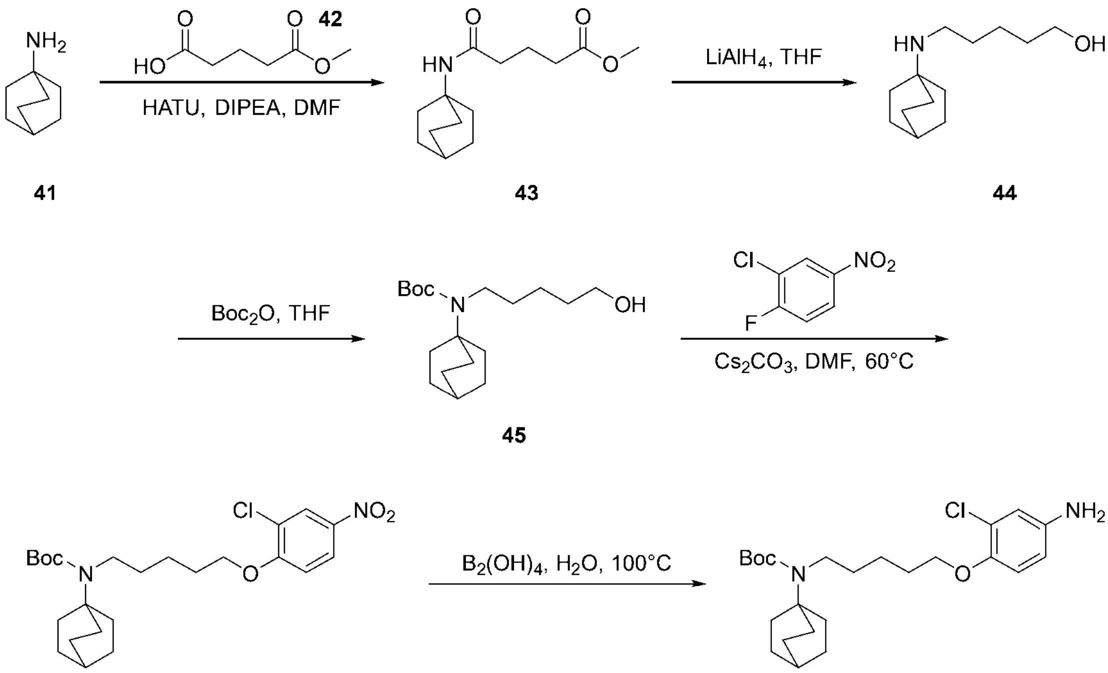


41 43 44

45

46 47

Compound 41 (5 mmol) and compound 42 (5 mmol) were dissolved in DMF (10 mL), and DIPEA (15 mmol) and HATU (6 mmol) were added. The mixture was stirred for reaction overnight. The reaction progress was detected by TLC

and LCMS. After the reaction was completed, the reaction solution was extracted with ethyl acetate and a saturated sodium carbonate solution. The organic phase was collected and extracted with a saturated sodium chloride solution. The organic phase was collected, dried over anhydrous sodium sulfate, and then filtered. The filtrate was concentrated under reduced pressure, and the residue was purified by silica gel column chromatography to give compound 43 (3.95 mmol, yield: 79%). LC-MS (ESI): $(M+H)^+$ calculated 254.2, found 254.3.

Compound 43 (3.95 mmol) was dissolved in THF (20 mL), and $LiAlH_4$ (16 mmol) was added in an ice bath under nitrogen atmosphere. The mixture was stirred for reaction at 60 °C. After 4 h, the reaction progress was detected by TLC and LCMS. After the reaction was completed, the reaction solution was cooled to room temperature, and an aqueous sodium hydroxide solution was added in an ice bath to quench the reaction. The solution after quenching was stirred for 2 h. The reaction solution containing compound 44 was directly used in the next step without further treatment. LC-MS (ESI): $(M+H)^+$ calculated 212.2, found 212.2.

$Boc_2O$ (5 mmol) was added to the reaction solution of compound 44. The mixture was stirred for reaction. After 2 h, the reaction progress was detected by TLC and LCMS. After the reaction was completed, the reaction solution was extracted with ethyl acetate and a saturated sodium chloride solution. The organic phase was collected, dried over anhydrous sodium sulfate, and then filtered. The filtrate was concentrated under reduced pressure, and the residue was purified by silica gel column chromatography to give compound 45 (3.47 mmol, yield: 88%). LC-MS (ESI): $(M+H)^+$ calculated 312.2, found 312.3.

Compound 45 (1.0 mmol) and 3-chloro-4-fluoro-nitrobenzene (1.5 mmol) were dissolved in DMF (5 mL), and cesium carbonate (2.0 mmol) was added. The mixture was stirred for reaction at 60 °C overnight under nitrogen atmosphere. The reaction progress was detected by TLC and LCMS. After the reaction was completed, a saturated ammonium chloride solution was added to quench the reaction, and the reaction solution was extracted with ethyl acetate and a saturated sodium chloride solution. The organic phase was collected, dried over anhydrous sodium sulfate, and then filtered. The filtrate was concentrated under reduced pressure, and the residue was purified by silica gel column chromatography to give compound 46 (0.76 mmol, yield: 76%). LC-MS (ESI): $(M+H)^+$ calculated 467.2, found 467.4.

Compound 46 (0.76 mmol) was dispersed in water (5 mL), and $B_2(OH)_4$ (5 mmol) was added. The mixture was heated to 100 °C and stirred overnight under nitrogen atmosphere. The reaction progress was detected by TLC and LCMS. After the reaction was completed, the reaction solution was extracted with ethyl acetate and a saturated sodium chloride solution. The organic phase was collected, dried over anhydrous sodium sulfate, and then filtered. The filtrate was concentrated under reduced pressure, and the residue was purified by silica gel column chromatography to give compound 47 (0.68 mmol, yield: 89%). LC-MS (ESI): $(M+H)^+$ calculated 437.2, found 437.4.

Referring to the method in Example 2, compound A76 was obtained by replacing compound 4 with compound 47. LC-MS (ESI): $(M+H)^+$ calculated 736.3, found 736.6.

Referring to the method in Example 2, compound B76 was obtained by replacing compound 4 with compound 47. LC-MS (ESI): $(M+H)^+$ calculated 636.3, found 636.5.

Referring to the synthetic steps and methods for compounds A76 and B76, A77-A78 and B77-B78 were obtained by replacing compound 41 and compound 42 with different amines and carboxylates, respectively.

| N-1 Compound | MS: $(M+H)^+$ calculated | MS: $(M+H)^+$ found | Compound | MS: $(M+H)^+$ calculated | MS: $(M+H)^+$ found |
|---|---|---|---|---|---|
| A77 | 710.3 | 710.6 | B77 | 610.3 | 610.5 |
| A78 | 698.2 | 698.5 | B78 | 598.2 | 598.4 |

Example 11: Synthesis of Compounds B79-B81

| Structural formula | $R_2$ | $L_2$ | N-1 Compound | Compound |
|---|---|---|---|---|
| 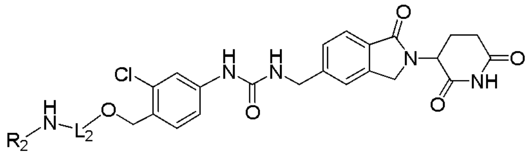 | 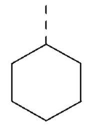 | 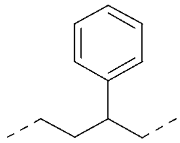 | A79 | B79 |
| | 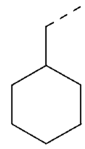 | 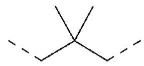 | A80 | B80 |
| | 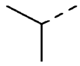 | 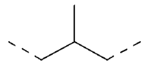 | A81 | B81 |

The synthesis of compound B80 was taken as an example

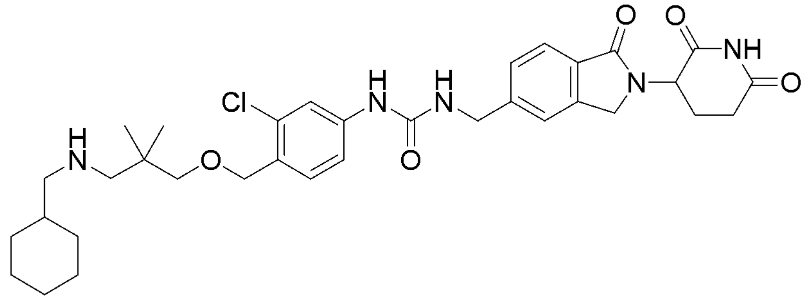

Synthesis of intermediate

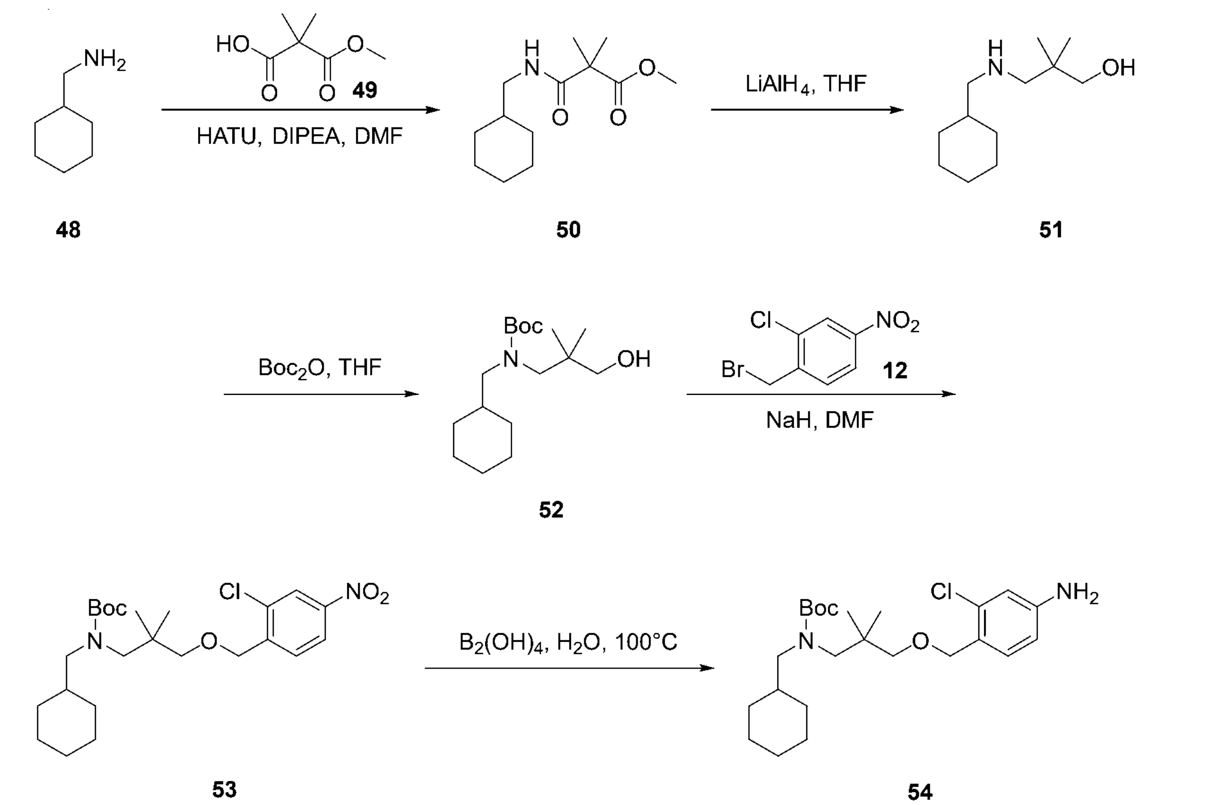


48 49 50 51 52 12 53 54

Compound 48 (5 mmol) and compound 49 (5 mmol) were dissolved in DMF (10 mL), and DIPEA (15 mmol) and HATU (6 mmol) were added. The mixture was stirred for reaction overnight. The reaction progress was detected by TLC and LCMS. After the reaction was completed, the reaction solution was extracted with ethyl acetate and a saturated sodium carbonate solution. The organic phase was collected and extracted with a saturated sodium chloride solution. The organic phase was collected, dried over anhydrous sodium sulfate, and then filtered. The filtrate was concentrated under reduced pressure, and the residue was purified by silica gel column chromatography to give compound 50 (3.85 mmol, yield: 77%). LC-MS (ESI): $(M+H)^+$ calculated 242.2, found 242.4.

Compound 50 (3.85 mmol) was dissolved in THF (20 mL), and $LiAlH_4$ (16 mmol) was added in an ice bath under nitrogen atmosphere. The mixture was stirred for reaction at 60 °C. After 4 h, the reaction progress was detected by TLC and LCMS. After the reaction was completed, the reaction solution was cooled to room temperature, and an aqueous sodium hydroxide solution was added in an ice bath to quench the reaction. The solution after quenching was stirred for 3 h. The reaction solution containing compound 51 was directly used in the next step without further treatment. LC-MS (ESI): $(M+H)^+$ calculated 200.2, found 200.2.

$Boc_2O$ (5 mmol) was added to the reaction solution of compound 51. The mixture was stirred for reaction. After 2 h, the reaction progress was detected by TLC and LCMS. After the reaction was completed, the reaction solution was extracted with ethyl acetate and a saturated sodium chloride solution. The organic phase was collected, dried over anhydrous sodium sulfate, and then filtered. The filtrate was concentrated under reduced pressure, and the residue was purified by silica gel column chromatography to give compound 52 (3.28 mmol, yield: 85%). LC-MS (ESI): $(M+H)^+$ calculated 300.2, found 300.5.

Compound 52 (1.0 mmol) was dissolved in DMF (5 mmol), and sodium hydride (1.5 mmol, 60%) was added in an ice bath under nitrogen atmosphere. The mixture was stirred for reaction for half an hour. A solution of compound 12 (1.5 mmol) in DMF (2 mL) was added to the reaction system. The mixture was stirred for reaction for 3 h. The reaction was detected by TLC and LCMS. After the reaction was completed, a saturated ammonium chloride solution was added to quench the reaction, and the reaction solution was extracted with ethyl acetate and a saturated sodium chloride solution. The organic phases were combined, dried over anhydrous sodium sulfate, and then filtered. The filtrate was concentrated under reduced pressure, and the residue was purified by silica gel column chromatography to give compound 53 (0.95 mmol, yield: 95%). LC-MS (ESI): $(M+H)^+$ calculated 469.2, found 469.4.

Compound 53 (0.95 mmol) was dispersed in water (5 mL), and $B_2(OH)_4$ (5 mmol) was added. The mixture was heated to 100 °C and stirred overnight under nitrogen atmosphere. The reaction progress was detected by TLC and LCMS. After the reaction was completed, the reaction solution was extracted with ethyl acetate and a saturated sodium chloride solution. The organic phase was collected, dried over anhydrous sodium sulfate, and then filtered. The filtrate was

concentrated under reduced pressure, and the residue was purified by silica gel column chromatography to give compound 54 (0.76 mmol, yield: 80%). LC-MS (ESI): $(M+H)^+$ calculated 439.3, found 439.5.

Referring to the method in Example 2, compound A80 was obtained by replacing compound 4 with compound 54. LC-MS (ESI): $(M+H)^+$ calculated 738.3, found 738.6.

Referring to the method in Example 2, compound B80 was obtained by replacing compound 4 with compound 54. LC-MS (ESI): $(M+H)^+$ calculated 638.3, found 638.5.

Referring to the synthetic steps and methods for compounds A80 and B80, A79 and A81 as well as B79 and B81 were obtained by replacing compound 48 and compound 49 with different amines and carboxylates, respectively.

| N-1 Compound | MS: $(M+H)^+$ calculated | MS: $(M+H)^+$ found | Compound | MS: $(M+H)^+$ calculated | MS: $(M+H)^+$ found |
|---|---|---|---|---|---|
| A79 | 786.3 | 786.6 | B79 | 686.3 | 686.6 |
| A81 | 670.2 | 670.4 | B81 | 570.2 | 570.5 |

Example 12: Synthesis of Compounds B82-B86

| Structural formula | $R_2$ | $R_7$ | $L_1$ | N-1 Compound | Compound |
|---|---|---|---|---|---|
| 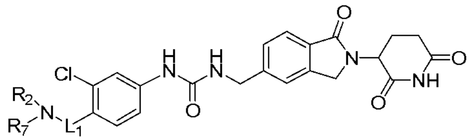 | 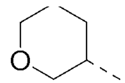 | H | $-(CH_2)_2-$ | A82 | B82 |
| | 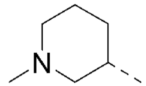 | H | $-(CH_2)_2-$ | A83 | B83 |
| | 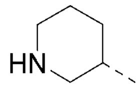 | $CH_3$ | $-(CH_2)_2-$ | A84 | B84 |
| | 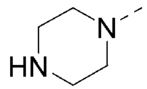 | | $-(CH_2)_4-$ | A85 | B85 |
| | 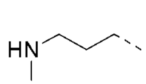 | 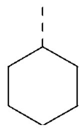 | $-(CH_2)_2-$ | A86 | B86 |

The synthesis of compound B84 was taken as an example

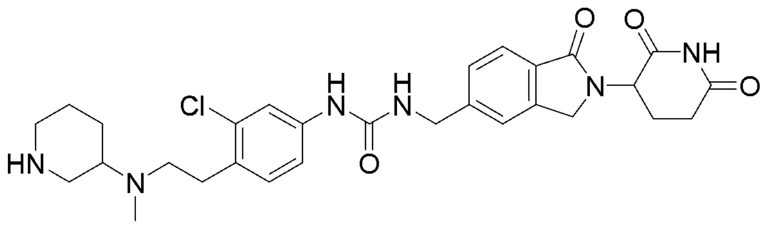

Synthesis of intermediate

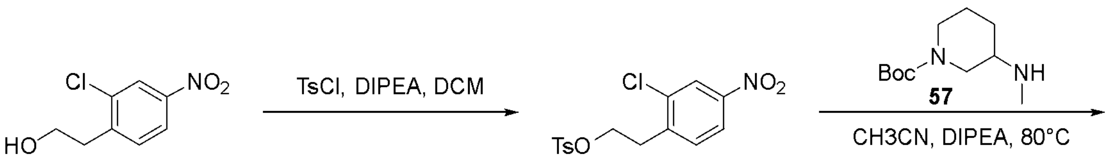


55 56

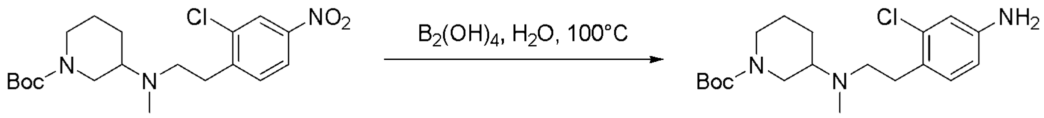


58 59

Compound 55 (5 mmol) was dissolved in DCM (10 mL), and TsCl (10 mmol) and DIPEA (10 mmol) were added. The mixture was stirred for reaction overnight. The reaction progress was detected by TLC and LCMS. After the reaction was completed, the reaction solution was concentrated under reduced pressure, and the residue was purified by silica gel column chromatography to give compound 56 (3.9 mmol, yield: 78%). LC-MS (ESI): $(M+H)^+$ calculated 356.0, found 356.1.

Compound 56 (2 mmol) and compound 57 (2.2 mmol) were dissolved in $CH_3CN$ (10 mL), and DIPEA (4 mmol) was added. The mixture was stirred for reaction at 80 °C under nitrogen atmosphere. The reaction progress was detected by TLC and LCMS. After the reaction was completed, the reaction solution was extracted with ethyl acetate and a saturated sodium chloride solution. The organic phases were combined, dried over anhydrous sodium sulfate, and then filtered. The filtrate was concentrated under reduced pressure, and the residue was purified by silica gel column chromatography to give compound 58 (1.52 mmol, yield: 76%). LC-MS (ESI): $(M+H)^+$ calculated 398.2, found 398.4.

Compound 58 (1.52 mmol) was dispersed in water (10 mL) and $B_2(OH)_4$ (10 mmol) was added. The mixture was heated to 100 °C and stirred overnight under nitrogen atmosphere. The reaction progress was detected by TLC and LCMS. After the reaction was completed, the reaction solution was extracted with ethyl acetate and a saturated sodium chloride solution. The organic phase was collected, dried over anhydrous sodium sulfate, and then filtered. The filtrate was concentrated under reduced pressure, and the residue was purified by silica gel column chromatography to give compound 59 (1.26 mmol, yield: 83%). LC-MS (ESI): $(M+H)^+$ calculated 368.2, found 368.4.

Referring to the method in Example 2, compound A84 was obtained by replacing compound 4 with compound 59. LC-MS (ESI): $(M+H)^+$ calculated 667.2, found 667.4.

Referring to the method in Example 2, compound B84 was obtained by replacing compound 4 with compound 59. LC-MS (ESI): $(M+H)^+$ calculated 567.2, found 567.4.

Referring to the synthetic steps and methods for compounds A84 and B84, A82-A83, A85-A86, B82-B83, and B85-B86 were obtained by replacing compound 55 and compound 57 with different alcohols and amines, respectively.

| N-1 Compound | MS: $(M+H)^+$ calculated | MS: $(M+H)^+$ found | Compound | MS: $(M+H)^+$ calculated | MS: $(M+H)^+$ found |
|---|---|---|---|---|---|
| A82 | 654.2 | 654.5 | B82 | 554.2 | 554.5 |
| A83 | 667.2 | 667.5 | B83 | 567.2 | 567.4 |
| A85 | 667.2 | 667.5 | B85 | 567.2 | 567.5 |
| A86 | 723.3 | 723.6 | B86 | 623.3 | 623.6 |

Example 13: Synthesis of Compounds B87-B90

| Structural formula | $R_2$ | N-1 Compound | Compound |
|---|---|---|---|
| 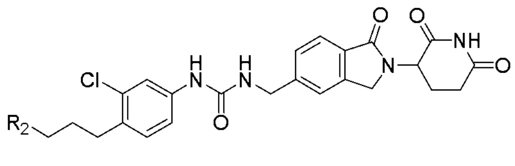 | 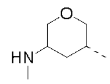 | A87 | B87 |
| | 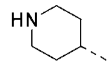 | A88 | B88 |
| | 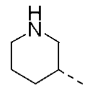 | A89 | B89 |
| | 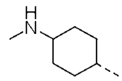 | A90 | B90 |

The synthesis of compound B87 was taken as an example

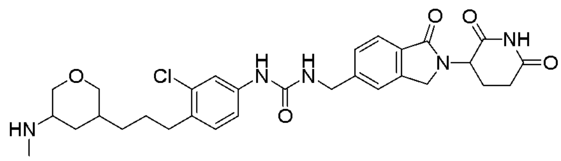

Synthesis of intermediate

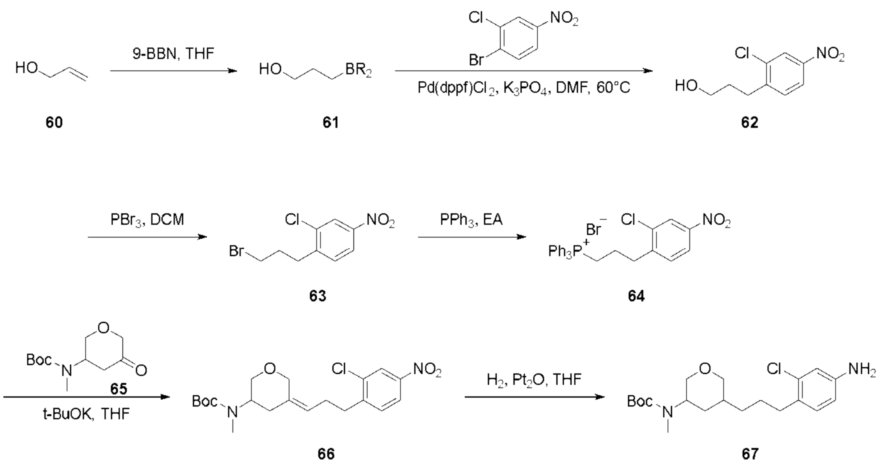

A solution of 9-BBN (15 mmol, 30 mL, 0.5 mmol/mL) in tetrahydrofuran was added to a solution of compound 60 (10 mmol) in tetrahydrofuran in an ice bath under nitrogen atmosphere. The mixture was heated to 80 °C and stirred for reaction. After the mixture was reacted overnight, the reaction progress was detected by TLC. After the reaction was completed, the reaction solution was cooled to room temperature, and the reaction solution containing compound 61 was directly used in the next step.

DMF (100 mL) and a solution of potassium phosphate (12.5 mmol) in water (30 mL) were added to the reaction solution of compound 61 under nitrogen atmosphere. The mixture was stirred for 10 min, and then 3-chloro-4-bromonitrobenzene (10 mmol) and Pd(dppf)$Cl_2$ (1 mmol) were added under nitrogen atmosphere. The mixture was heated to 90 °C and reacted overnight. The reaction progress was detected by TLC and LCMS. After the starting materials were completely consumed, the reaction solution was cooled to room temperature and extracted with ethyl acetate and a saturated ammonium chloride solution. Insoluble substances in the system were removed by filtration, and then the organic phase was extracted with a saturated sodium chloride solution. The organic phase was collected, dried over anhydrous sodium sulfate, and then filtered. The filtrate was concentrated under reduced pressure, and the residue was purified by silica gel column chromatography to give compound 62 (7.59 mmol, yield: 76%). LC-MS (ESI): $(M+H)^+$ calculated 216.0, found 217.1.

Compound 62 was dissolved in DCM (2 mmol), and phosphorus tribromide (4 mmol) was added in an ice bath. The mixture was reacted under nitrogen atmosphere. After four hours, the reaction progress was detected by TLC and LCMS. After the reaction was completed, water was added in an ice bath to quench the reaction, and the reaction solution was extracted with ethyl acetate and a saturated sodium chloride solution. The organic phase was collected, dried over anhydrous sodium sulfate, and then filtered. The filtrate was concentrated under reduced pressure, and the residue was purified by silica gel column chromatography to give compound 63 (1.48 mmol, yield: 74%).

Compound 63 (1.48 mmol) was dissolved in ethyl acetate (10 mL), and triphenylphosphine (1.6 mmol) was added. The mixture was reacted at room temperature under nitrogen atmosphere. After 2 h, the reaction progress was detected by TLC and LCMS. After the reaction was completed, the solid was collected by filtration, washed with ethyl acetate, and dried in vacuum to give compound 64 (1.42 mmol, yield: 96%). LC-MS (ESI): $M^+$ calculated 460.1, found 460.3.

Compound 64 (1.42 mmol) was dissolved in THF (20 mL), and t-BuOK (1.5 mmol) was added in an ice bath under atmosphere. After half an hour, a solution of compound 65 (1.5 mmol) in THF (5 mL) was added. The mixture was stirred for reaction under nitrogen atmosphere. After 2 h, the reaction progress was detected by TLC and LCMS. After the reaction was completed, a saturated ammonium chloride solution was added in an ice bath to quench the reaction, and the reaction solution was extracted with ethyl acetate and a saturated sodium chloride solution. The organic phase was collected, dried over anhydrous sodium sulfate, and then filtered. The filtrate was concentrated under reduced pressure, and the residue was purified by silica gel column chromatography to give compound 66 (1.25 mmol, yield: 88%). LC-MS (ESI): $(M+H)^+$ calculated 411.2, found 411.3.

Compound 66 (0.5 mmol) was dissolved in THF (5 mL), and platinum dioxide (50 mg) was added under nitrogen atmosphere. The mixture was purged with hydrogen and then stirred for reaction. After 2 h, the reaction progress was detected by TLC and LCMS. After the reaction was completed, celite was added, and the solid was removed by filtration. The filtrate was concentrated under reduced pressure, and the residue was purified by silica gel column chromatography to give compound 67 (0.44 mmol, yield: 88%). LC-MS (ESI): $(M+H)^+$ calculated 383.2, found 383.3.

Referring to the method in Example 2, compound A87 was obtained by replacing compound 4 with compound 67. LC-MS (ESI): $(M+H)^+$ calculated 682.2, found 682.5.

Referring to the method in Example 2, compound B87 was obtained by replacing compound 4 with compound 67. LC-MS (ESI): $(M+H)^+$ calculated 582.2, found 582.5.

Referring to the synthetic steps and methods for compounds A87 and B87, A88-A90 and B88-B90 were obtained by replacing compound 65 with different ketones.

| N-1 Compound | MS: $(M+H)^+$ calculated | MS: $(M+H)^+$ found | Compound | MS: $(M+H)^+$ calculated | MS: $(M+H)^+$ found |
|---|---|---|---|---|---|
| A88 | 652.2 | 652.5 | B88 | 552.2 | 552.4 |
| A89 | 652.2 | 652.6 | B89 | 552.2 | 552.5 |
| A90 | 680.3 | 680.6 | B90 | 580.3 | 580.5 |

Example 14: Synthesis of Compounds B91-B93

| Structural formula | $R_2$ | N-1 Compound | Compound |
|---|---|---|---|
| 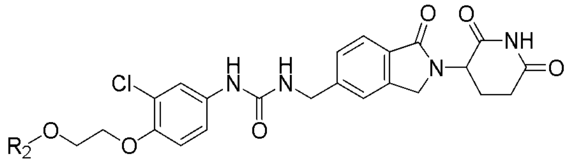 | 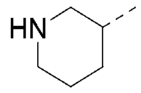 | A91 | B91 |
| | 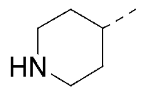 | A92 | B92 |
| | 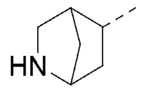 | A93 | B93 |

The synthesis of compound B91 was taken as an example

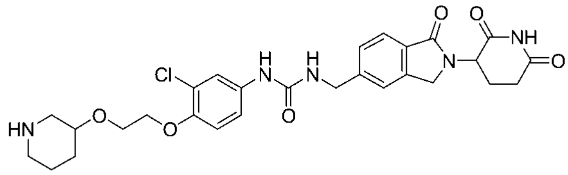

Synthesis of intermediate

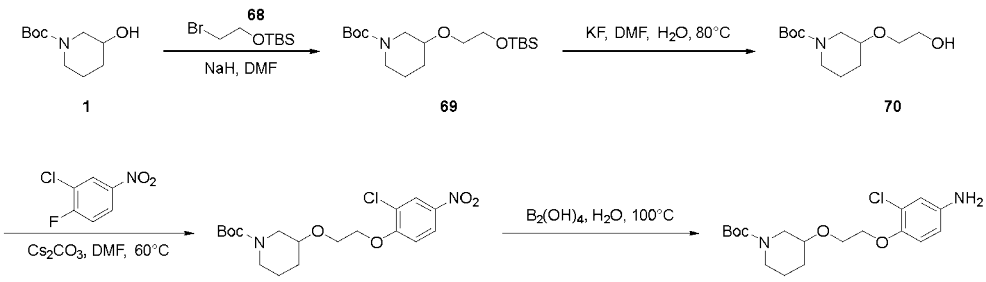


1 69 70 71 72

Compound 1 (3.0 mmol) and compound 68 (6.0 mmol) were dissolved in DMF (5 mL), and sodium hydride (6 mmol, 60%) was added in an ice bath under nitrogen atmosphere. The mixture was stirred for reaction. After 4 h, the reaction progress was detected by TLC and LCMS. After the reaction was completed, a saturated ammonium chloride solution was added in an ice bath to quench the reaction, and the reaction solution was extracted with ethyl acetate and a saturated sodium chloride solution. The organic phase was collected, dried over anhydrous sodium sulfate, and then filtered. The filtrate was concentrated under reduced pressure, and the residue was purified by silica gel column chromatography to give compound 69 (0.9345 mmol, yield: 31%). LC-MS (ESI): $(M+H)^+$ calculated 360.2, found 360.3.

Compound 69 (0.9345 mmol) was dissolved in a mixed solvent of DMF (4 mL) and water (1 mL), and potassium fluoride (4.673 mmol) was added. The mixture was stirred for reaction at 80 °C overnight under nitrogen atmosphere. The reaction progress was detected by TLC and LCMS. After the reaction was completed, the reaction solution was extracted with ethyl acetate and a saturated sodium chloride solution. The organic phase was collected, dried over anhydrous sodium sulfate, and then filtered. The filtrate was concentrated under reduced pressure, and the residue was purified by silica gel column chromatography to give compound 70 (0.7288 mmol, 77.99%). LC-MS (ESI): $(M+H)^+$ calculated 245.2, found 245.3.

Compound 70 (0.7288 mmol) and 3-chloro-4-fluoro-nitrobenzene (1.45 mmol) were dissolved in DMF (4 mL), and cesium carbonate (2.90 mmol) was added. The mixture was stirred for reaction at 60 °C overnight under nitrogen atmosphere. The reaction progress was detected by TLC and LCMS. After the reaction was completed, a saturated ammonium chloride solution was added to quench the reaction, and the reaction solution was extracted with ethyl acetate and a saturated sodium chloride solution. The organic phase was collected, dried over anhydrous sodium sulfate, and then filtered. The filtrate was concentrated under reduced pressure, and the residue was purified by silica gel column chromatography to give compound 71 (0.6175 mmol, yield: 85%). LC-MS (ESI): $(M+H)^+$ calculated 401.1, found 401.2.

Compound 71 (0.5723 mmol) was dissolved in DMF (5 mL). $B_2(OH)_4$ (11.445 mmol) was added, followed by the addition of 4,4'-bipyridine (0.005723 mmol). The mixture was stirred for reaction for 10 min. The reaction progress was detected by TLC and LCMS. After the reaction was completed, the reaction solution was extracted with ethyl acetate and a saturated sodium chloride solution. The organic phase was collected, dried over anhydrous sodium sulfate, and then filtered. The filtrate was concentrated under reduced pressure, and the residue was purified by silica gel column chromatography to give compound 72 (0.5323 mmol, yield: 93%). LC-MS (ESI): $(M+H)^+$ calculated 371.2, found 371.3.

Referring to the method in Example 2, compound A91 was obtained by replacing compound 4 with compound 72. LC-MS (ESI): $(M+H)^+$ calculated 670.2, found 670.5. HRMS (ESI): $(M+Na)^+$ calculated 692.2458, found 692.2459. $^1H$ NMR (400 MHz, DMSO-$d_6$) δ 10.98 (s, 1H), 8.62 (s, 1H), 7.69 (d, *J* = 7.8 Hz, 1H), 7.63 (d, *J* = 2.6 Hz, 1H), 7.51 (s, 1H), 7.44 (dd, *J* = 7.9, 1.4 Hz, 1H), 7.18 (dd, *J* = 8.9, 2.6 Hz, 1H), 7.04 (d, *J* = 9.0 Hz, 1H), 6.74 (t, *J* = 6.1 Hz, 1H), 5.10 (dd, *J* = 13.3, 5.1 Hz, 1H), 4.52 - 4.36 (m, 3H), 4.31 (d, *J* = 17.3 Hz, 1H), 4.08 (t, *J* = 4.7 Hz, 2H), 3.83 - 3.69 (m, 2H), 3.53 (s, 1H), 3.41 (dt, *J* = 7.5, 3.8 Hz, 1H), 3.30 - 2.99 (m, 3H), 2.91 (ddd, *J* = 17.2, 13.6, 5.4 Hz, 1H), 2.65 - 2.55 (m, 1H), 2.45 - 2.30 (m, 1H), 2.00 (ddd, *J* = 7.2, 4.7, 2.2 Hz, 1H), 1.83 (s, 1H), 1.50 (d, *J* = 24.3 Hz, 1H), 1.37 (s, 9H), 1.29 (td, *J* = 8.7, 4.3 Hz, 1H).

Referring to the method in Example 2, compound B91 was obtained by replacing compound 4 with compound 72. LC-MS (ESI): $(M+H)^+$ calculated 570.2, found 570.5. HRMS (ESI): $(M+H)^+$ calculated 570.2114, found 570.2138. $^1H$ NMR (400 MHz, DMSO-$d_6$) δ 10.98 (s, 1H), 9.00 (s, 2H), 7.69 (d, *J* = 7.8 Hz, 1H), 7.64 (d, *J* = 2.6 Hz, 1H), 7.51 (s, 1H), 7.44 (d, *J* = 7.8 Hz, 1H), 7.20 (dd, *J* = 8.9, 2.6 Hz, 1H), 7.05 (d, *J* = 8.9 Hz, 1H), 6.97 (t, *J* = 6.1 Hz, 1H), 5.10 (dd, *J* = 13.3, 5.1 Hz, 1H), 4.53 - 4.36 (m, 3H), 4.31 (d, *J* = 17.3 Hz, 1H), 4.18 - 4.06 (m, 2H), 3.90 - 3.79 (m, 2H), 3.79 - 3.71 (m, 1H), 3.23 - 3.09 (m, 1H), 3.08 - 2.84 (m, 4H), 2.65 - 2.55 (m, 1H), 2.46 - 2.30 (m, 1H), 2.05 - 1.95 (m, 1H), 1.88 - 1.74 (m, 2H), 1.74 - 1.50 (m, 2H).

Referring to the synthetic steps and methods for compounds A91 and B91, A92-A93 and B92-B93 were obtained by replacing compound 1 with different alcohols.

| N-1 Compound | MS: $(M+H)^+$ calculated | MS: $(M+H)^+$ found | Compound | MS: $(M+H)^+$ calculated | MS: $(M+H)^+$ found |
|---|---|---|---|---|---|
| A92 | 670.2 | 670.5 | B92 | 570.2 | 570.4 |
| A93 | 682.2 | 682.5 | B93 | 582.2 | 582.5 |

Example 17: Preparation of Antibody-Drug Conjugates (ADCs) of Compounds of the Present Disclosure Preparation of linker-drugs:

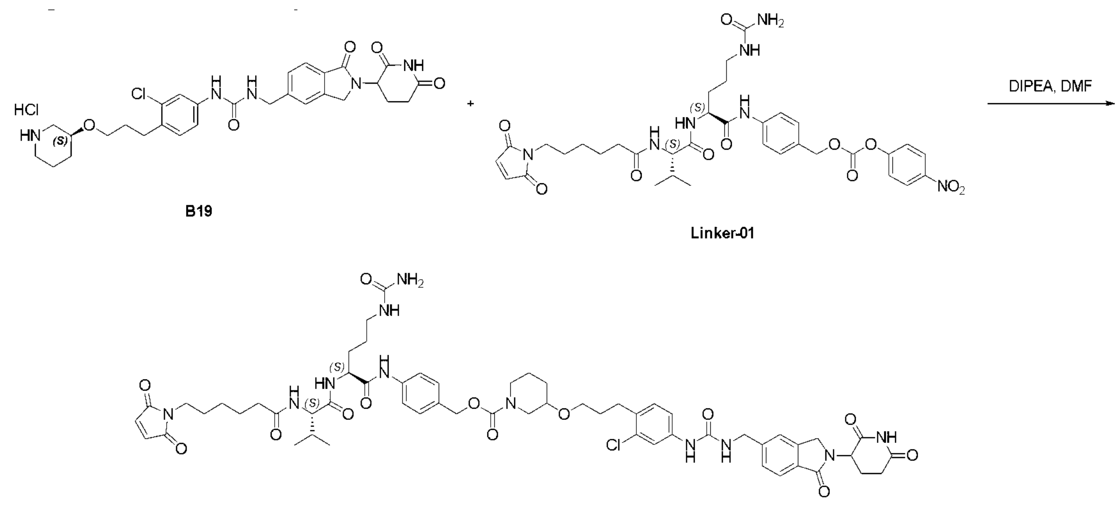


B19

Linker-01

LD01

B19 (0.1 mmol) was dissolved in DMF (2 mL), and Linker-01 (0.11 mmol) was added to the solution, followed by the dropwise addition of DIPEA (0.3 mmol). The mixture was stirred for reaction. The reaction progress was detected by TLC and LCMS. After the reaction was completed, the reaction solution was extracted with ethyl acetate and a saturated sodium chloride solution. The organic phase was collected, dried over anhydrous sodium sulfate, and then filtered. The filtrate was concentrated under reduced pressure, and the residue was purified by silica gel column chromatography to

give compound LD01 (0.068 mmol, 68%). LC-MS (ESI): $(M+H)^+$ calculated 1166.5, found 1167.0.

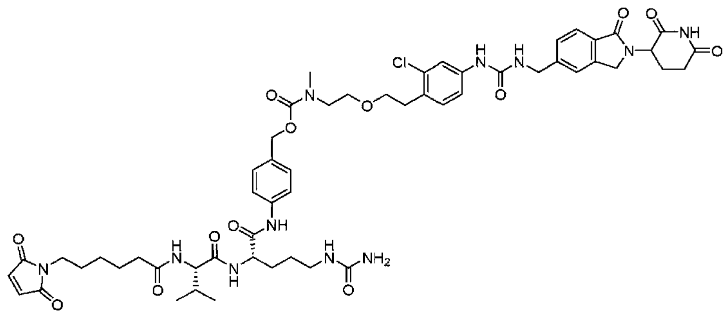

LD09

Smol006 (0.1 mmol) was dissolved in DMF (2 mL), and Linker-01 (0.11 mmol) was added to the solution, followed by the dropwise addition of DIPEA (0.3 mmol). The mixture was stirred for reaction. The reaction progress was detected by TLC and LCMS. After the reaction was completed, the reaction solution was extracted with ethyl acetate and a saturated sodium chloride solution. The organic phase was collected, dried over anhydrous sodium sulfate, and then filtered. The filtrate was concentrated under reduced pressure, and the residue was purified by silica gel column chromatography to give compound LD09 (0.063 mmol, 63%). LC-MS (ESI): $(M+H)^+$ calculated 1126.5, found 1127.0.

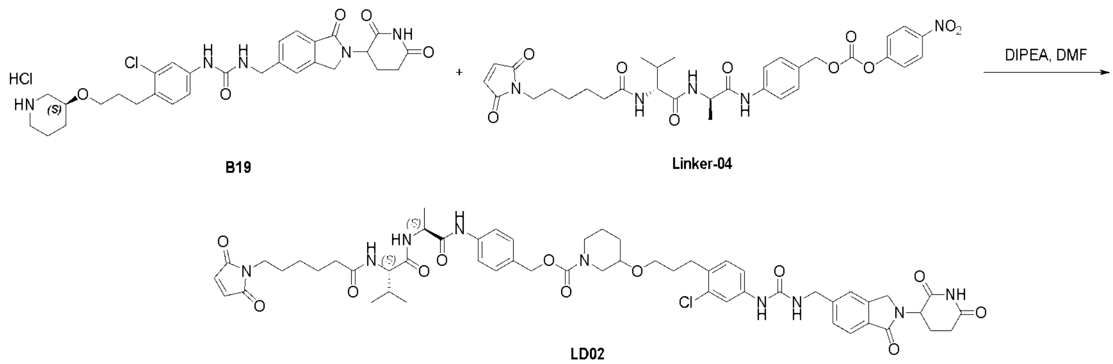


B19 Linker-04

LD02

B19 (0.1 mmol) was dissolved in DMF (2 **mL),** and Linker-04 (0.11 mmol) was added to the solution, followed by the dropwise addition of DIPEA (0.3 mmol). The mixture was stirred for reaction. The reaction progress was detected by TLC and LCMS. After the reaction was completed, the reaction solution was extracted with ethyl acetate and a saturated sodium chloride solution. The organic phase was collected, dried over anhydrous sodium sulfate, and then filtered. The filtrate was concentrated under reduced pressure, and the residue was purified by silica gel column chromatography to give compound LD02 (0.085 mmol, 85%). LC-MS (ESI): $(M+H)^+$ calculated 1080.4, found 1080.9.

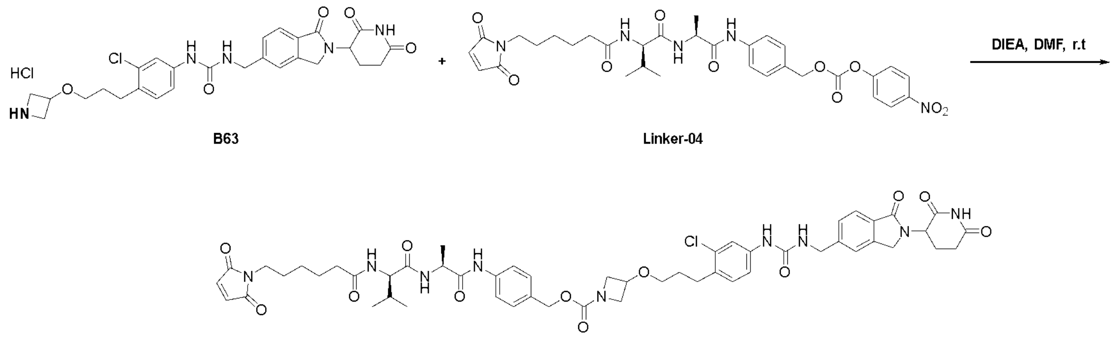


B63 Linker-04

LD06

B63 (0.1 mmol) was dissolved in DMF (2 **mL),** and Linker-04 (0.11 mmol) was added to the solution, followed by the dropwise addition of DIPEA (0.3 mmol). The mixture was stirred for reaction. The reaction progress was detected by TLC and LCMS. After the reaction was completed, the reaction solution was extracted with ethyl acetate and a saturated sodium chloride solution. The organic phase was collected, dried over anhydrous sodium sulfate, and then filtered. The

filtrate was concentrated under reduced pressure, and the residue was purified by silica gel column chromatography to give compound LD06 (0.083 mmol, 83%). LC-MS (ESI): $(M+H)^+$ calculated 1052.4, found 1052.7.

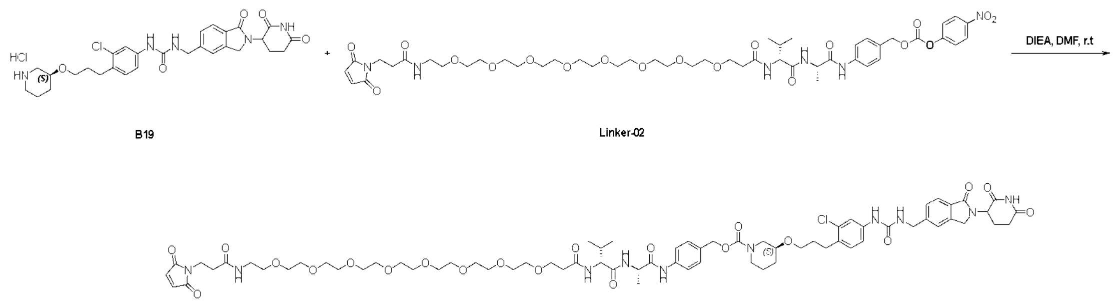

B19 Linker-02

LD07

B19 (0.1 mmol) was dissolved in DMF (2 mL), and Linker-02 (0.11 mmol) was added to the solution, followed by the dropwise addition of DIPEA (0.3 mmol). The mixture was stirred for reaction. The reaction progress was detected by TLC and LCMS. After the reaction was completed, the reaction solution was extracted with ethyl acetate and a saturated sodium chloride solution. The organic phase was collected, dried over anhydrous sodium sulfate, and then filtered. The filtrate was concentrated under reduced pressure, and the residue was purified by silica gel column chromatography to give compound LD07 (0.053 mmol, 53%). LC-MS (ESI): $(M+H)^+$ calculated 1631.6, found 1632.0.

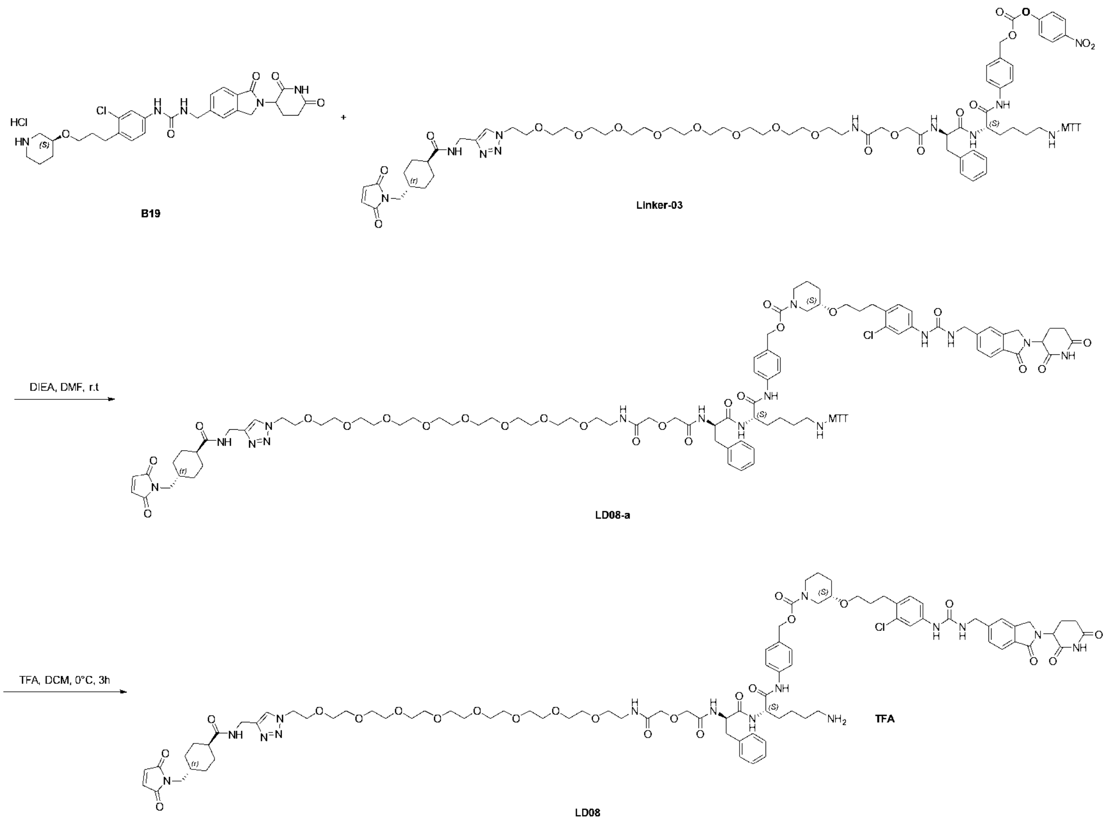

B19 Linker-03

LD08-a

LD08

B19 (0.1 mmol) was dissolved in DMF (2 mL), and Linker-03 (0.11 mmol) was added to the solution, followed by the dropwise addition of DIPEA (0.3 mmol). The mixture was stirred for reaction. The reaction progress was detected by TLC and LCMS. After the reaction was completed, the reaction solution was extracted with ethyl acetate and a saturated sodium chloride solution. The organic phase was collected, dried over anhydrous sodium sulfate, and then filtered. The filtrate was concentrated under reduced pressure, and the residue was purified by silica gel column chromatography to give compound LD08-a (0.075 mmol, 75%). LC-MS (ESI): $(M+2H)^{2+}$ calculated 1030.0, found 1030.3.

LD08-a (0.075 mmol) was dissolved in DCM (2 mL), and TFA (0.5 mmol) was added to the solution. The mixture was stirred for reaction. The reaction progress was detected by TLC and LCMS. After the reaction was completed, the reaction solution was concentrated under reduced pressure to remove the solvent and extracted with ethyl acetate and

purified water. The aqueous phase was collected and lyophilized to give compound LD08 (0.061 mmol, 81%). LC-MS (ESI): $(M+H)^+$ calculated 1802.8, found 1803.2.

The following linker-drugs were prepared by methods similar to those described above for LD01 and LD09, with the starting materials replaced:

| Compound | Sr | L' | T | C | D |
|---|---|---|---|---|---|
| LD03 | Sr1 | - | T1 | C1 | B61 |
| LD04 | Sr1 | - | T9 | C1 | B61 |
| LD05 | Sr1 | - | T1 | C1 | B63 |
| LD09 | Sr1 | - | T1 | C1 | SMol006 |
| LD10 | Sr1 | - | T6 | C2 | B19 |
| LD11 | Sr2 | L'1 | T9 | C1 | B19 |
| LD12 | Sr3 | L'2 | T5 | C1 | B19 |
| LD13 | Sr2 | L'1 | T2 | - | B19 |
| LD14 | Sr2 | L'1 | T9 | - | B19 |
| LD15 | Sr5 | - | T11 | C1 | B19 |
| LD16 | Sr5 | L'2 | T5 | C1 | B19 |
| LD17 | Sr6 | - | T9 | C1 | B19 |
| LD18 | Sr8 | - | T9 | C1 | B19 |
| LD19 | Sr4 | L'4 | T9 | C1 | B19 |
| LD20 | Sr10 | L'5 | T9 | C1 | B19 |
| LD21 | Sr7 | - | T9 | C1 | B19 |
| LD22 | Sr1 | - | T6 | C2 | B61 |
| LD23 | Sr2 | L'1 | T9 | C1 | B61 |
| LD24 | Sr3 | L'2 | T5 | C1 | B61 |
| LD25 | Sr2 | L'1 | T2 | - | B61 |
| LD26 | Sr2 | L'1 | T9 | - | B61 |
| LD27 | Sr5 | - | T11 | C1 | B61 |
| LD28 | Sr5 | L'2 | T5 | C1 | B61 |
| LD29 | Sr1 | - | T6 | C2 | B63 |
| LD30 | Sr2 | L'1 | T9 | C1 | B63 |
| LD31 | Sr3 | L'2 | T5 | C1 | B63 |
| LD32 | Sr2 | L'1 | T2 | - | B63 |
| LD33 | Sr2 | L'1 | T9 | - | B63 |
| LD34 | Sr5 | - | T11 | C1 | B63 |
| LD35 | Sr5 | L'2 | T5 | C1 | B63 |
| LD36 | Sr11 | L'7 | T9 | C1 | B19 |
| LD37 | Sr10 | L'7 | T9 | C1 | B19 |
| LD38 | Sr9 | - | T9 | C1 | B19 |
| LD39 | Sr1 | L'5 | T9 | C1 | B19 |
| LD40 | Sr1 | L'4 | T9 | C1 | B19 |
| LD41 | Sr1 | L'7 | T9 | C1 | B19 |

(continued)

| Compound | Sr | L' | T | C | D |
|---|---|---|---|---|---|
| LD42 | Sr1 | L'3 | T9 | C1 | B19 |
| LD43 | Sr1 | - | T4 | C1 | B19 |
| LD44 | Sr1 | - | T11 | C1 | B19 |
| LD45 | Sr1 | - | T10 | C1 | B19 |
| LD46 | Sr1 | - | T3 | C1 | B19 |
| LD47 | Sr1 | L'1 | - | - | B19 |

Preparation of ADCs:

General method: An antibody sample was diluted to about 10 mg/mL with a suitable buffer (consistent with the sample buffer), and an appropriate amount of reducing agent TCEP was added, with the number of equivalents adjusted according to a target DAR (8-10 equivalents for a target DAR of 8, or 2-3 equivalents for a target DAR of 4). The pH was adjusted to 7-7.4 with a Tris buffer, and the mixture was reduced at room temperature for 1-1.5 h. The intermediate state of antibody reduction could be monitored by CE-SDS. After the antibody was completely reduced, an appropriate amount of saturated citric acid solution was first added to adjust the pH to about 6.5, and then an excess of a solution of the linker-drug in DMSO was added to make the equivalents of the linker-drug 15-20 times those of the antibody. The conjugation reaction was performed at room temperature for about 30 min. After the conjugation was completed, the reaction solution was first filtered, and then subjected to buffer exchange by ultrafiltration using a centrifugal concentration tube to remove excess linker-drug and other small-molecule impurities. After the purification was completed, the resulting sample was determined for DAR by hydrophobic chromatography or ultraviolet spectrophotometry.

Examples of ADC preparation were as follows:

ADC01-01-8: The antibody Trastuzumab was diluted to 10 mg/mL with a phosphate buffer, and 8 equivalents of reducing agent TCEP were added. Then, the pH was adjusted to 7-7.4 with a Tris buffer, and the mixture was reduced at room temperature for 1.5 h, so that the inter-chain disulfide bonds of the antibody were reduced into sulfhydryl. The intermediate state of antibody reduction could monitored by CE-SDS. After the antibody was completely reduced, an appropriate amount of saturated citric acid solution was added to adjust the pH to about 6.5, and then 20 equivalents of a solution of LD01 in DMSO were added. The conjugation reaction was performed at room temperature for 30 min. After the conjugation was completed, the reaction solution was first filtered, and then subjected to buffer exchange by ultrafiltration using a centrifugal concentration tube to remove excess linker-drug and other small-molecule impurities. After the purification was completed, the resulting sample was determined for DAR by hydrophobic chromatography or ultraviolet spectrophotometry.

ADC01-01-4: The antibody Trastuzumab was diluted to 10 mg/mL with a phosphate buffer, and 2.2 equivalents of reducing agent TCEP were added. Then, the pH was adjusted to 7-7.4 with a Tris buffer, and the mixture was reduced at room temperature for 1 h, so that the inter-chain disulfide bonds of the antibody were reduced into sulfhydryl. The intermediate state of antibody reduction could monitored by CE-SDS. After the antibody was completely reduced, an appropriate amount of saturated citric acid solution was added to adjust the pH to about 6.5, and then 15 equivalents of a solution of LD01 in DMSO were added. The conjugation reaction was performed at room temperature for 30 min. After the conjugation was completed, the reaction solution was first filtered, and then subjected to buffer exchange by ultrafiltration using a centrifugal concentration tube to remove excess linker-drug and other small-molecule impurities. After the purification was completed, the resulting sample was determined for DAR by hydrophobic chromatography or ultraviolet spectrophotometry.

The amino acid sequences of the antibodies used are as follows:

HER2
SYM001 (Trastuzumab)
> Heavy chain (SEQ ID NO. 1)

EVQLVESGGGLVQPGGSLRLSCAASGFNIKDTYIHWVRQAPGKGLEWVARIYPTNGYTRYADSVKGRFT
ISADTSKNTAYLQMNSLRAEDTAVYYCSRWGGDGFYAMDYWGQGTLVTVSSASTKGPSVFPLAPSSKS
TSGGTAALGCLVKDYFPEPVTVSWNSGALTSGVHTFPAVLQSSGLYSLSSVVTVPSSSLGTQTYICNVNH
KPSNTKVDKKVEPKSCDKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALPAPIEKTISKAKGQP
REPQVYTLPPSREEMTKNQVSLTCLVKGFYPSDIAVEWESNGQPENNYKTTPPVLDSDGSFFLYSKLTVD
KSRWQQGNVFSCSVMHEALHNHYTQKSLSLSPGK

> Light chain (SEQ ID NO. 2)

DIQMTQSPSSLSASVGDRVTITCRASQDVNTAVAWYQQKPGKAPKLLIYSASFLYSGVPSRFSGSRSGTD
FTLTISSLQPEDFATYYCQQHYTTPPTFGQGTKVEIKRTVAAPSVFIFPPSDEQLKSGTASVVCLLNNFYPR
EAKVQWKVDNALQSGNSQESVTEQDSKDSTYSLSSTLTLSKADYEKHKVYACEVTHQGLSSPVTKSFNR
GEC

SYM007 (pertuzumab)
> Heavy chain (SEQ ID NO. 3)

EVQLVESGGGLVQPGGSLRLSCAASGFTFTDYTMDWVRQAPGKGLEWVADVNPNSGGSIYNQRFKGRF

TLSVDRSKNTLYLQMNSLRAEDTAVYYCARNLGPSFYFDYWGQGTLVTVSSASTKGPSVFPLAPSSKST
SGGTAALGCLVKDYFPEPVTVSWNSGALTSGVHTFPAVLQSSGLYSLSSVVTVPSSSLGTQTYICNVNHK
PSNTKVDKKVEPKSCDKTHTCPPCPAPELLGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIEKTISKAKGQPR
EPQVYTLPPSREEMTKNQVSLTCLVKGFYPSDIAVEWESNGQPENNYKTTPPVLDSDGSFFLYSKLTVDK
SRWQQGNVFSCSVMHEALHNHYTQKSLSLSPG

> Light chain (SEQ ID NO. 4)

DIQMTQSPSSLSASVGDRVTITCKASQDVSIGVAWYQQKPGKAPKLLIYSASYRYTGVPSRFSGSGSGTDF
TLTISSLQPEDFATYYCQQYYIYPYTFGQGTKVEIKRTVAAPSVFIFPPSDEQLKSGTASVVCLLNNFYPRE
AKVQWKVDNALQSGNSQESVTEQDSKDSTYSLSSTLTLSKADYEKHKVYACEVTHQGLSSPVTKSFNR
GEC

Claudin 18.2
SYM003
> Heavy chain (SEQ ID NO. 5)

EVQLSESGGALVQPGESLRLSCAASGFTFSSYAMTWVRQAPGKGLEWVSSLSGSGRSTYYAASIKGRFTI
SRDNSKNTLYLQMSSLRAEDTAIYYCAKSLSYYHYYFDYWGQGTLVTVSSASTKGPSVFPLAPSSKSTSG
GTAALGCLVKDYFPEPVTVSWNSGALTSGVHTFPAVLQSSGLYSLSSVVTVPSSSLGTQTYICNVNHKPS
NTKVDKKVEPKSCDKTHTCPPCPAPELLGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPIEKTISKAKGQPRE
PQVYTLPPSRDELTKNQVSLTCLVKGFYPSDIAVEWESNGQPENNYKTTPPVLDSDGSFFLYSKLTVDKS
RWQQGNVFSCSVMHEALHNHYTQKSLSLSPGK

> Light chain (SEQ ID NO. 6)

DIQLTQSPSFLSASVGDRVPITCRASQDISNYLAWYQQKPGKAPKLLIYSASTLQSGVPSRFSGSGSGTEFT LTISSLQPEDFASYHCQQVKTYPLTFGGGTKVEIKRTVAAPSVFIFPPSDEQLKSGTASVVCLLNNFYPREA KVQWKVDNALQSGNSQESVTEQDSKDSTYSLSSTLTLSKADYEKHKVYACEVTHQGLSSPVTKSFNRGE C

DLL3
SYM004
> Heavy chain (SEQ ID NO. 7)

QVQLVQSGAEVKKPGASVKVSCKASGYTFTNYGMNWVRQAPGQGLEWMGWINTYTGEPTYADDFKG RVTMTTDTSTSTAYMELRSLRSDDTAVYYCARIGDSSPSDYWGQGTLVTVSSASTKGPSVFPLAPSSKST SGGTAALGCLVKDYFPEPVTVSWNSGALTSGVHTFPAVLQSSGLYSLSSVVTVPSSSLGTQTYICNVNHK PSNTKVDKKVEPKSCDKTHTCPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTISKAKGQPR EPQVYTLPPSRDELTKNQVSLTCLVKGFYPSDIAVEWESNGQPENNYKTTPPVLDSDGSFFLYSKLTVDK SRWQQGNVFSCSVMHEALHNHYTQKSLSLSPG

> Light chain (SEQ ID NO. 8)

EIVMTQSPATLSVSPGERATLSCKASQSVSNDVVWYQQKPGQAPRLLIYYASNRYTGIPARFSGSGSGTEF TLTISSLQSEDFAVYYCQQDYTSPWTFGQGTKLEIKRTVAAPSVFIFPPSDEQLKSGTASVVCLLNNFYPR EAKVQWKVDNALQSGNSQESVTEQDSKDSTYSLSSTLTLSKADYEKHKVYACEVTHQGLSSPVTKSFNR GEC

BCMA
SYM008 (Belantamab)
> Heavy Chain (SEQ ID NO. 9)

QVQLVQSGAEVKKPGSSVKVSCKASGGTFSNYWMHWVRQAPGQGLEWMGATYRGHSDTYYNQKFKG RVTITADKSTSTAYMELSSLRSEDTAVYYCARGAIYDGYDVLDNWGQGTLVTVSSASTKGPSVFPLAPSS KSTSGGTAALGCLVKDYFPEPVTVSWNSGALTSGVHTFPAVLQSSGLYSLSSVVTVPSSSLGTQTYICNV NHKPSNTKVDKKVEPKSCDKTHTCPPCPAPELLGGPSVFLFPPKPKDTLMISRTPEVTCVVVDVSHEDPE VKFNWYVDGVEVHNAKTKPREEQYNSTYRVVSVLTVLHQDWLNGKEYKCKVSNKALPAPIEKTISKAK GQPREPQVYTLPPSRDELTKNQVSLTCLVKGFYPSDIAVEWESNGQPENNYKTTPPVLDSDGSFFLYSKL TVDKSRWQQGNVFSCSVMHE ALHNHYTQKSLSLSPGK

> Light Chain (SEQ ID NO. 10)

DIQMTQSPSSLSASVGDRVTITCSASQDISNYLNWYQQKPGKAPKLLIYYTSNLHSGVPSRFSGSGSGTDF TLTISSLQPEDFATYYCQQYRKLPWTFGQGTKLEIKRTVAAPSVFIFPPSDEQLKSGTASVVCLLNNFYPR EAKVQWKVDNALQSGNSQESVTEQDSKDSTYSLSSTLTLSKADYEKHKVYACEVTHQGLSSPVTKSFNR GEC

The obtained antibody-drug conjugates are shown in the table below:

| ADC drug No. | Antibody | Linker-drug No. | DAR |
|---|---|---|---|
| ORM-5029 | SYM007 | LD09 | 3.50 |
| ADC01-01-8 | SYM001 | LD01 | 7.80 |
| ADC01-01-4 | SYM001 | LD01 | 3.90 |
| ADC01-02-8 | SYM001 | LD02 | 7.31 |

(continued)

| ADC drug No. | Antibody | Linker-drug No. | DAR |
|---|---|---|---|
| ADC01-02-4 | SYM001 | LD02 | 3.90 |
| ADC01-06-8 | SYM001 | LD06 | 7.01 |
| ADC01-06-4 | SYM001 | LD06 | 3.88 |
| ADC01-07-4 | SYM001 | LD07 | 4.44 |
| ADC01-08-8 | SYM001 | LD08 | 7.00 |
| ADC01-08-4 | SYM001 | LD08 | 3.10 |
| ADC03-01-8 | SYM003 | LD01 | 7.96 |
| ADC03-01-4 | SYM003 | LD01 | 4.40 |
| ADC04-01-8 | SYM004 | LD01 | 8.00 |
| ADC04-02-8 | SYM004 | LD02 | 7.92 |
| ADC04-06-8 | SYM004 | LD06 | 8.00 |
| ADC04-06-4 | SYM004 | LD06 | 2.98 |
| ADC04-07-8 | SYM004 | LD07 | 7.22 |
| ADC04-07-4 | SYM004 | LD07 | 4.17 |
| ADC04-08-8 | SYM004 | LD08 | 6.89 |
| ADC04-08-4 | SYM004 | LD08 | 3.20 |
| ADC04-09-8 | SYM004 | LD09 | 7.89 |
| ADC04-09-4 | SYM004 | LD09 | 3.72 |
| ADC07-01-8 | SYM007 | LD01 | 7.90 |
| ADC07-01-4 | SYM007 | LD01 | 4.00 |
| ADC07-02-8 | SYM007 | LD02 | 6.90 |
| ADC07-02-4 | SYM007 | LD02 | 3.50 |
| ADC07-06-8 | SYM007 | LD06 | 7.41 |
| ADC07-06-4 | SYM007 | LD06 | 3.53 |
| ADC07-08-8 | SYM007 | LD08 | 7.36 |
| ADC07-08-4 | SYM007 | LD08 | 3.03 |
| ADC07-09-8 | SYM007 | LD09 | 7.98 |
| ADC07-09-4 | SYM007 | LD09 | 4.70 |
| ADC08-01-8 | SYM008 | LD01 | 7.28 |
| ADC08-01-4 | SYM008 | LD01 | 2.18 |

Test Example 1: *In Vitro* Anti-Tumor Activity of Molecular Glue Compounds of the Present Disclosure

Different tumor cells were all commercially available and used for evaluating the anti-tumor inhibitory activity of the compounds of the present disclosure. Various tumor cells were cultured until the cell confluency reached 80%-90%, with cell viability above 90% as determined by trypan blue exclusion. Then, the cells were plated. Specifically, after cell density optimization, 100 μL of each of the cell suspensions of HL-60 and SK-BR-3 cells (cell density: 3000 cells/well), Romas cells (cell density: 6000 cells/well), NCI-H929 cells (cell density: 4000 cells/well), and NCI-H82 cells (cell density: 5000 cells/well) was plated in a BeyoGold™ ultra-low attachment black clear-bottom 96-well plate (Cat. No.: FULA965-24pcs), and the plate was incubated overnight. The next day, after the adherent cells were adhered to the wall and suspension cells requiring aggregation were aggregated, 50 μL of culture medium was aspirated from each well of the 96-well plate. Then, the compounds diluted with the corresponding culture media were added. Specifically, the compounds were serially diluted at a 5-fold gradient starting from the highest concentration of 1 μM, with a final DMSO

concentration of 1‰, and the test compounds were then added to the cell culture plate. The plate was incubated in an incubator at 37 °C with 5% carbon dioxide for 96 h. Before measurement, the CellTiter-Lumi™ luminescent cell viability assay reagent was equilibrated to room temperature in the dark. Then, 50 μL of the CellTiter-Lumi™ (Cat. No.: C0065XL) cell viability assay reagent was added to each well of the 96-well plate. The plate was shaken in the dark on a shaker at 300 rpm for 10 min to ensure complete cell lysis. After the reaction was completed, the luminescence was measured on a multi-mode microplate reader. The cell inhibition rate was determined according to the luminescence. Then, a cell viability curve was generated by Graphpad Prism 8.0, and the IC50 values were calculated. The compounds of the present disclosure were tested for activity by the method described above. The test results for some of the compounds are shown in Table 1.

Table 1. Cell inhibitory activity (IC50 values)

| Compound No. | HL-60 | SK-BR-3 | Romas | NCI-H929 | NCI-H82 |
|---|---|---|---|---|---|
| Smol006 | B | D | D | C | C |
| B7 | B | B | B | B | B |
| B8 | B | B | B | B | B |
| B9 | A | B | B | A | B |
| B10 | B | B | B | A | D |
| B13 | A | B | B | A | B |
| B14 | A | B | A | A | B |
| B15 | A | B | B | B | D |
| B19 | A | A | A | A | B |
| B20 | A | A | A | A | B |
| B21 | A | B | B | B | D |
| B25 | A | A | A | A | B |
| B43 | A | A | A | A | B |
| B44 | A | A | A | A | B |
| B45 | A | A | A | A | B |
| B46 | A | C | C | B | D |
| B47 | B | D | D | B | D |
| B48 | A | C | C | B | D |
| B51 | B | C | C | B | D |
| B53 | A | A | A | A | B |
| B54 | A | B | B | A | B |
| B61 | A | A | A | A | B |
| B63 | A | A | B | A | B |
| B64 | A | A | A | A | B |
| B65 | A | A | A | A | B |
| B66 | A | A | A | A | B |
| B67 | A | A | A | A | B |
| B68 | A | A | A | A | B |
| B69 | A | A | A | A | B |
| B70 | A | B | B | A | B |
| B71 | A | A | A | A | B |

(continued)

| Compound No. | HL-60 | SK-BR-3 | Romas | NCI-H929 | NCI-H82 |
|---|---|---|---|---|---|
| B72 | A | B | B | A | B |
| Note: in the table, A indicates an $IC_{50}$ value of < 10 nM, B indicates an $IC_{50}$ value between 10 nM and 50 nM, C indicates an $IC_{50}$ value between 50 nM and 100 nM, and D indicates an $IC_{50}$ value of > 100 nM. | | | | | |

The above results show that the compounds of the present disclosure have higher cell viability than Smol006 in cells of different indications, thus demonstrating greater application potential.

Test Example 2: GSPT1 Protein Degradation Validation of Compounds of the Present Disclosure

This experiment was performed to evaluate the targeting property of the compounds. An HiBiT protein tag was added to the N-terminus of the endogenous GSPT1 protein of HEK293T cells by CRISPR gene editing technology, so as to construct a HiBiT-GSPT1 HEK293T stably transfected cell strain. HiBiT-GSPT1 HEK293T cells were cultured in DMEM containing 10% FBS and 1% PenStrep. When the cell confluency reached 80%-90%, the cells were digested with trypsin, pipetted into single cells, and resuspended in a corresponding culture medium to form a cell suspension. The cells were counted using Cell Countess (NanoEnTek, #Cat. EVE-MC2), and the cell viability was determined by trypan blue exclusion to ensure that the cell viability was above 90%. The cells were plated in a 384-well plate (Corning, #Cat. 3764) at 10,000 cells/40 μL of cell suspension per well, and cultured in an incubator at 37 °C with 5% $CO_2$ overnight. The next day, the test compounds were added to the 384-well plate under conditions of 30 μM highest concentration, 5-fold serial dilution, and 1‰ DMSO. The plate was incubated in the incubator at 37 °C with 5% $CO_2$ for another 24 h. Before measurement, the Nano-Glo HiBiT reagent was equilibrated to room temperature, the LgBiT protein was diluted with the Nano-Glo HiBiT Lytic buffer according to a dilution ratio of 1:100, and the Nano-Glo@HiBiT Lytic substrate was diluted to an appropriate volume according to a ratio of 1:50. After the cell culture plate was equilibrated at room temperature for 10 min, 40 μL of Nano-Glo HiBiT Lytic assay reagent was added to each well. The plate was shaken on a microplate shaker at a rotation speed of 300 rpm for 3 min in the dark. After the reaction was completed, the luminescencevalues were read on BMG PHERASTAR FS (BMG LRBTECH, Cat. No.: PHERAstar FSX), and the DC50 values were calculated using GraphPad Prism 8.0.

Table 2. GSPT1 protein degradation

| Compound No. | DC50 (nM) | SK-BR-3 IC50 (nM) |
|---|---|---|
| A19 | 15.96 | 1.217 |
| B19 | 66.18 | 3.616 |
| Smol006 | 273.5 | 32.28 |

The above results show that the ability of the compounds of the present disclosure to degrade the GSPT1 protein was not affected after derivatization, and the anti-tumor mechanism of the compounds still involves catalyzing the GSPT1 protein degradation in cells.

Test Example 3: Activity Validation of Compounds of the Present Disclosure in Cell Strains with High Expression of Efflux Pump Proteins

Three types of cells used for evaluating the activity of the compounds of the present disclosure against a cell strain with high expression of efflux pump proteins were all purchased from Nanjing Cobioer Biosciences Co., Ltd., and all the cells were derived from ATCC (American Type Culture Collection). Various tumor cells were cultured until the cell confluency reached 80%-90%, with cell viability above 90% as determined by trypan blue exclusion. Then, the cells were plated. Specifically, after cell density optimization, 75 μL of each of three cell suspensions of SNU-5, HCT-15, and AsPc-1 cells (3000 cells/well) was inoculated into a BeyoGold™ ultra-low attachment black clear-bottom 96-well plate (Cat. No.: FULA965-24pcs), and the plate was incubated overnight in a cell incubator at 37 °C with carbon dioxide. The next day, after the cells adhered to the wall, 75 μL of the compounds diluted with the corresponding culture media were added. Specifically, the compounds were serially diluted at a 5-fold gradient starting from the highest concentration of 1 μM, with a final DMSO concentration of 1‰, and the test compounds were then added to the cell culture plate. The plate was incubated in an incubator at 37 °C with 5% carbon dioxide for 96 h. Before measurement, the CellTiter-Lumi™ luminescent cell viability assay reagent was equilibrated to room temperature in the dark. Then, 50 μL of the CellTiter-Lumi™ (Cat. No.: C0065XL) cell viability assay reagent was added to each well of the 96-well plate. The plate was shaken in the dark on a

shaker at 300 rpm for 10 min to ensure complete cell lysis. After the reaction was completed, the chemiluminescence intensity was measured on a multi-mode microplate reader. The cell viability inhibition rate was determined according to the chemiluminescence intensity. Then, a cell viability curve was generated by Graphpad Prism 8.0, and the IC50 values were calculated. The results are shown in Table 3.

Table 3. Activity of the compounds of the present disclosure in cell strains with high expression of efflux pump proteins (IC50 value)

| Compound No. | SNU-5 | HCT-15 | AsPc-1 |
|---|---|---|---|
| Smol006 | D | D | B |
| B19 | A | C | B |
| Note: in the table, A indicates an $IC_{50}$ value of < 50 nM, B indicates an $IC_{50}$ value between 50 nM and 200 nM, C indicates an $IC_{50}$ value between 200 nM and 1000 nM, and D indicates an $IC_{50}$ value of > 1000 nM. | | | |

The above results show that the compounds of the present disclosure have higher cell viability than Smol006 in cells with high expression of different efflux pump proteins, thus demonstrating greater application potential.

Test Example 4: Evaluation of Metabolic Stability of Compound of the Present Disclosure in Liver Microsomes

1. Test method

Each of the test compounds B61, B63, and B19 was incubated in duplicate with liver microsomes from mice, rats, dogs, monkeys, and humans (the final concentration of the system was 0.5 mg/mL). The final concentration of the substrate in the incubation system was 1 μM, and the incubation time was 60 min. Sampling was performed at 0 min, 5 min, 15 min, 30 min, and 60 min. The reaction was stopped with glacial acetonitrile containing an internal standard (testosterone-d3 at a concentration of 10 ng/mL). Verapamil was used as a positive control. The samples were analyzed by LC-MS/MS to determine the concentrations of B61, B63, and B19, and the remaining percentage was calculated. The natural logarithm of the remaining percentage was subjected to linear fitting with time to determine the elimination rate constant (k). The half-life ($t_{1/2}$), intrinsic clearance rate ($CL_{int}$, *in vitro),* and hepatic clearance rate ($CL_{hb}$) were calculated according to the following formulas:

$t_{1/2} = 0.693 / k$

$CL_{int}$, *in vitro* = k × incubation solution volume (mL)/liver microsome amount (mg) × (microsome (mg)/liver weight (g)) × (liver weight (g)/body weight (kg))

$CL_{hb}$ = hepatic blood flow (mL/min/kg) × CLint, *in vitro* / (hepatic blood flow (mL/min/kg) + CLint, *in vitro)*

Criteria for determination: $CL_{hb}$ less than 20% of hepatic blood flow indicates low clearance, and $CL_{hb}$ greater than 80% of hepatic blood flow indicates high clearance.

2. Results

The intrinsic clearance rates of B61 in liver microsomes from humans, monkeys, dogs, rats, and mice were 7.03 mL/min/kg, 13.7 mL/min/kg, 11.8 mL/min/kg, 19.8 mL/min/kg, and 32.2 mL/min/kg, respectively.

The intrinsic clearance rates of B63 in liver microsomes from humans, monkeys, and rats were 0.190 mL/min/kg, 0.257 mL/min/kg, and 1.39 mL/min/kg, respectively, and almost no elimination was observed in humans, monkeys, dogs, rats, and mice, with the substrate remaining percentages after 60 min of incubation being 98.8%, 101%, 103%, 94.9%, and 102%, respectively.

The intrinsic clearance rates of B19 in liver microsomes from humans, monkeys, rats, and mice were 5.80 mL/min/kg, 11.3 mL/min/kg, 6.55 mL/min/kg, and 17.0 mL/min/kg, respectively, with almost no elimination in dogs and mice, and the remaining percentages of the substrate after 60 min of incubation were 118% and 92.8%, respectively.

The results of metabolic stability of B61, B63, and B19 in liver microsomes from mice, rats, dogs, monkeys, and humans are shown in Table 4 below.

Table 4. Metabolic stability parameters of liver microsomes of different species

| Drug | Species | T1/2 (min) | Cl int (mL/min/kg) | Cl hb (mL/min/kg) | Parent drug remaining % |
|---|---|---|---|---|---|
| B61 | Human | 247 | 7.03 | 5.27 | 83.4 |
| | Monkey | 148 | 13.7 | 10.5 | 79.4 |
| | Dog | 293 | 11.8 | 8.55 | 86.4 |
| | Rat | 125 | 19.8 | 14.6 | 71.0 |
| | Mouse | 174 | 32.2 | 23.7 | 79.8 |
| B63 | Human | 9138 | 0.190 | 0.189 | 98.8 |
| | Monkey | 7892 | 0.257 | 0.255 | 101 |
| | Dog | - | - | - | 103 |
| | Rat | 1789 | 1.39 | 1.35 | 94.9 |
| | Mouse | - | - | - | 102 |
| B19 | Human | 300 | 5.80 | 4.55 | 80.5 |
| | Monkey | 180 | 11.3 | 8.98 | 77.7 |
| | Dog | - | - | - | 118 |
| | Rat | 379 | 6.55 | 5.85 | 88.1 |
| | Mouse | 329 | 17.0 | 14.3 | 92.8 |

Test Example *5: In Vitro* Anti-Tumor Activity of ADCs of the Present Disclosure

Tumor cell lines derived from different tumors were all commercially available, and the cells were all from ATCC (American Type Culture Collection) and used for evaluating the anti-tumor inhibitory activity of the ADCs of the present disclosure. Various tumor cells were cultured until the cell confluency reached 80%-90%, with cell viability above 90% as determined by trypan blue exclusion. Then, the cells were plated. Different cell strains were subjected to density optimization to 3000-5000 cells/well, 75 μL of each of the cell suspensions was plated in a BeyoGold™ ultra-low attachment black clear-bottom 96-well plate (Beyotime, Cat. No.: FULA965-24pcs), and the plate was cultured overnight. The next day, after the adherent cells were adhered to the wall, ADC samples diluted with the corresponding culture media were added. Specifically, the test ADC samples were serially diluted at a 5-fold gradient starting from the highest concentration of 100 nM, and then added to the cell culture plate. The plate was incubated in an incubator at 37 °C with 5% carbon dioxide for 6 days. Before measurement, the CellTiter-Lumi™ luminescent cell viability assay reagent was equilibrated to room temperature in the dark. Then, 50 μL of the CellTiter-Lumi™ (Beyotime, Cat. No.: C0065XL) cell viability assay reagent was added to each well of the 96-well plate. The plate was shaken in the dark on a shaker at 300 rpm for 10 min to ensure complete cell lysis. After the reaction was completed, the luminescence was measured on a multi-mode microplate reader. The cell inhibition rate was determined according to the luminescence. Then, a cell viability curve was generated by Graphpad Prism 8.0, and the IC50 values were calculated. The activity data of each ADC sample in different tumor cell strains are shown in the table below:

**BT-474**

| **ADC drug No.** | IC50 (nM) | Max inhibition rate (%) |
|---|---|---|
| **ORM-5029** | 0.01528 | 81.36 |
| **ADC01-01-8** | 0.002829 | 76.94 |
| **ADC07-01-8** | 0.003748 | 73.38 |
| **ADC01-01-4** | 0.005559 | 79.37 |
| **ADC07-01-4** | 0.0001949 | 77.91 |

**SK-BR-3**

| ADC drug No. | IC50 (nM) | Max inhibition rate (%) |
|---|---|---|
| **ORM-5029** | 0.04314 | 95.57 |
| **ADC01-01-8** | 0.008852 | 97.20 |
| **ADC07-01-8** | 0.01296 | 97.11 |
| **ADC01-01-4** | 0.01479 | 96.65 |
| **ADC07-01-4** | 0.03135 | 97.40 |

**NCI-N87**

| ADC drug No. | IC50 (nM) | Max inhibition rate (%) |
|---|---|---|
| **ADC01-01-8** | 0.2212 | 74.44 |
| **ADC07-01-8** | 0.175 | 65.23 |
| **ADC01-01-4** | 0.3965 | 79.63 |
| **ADC07-01-4** | 0.6134 | 67.37 |

**T47D**

| ADC drug No. | IC50 (nM) | Max inhibition rate (%) |
|---|---|---|
| **ADC01-01-8** | 1.449 | 87.07 |
| **ADC07-01-8** | 1.02 | 86.69 |
| **ADC01-01-4** | 7.064 | 83.98 |
| **ADC07-01-4** | 7.838 | 84.26 |

**SK-BR-3**

| ADC drug No. | IC50 (nM) | Max inhibition rate (%) |
|---|---|---|
| **ORM-5029** | 0.02809 | 97.30 |
| **ADC01-02-8** | 0.003352 | 97.76 |
| **ADC01-06-8** | 0.01116 | 96.38 |
| **ADC01-06-4** | 0.02107 | 97.32 |
| **ADC01-08-8** | 0.0089 | 97.17 |
| **ADC01-08-4** | 0.0179 | 97.14 |

**NCI-H82**

| ADC drug No. | IC50 (nM) | Max inhibition rate (%) |
|---|---|---|
| **ADC04-01-8** | 0.06064 | 93.64 |
| **ADC04-09-8** | 0.2235 | 79.01 |
| **ADC04-02-8** | 0.0227 | 91.54 |
| **ADC04-07-8** | 0.04488 | 89.01 |
| **ADC04-08-8** | 0.118 | 94.01 |

**NCI-H929**

| ADC drug No. | IC50 (nM) | Max inhibition rate (%) |
|---|---|---|
| **ADC08-01-4** | 0.07176 | 96.90 |
| **ADC08-01-8** | 0.06538 | 96.22 |

The ADC samples obtained using the small-molecule compounds of the present disclosure as payloads all have high activity in tumor cells of different indications, thus demonstrating great application prospects.

## Claims

**1.** A compound having a structure represented by formula (I), or a tautomer, a mesomer, a racemate, an enantiomer or a diastereoisomer thereof, and a pharmaceutically acceptable salt, a hydrate or a solvate thereof:

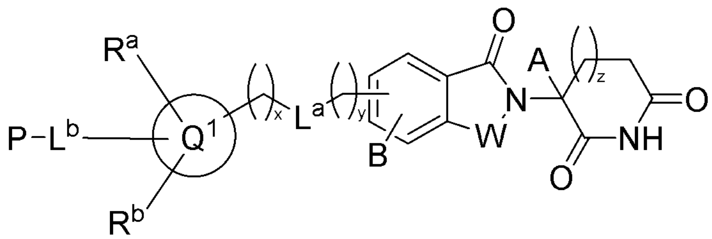

I

wherein,

A is selected from H, D, halogen, $C_{1-6}$ alkyl, $C_{1-6}$ deuterated alkyl, and $C_{1-6}$ haloalkyl;
B is selected from H, D, halogen, $-NH_2$, $-NO_2$, $C_{1-6}$ alkyl, $C_{1-6}$ deuterated alkyl, and $C_{1-6}$ haloalkyl;
W is selected from -C(=O)- or $-CH_2-$;
$L^a$ is an alkylene chain of 1 to 4 carbons, wherein each alkylene can be independently replaced with -NH-, -O-, -C(=O)-, -C(=NH)-, -C(=S)-, $-CF_2-$, -S(=O)-, $-S(=O)_2-$, -C(-OH)H-, $-C(NH_2)H-$, or -C(=N-C≡N)-;
x is selected from an integer of 0-4, such as 0, 1, 2, 3, or 4;
y is selected from an integer of 0-4, such as 0, 1, 2, 3, or 4;
z is selected from an integer of 0-4, such as 0, 1, 2, 3, or 4;
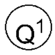 is selected from $C_{6-14}$ aryl, 5- to 6-membered heteroaryl, and 3- to 18-membered cycloalkyl;
$R^a$ and $R^b$ are each independently selected from H, D, halogen, -OH, -CN, $-NO_2$, $-NH_2$, $C_{1-6}$ alkyl, $C_{1-6}$ haloalkyl, $C_{1-6}$ alkoxy, $C_{1-6}$ haloalkoxy, $-R^c$, $-OR^c$, $-CH(R^c)OH$, $-CH_2CH(R^c)OH$, and $-NH(R^c)$;
$R^c$ is selected from H, 3- to 8-membered cycloalkyl, $C_{6-14}$ aryl, benzyl, and $C_{1-6}$ alkyl;
$L^b$ is selected from optionally substituted $C_{1-20}$ (such as $C_{1-18}$, $C_{1-16}$, $C_{1-14}$, $C_{1-12}$, $C_{1-10}$, $C_{1-8}$, $C_{1-6}$, $C_{1-4}$, $C_{3-12}$, $C_1$, $C_2$, $C_3$, $C_4$, $C_5$, $C_6$, $C_7$, $C_8$, $C_9$, $C_{10}$, $C_{11}$, $C_{12}$, $C_{13}$, $C_{14}$, $C_{15}$, and $C_{16}$) alkylene, wherein each alkylene can be independently replaced with $-CR^eR^f-$, -O-, -S-, $-NR^d-$, 3- to 8-membered cycloalkylene, or 3- to 8-membered heterocyclylene;

$R^d$ is selected from H, $C_{1-12}$ alkyl, 3- to 8-membered cycloalkyl, benzyl, $C_{6-14}$ aryl, 5- to 6-membered heteroaromatic ring, $C_{1-12}$ alkoxycarbonyl, -Boc, -Cbz, -Fmoc, acetyl, and trifluoroacetyl;

$R^e$ and $R^f$ are each independently selected from H, $C_{1-12}$ alkyl, 3- to 8-membered cycloalkyl, benzyl, $C_{6-14}$ aryl, and 5- to 6-membered heteroaryl;

P is selected from -H; when the atom attached to P is a nitrogen atom, P can be selected from linear or branched aliphatic oxycarbonyl of 1-12 carbons, -Boc, -Cbz, -Fmoc, formyl, acetyl, and trifluoroacetyl; when the atom attached to P is an oxygen atom, P can be selected from acetyl, trifluoroacetyl, trimethylsilyl, dimethyl *tert*-butylsilyl, and diphenyl *tert*-butylsilyl.

**2.** A compound having a structure represented by formula (I-1), or a tautomer, a mesomer, a racemate, an enantiomer or a diastereoisomer thereof, and a pharmaceutically acceptable salt, a hydrate or a solvate thereof:

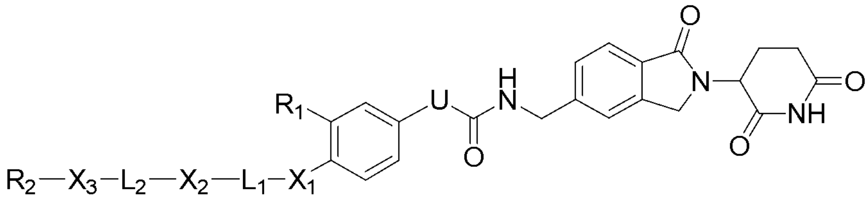

I-1

wherein U is selected from -NH-, $-CH_2-$, and $-CF_2-$;

$R_1$ is selected from hydrogen, halogen, $C_{1-6}$ alkyl, $C_{1-6}$ haloalkyl, and 3- to 8-membered cycloalkyl;

$X_1$ and $X_2$ are each independently selected from -O-, -S-, and a bond;

$X_3$ is selected from $-N(R_7)-$ and a bond;

$L_1$ and $L_2$ are each independently selected from: a bond, $-C_{1-6}$ alkyl-, $-C_{1-6}$ alkyl-$CH(R_3)$-$C_{1-6}$ alkyl-, $-C(R_3)(R_4)$-$C_{1-6}$ alkyl-, $-C_{1-6}$ alkyl-3- to 8-membered cycloalkyl-$C_{1-6}$ alkyl-, -3- to 8-membered cycloalkyl-$C_{1-6}$ alkyl-, $-C_{1-6}$ alkyl-3- to 8-membered heterocyclyl-$C_{1-6}$ alkyl-, and -3- to 8-membered heterocyclyl-$C_{1-6}$ alkyl-;

$R_3$ and $R_4$ are each independently selected from H, $C_{1-6}$ alkyl, $C_{6-14}$ aryl, and 3- to 8-membered cycloalkyl, or $R_3$ and $R_4$, together with the carbon atom attached thereto, form 3- to 8-membered cycloalkyl or 3- to 8-membered heterocyclyl;

$R_2$ is selected from H and the following optionally substituted substituents: $C_{1-6}$ alkyl, 3- to 16-membered cycloalkyl, 3- to 16-membered heterocyclyl, $-C_{1-6}$ alkyl-$C(R_5)(R_6)$-$C_{1-6}$ alkyl-, and $-C_{1-6}$ alkyl-$C(R_5)(R_6)$-;

$R_5$ and $R_6$ are each independently selected from: H, $C_{1-6}$ alkyl, $C_{6-14}$ aryl, and 3- to 8-membered cycloalkyl, or $R_5$ and $R_6$, together with the carbon atom attached thereto, form 3- to 8-membered cycloalkyl or 3- to 8-membered heterocyclyl;

$R_7$ is selected from: H and the following optionally substituted substituents: $C_{1-6}$ alkyl, 3- to 8-membered cycloalkyl, and 3- to 8-membered heterocyclyl, or $-N(R_7)(R_2)$ forms 3- to 16-membered heterocyclyl,

wherein the optionally substituted substituents are selected from -OH, $C_{1-6}$ alkyl, $(R_8)(R_9)NH-$, 3- to 8-membered heterocyclyl, $C_{1-6}$ alkyloxycarbonyl (such as tert-butoxycarbonyl (Boc), methoxycarbonyl, ethoxycarbonyl, and propoxycarbonyl), benzyloxycarbonyl (Cbz), fluorenylmethoxycarbonyl (Fmoc), and allyloxycarbonyl (Alloc);

$R_8$ and $R_9$ are each independently selected from H, $C_{1-6}$ alkyl, 3- to 8-membered cycloalkyl, 3- to 8-membered heterocyclyl, $C_{6-14}$ aryl, and benzyl;

heteroatoms of the heterocyclyl are selected from O, S, and N.

**3.** The compound having a structure represented by formula (I-1), or the tautomer, the mesomer, the racemate, the enantiomer or the diastereoisomer thereof, and the pharmaceutically acceptable salt, the hydrate or the solvate thereof according to claim 2, wherein formula (I-1) has structures represented by formula (I-2) to formula (I-11):

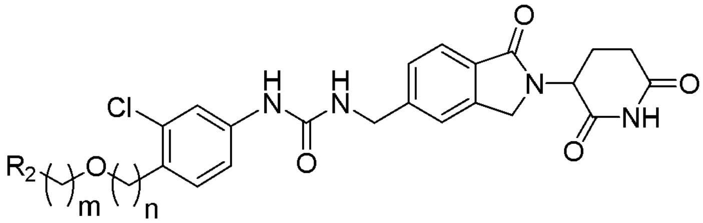

I-2

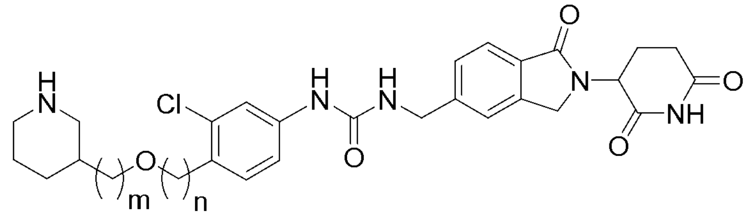

I-3

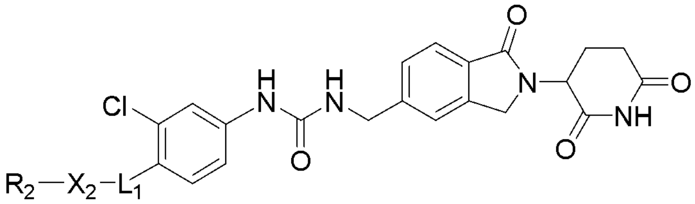

I-4

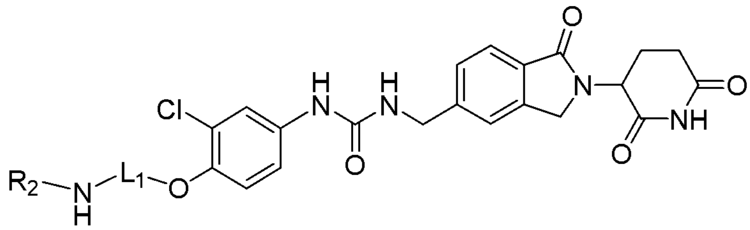

I-5

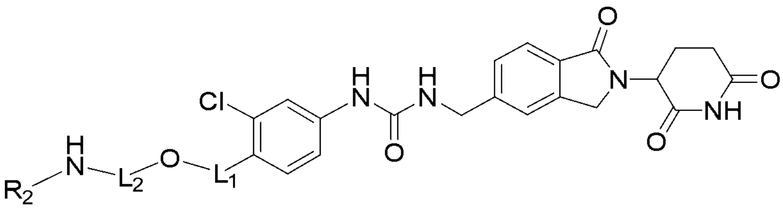

I-6

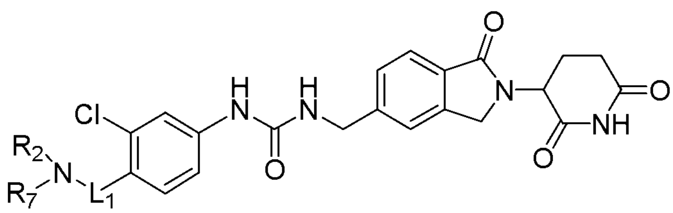

I-7

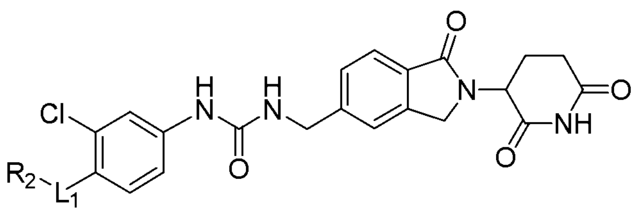

I-8

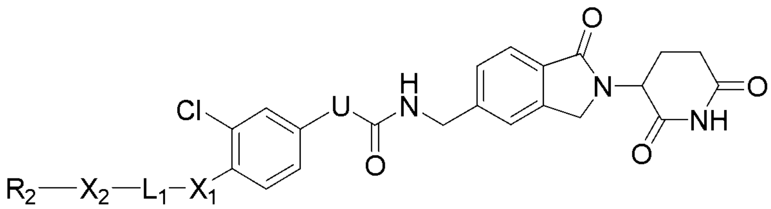

I-9

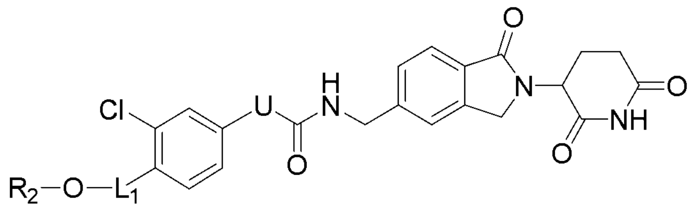

I-10

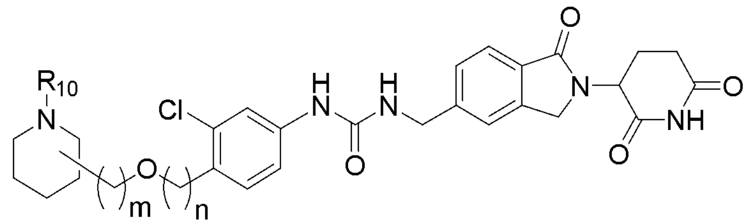

I-11a

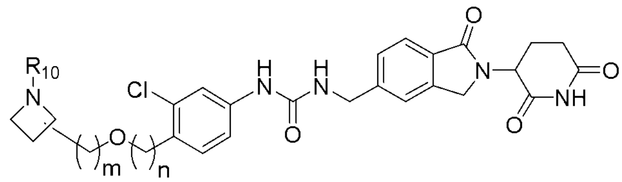

I-11b

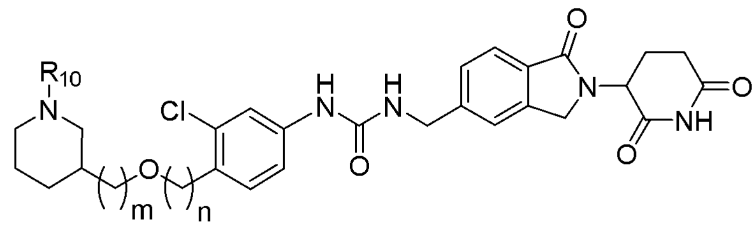

I-12a

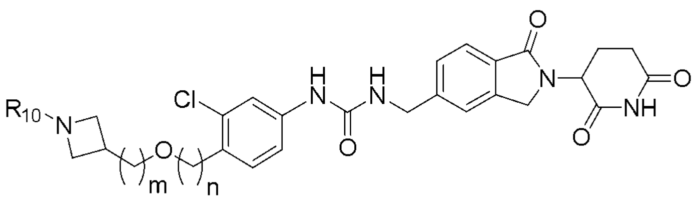

I-12b

wherein U, $X_1$, $X_2$, $L_1$, $L_2$, $R_2$, and $R_7$ are defined as in formula (I-1);
$R_{10}$ is selected from $C_{1-6}$ alkyloxycarbonyl (such as $C_{1-3}$ alkyloxycarbonyl, tert-butoxycarbonyl (Boc), methoxycarbonyl, ethoxycarbonyl, and propoxycarbonyl), benzyloxycarbonyl (Cbz), fluorenylmethoxycarbonyl (Fmoc),

and allyloxycarbonyl (Alloc);
m and n are each independently selected from an integer of 0-6, such as 0, 1, 2, 3, 4, 5, or 6.

**4.** The compounds having structures represented by formula (I-1) to formula (I-11), or the tautomers, the mesomers, the racemates, the enantiomers or the diastereoisomers thereof, and the pharmaceutically acceptable salts, the hydrates or the solvates thereof according to claim 2 or 3, wherein $R_2$ is selected from the following optionally substituted substituents: 3- to 8-membered monocyclic heterocyclyl, 6- to 14-membered spiro heterocyclyl, 5- to 14-membered fused heterocyclyl, and 5- to 14-membered bridged heterocyclyl, wherein heteroatoms of the heterocyclyl are selected from nitrogen.

**5.** The compounds having structures represented by formula (I-1) to formula (I-11), or the tautomers, the mesomers, the racemates, the enantiomers or the diastereoisomers thereof, and the pharmaceutically acceptable salts, the hydrates or the solvates thereof according to claim 2 or 3, wherein $R_2$ is selected from the following optionally substituted substituents: azetidinyl, pyrrolidinyl, piperidinyl,

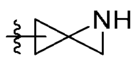, 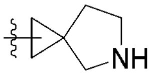, 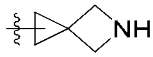,

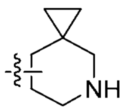, 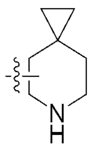, 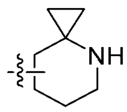, 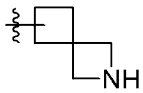, 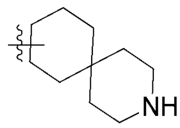, 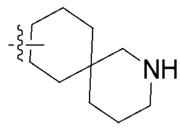, 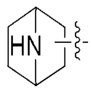,

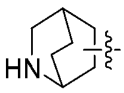, 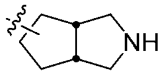, 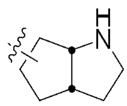, 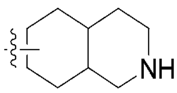, 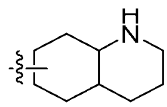, 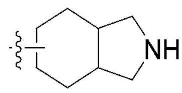,

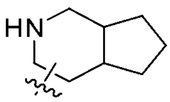, 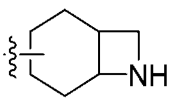, 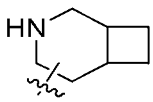, 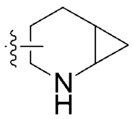, and 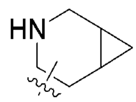,

wherein

“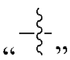”

represents a linking bond.

**6.** The compounds having structures represented by formula (I-1) to formula (I-11), or the tautomers, the mesomers, the racemates, the enantiomers or the diastereoisomers thereof, and the pharmaceutically acceptable salts, the hydrates or the solvates thereof according to claim 2 or 3, wherein: $L_1$ and $L_2$ are each independently selected from methylene,

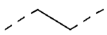, 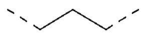, 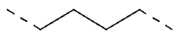,

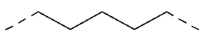, 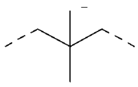, 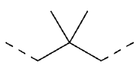, 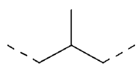, 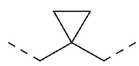, 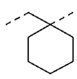, 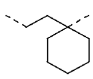, 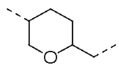,

and

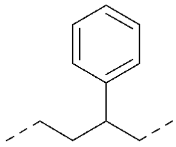;

$R_2$ is selected from the following optionally substituted substituents:

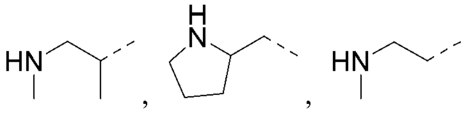
,
,
,

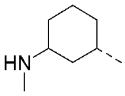, 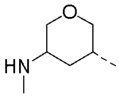, 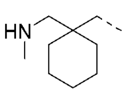, 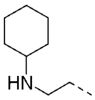, 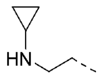, 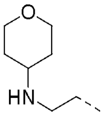, 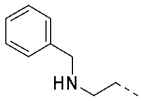, 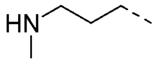,

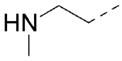, 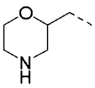, 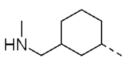, 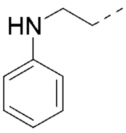, 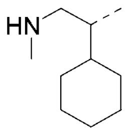, 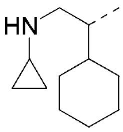, 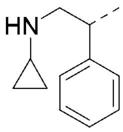, 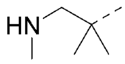,

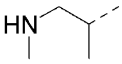, 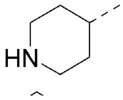, 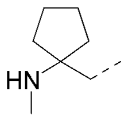, 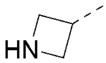, 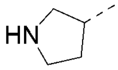, 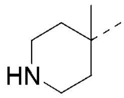, 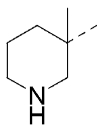, 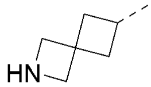,

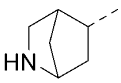, 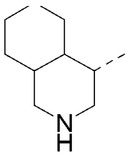, 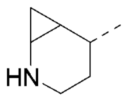, 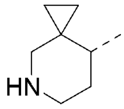, 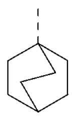, 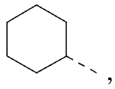, 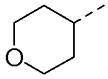, 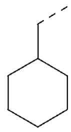, 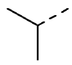,

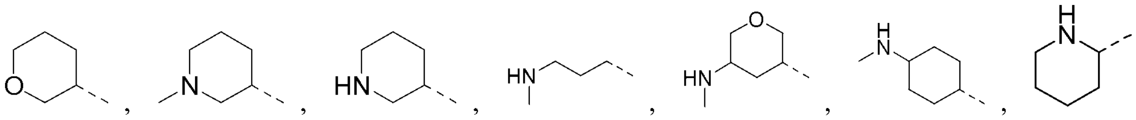
, , , , , , ,

and

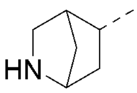;

$R_7$ is selected from H, methyl, and

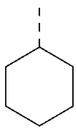;

or $R_2$ and $R_7$, together with the N connected thereto, form

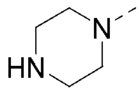

;

the optionally substituted substituents are selected from: H, $C_{1-3}$ alkyl, $C_{1-6}$ alkyloxycarbonyl (such as $C_{1-3}$ alkyloxycarbonyl, tert-butoxycarbonyl (Boc), methoxycarbonyl, ethoxycarbonyl, and propoxycarbonyl), benzyloxycarbonyl (Cbz), fluorenylmethoxycarbonyl (Fmoc), and allyloxycarbonyl (Alloc),
wherein "---" represents a linking bond.

**7.** The compound having the structure represented by formula (I) or formula (I-1), or the tautomer, the mesomer, the racemate, the enantiomer or the diastereoisomer thereof, and the pharmaceutically acceptable salt, the hydrate or the solvate thereof according to claim 1 or 2, having the following structures:

| | |
|---|---|
| 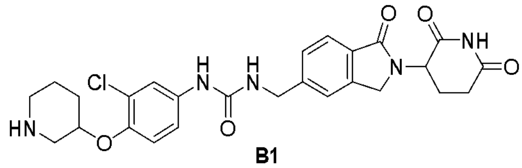 B1 | 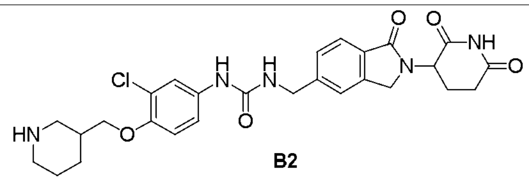 B2 |
| 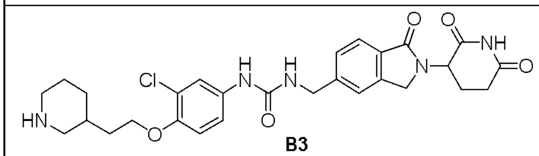 B3 | 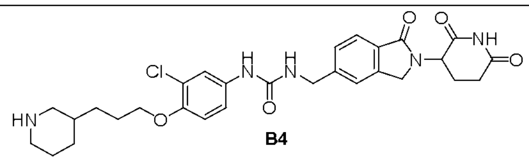 B4 |
| 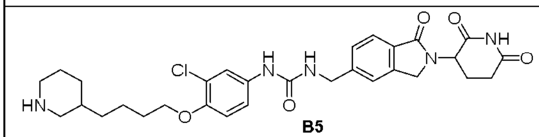 B5 | 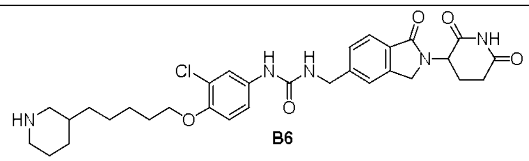 B6 |
| 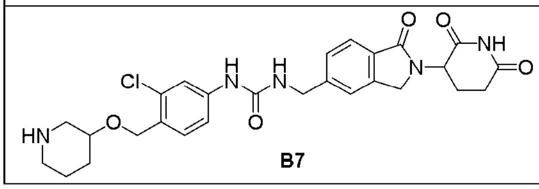 B7 | 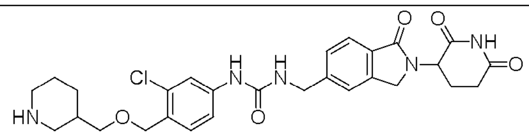 B8 |

| | |
|---|---|
| 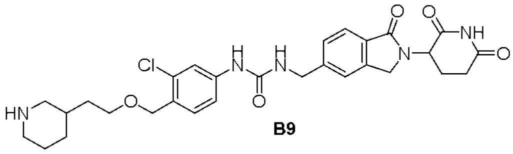 B9 | 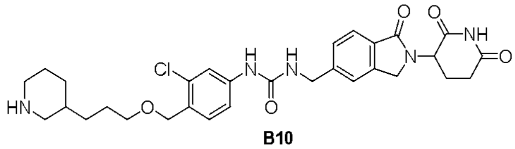 B10 |
| 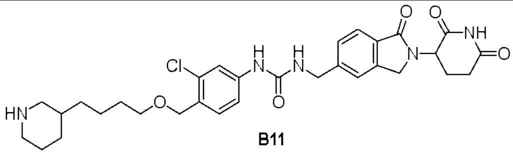 B11 | 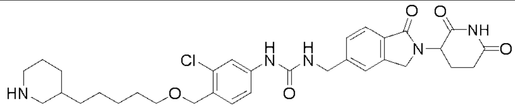 B12 |
| 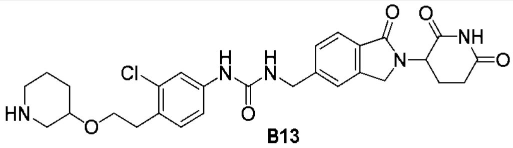 B13 | 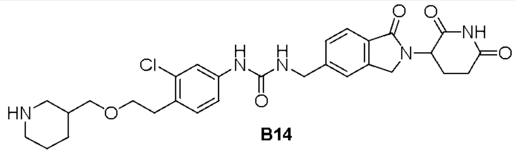 B14 |
| 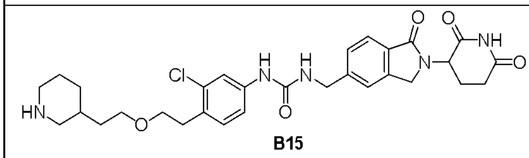 B15 | 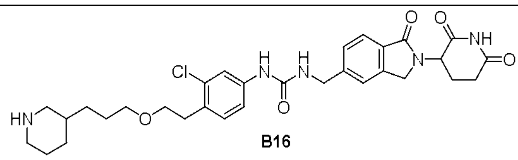 B16 |
| 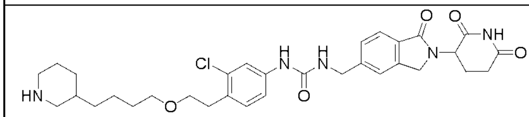 B17 | 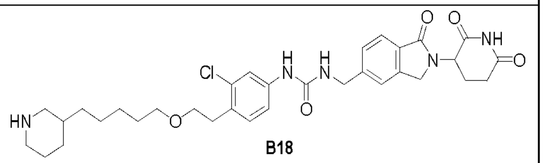 B18 |
| 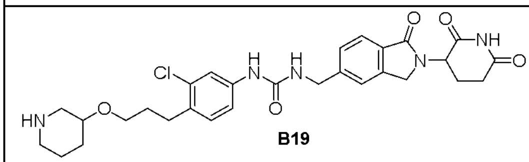 B19 | 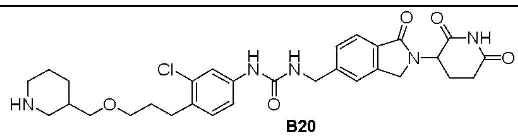 B20 |
| 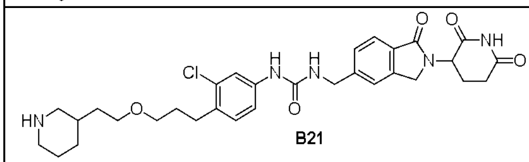 B21 | 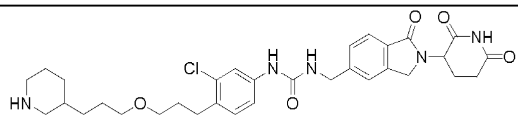 B22 |
| 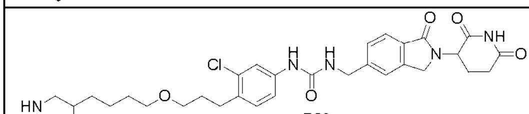 B23 | 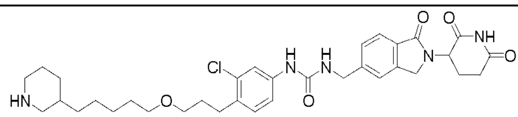 B24 |
| 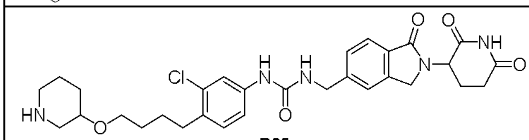 B25 | 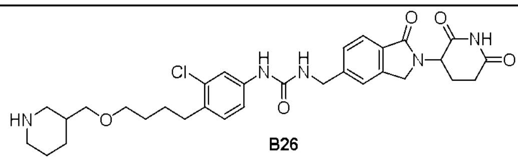 B26 |
| 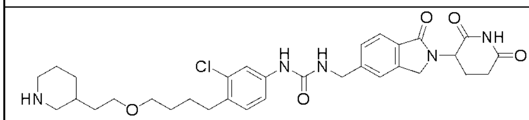 B27 | 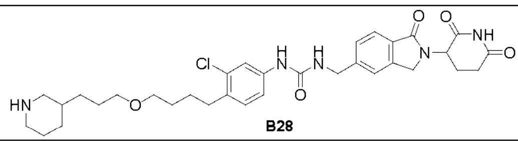 B28 |
| 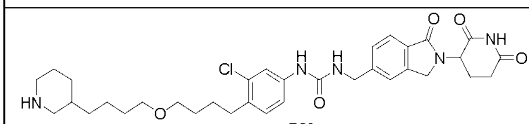 B29 | 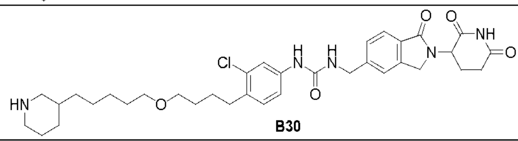 B30 |
| 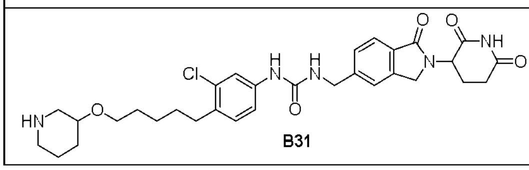 B31 | 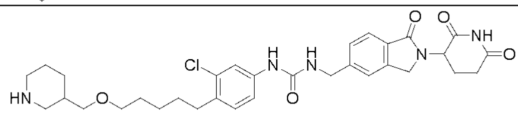 B32 |

| | |
|---|---|
| 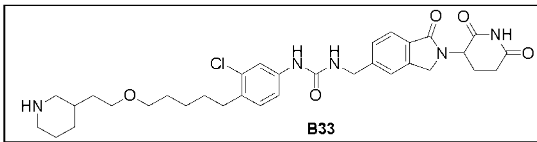 B33 | 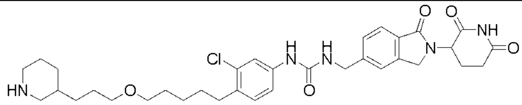 B34 |
| 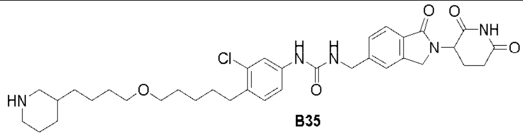 B35 | 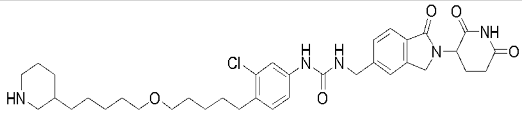 B36 |
| 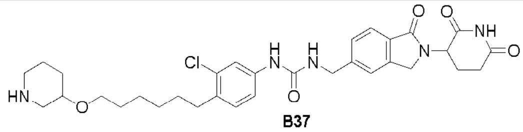 B37 | 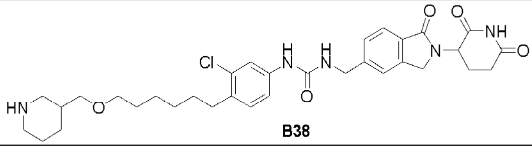 B38 |
| 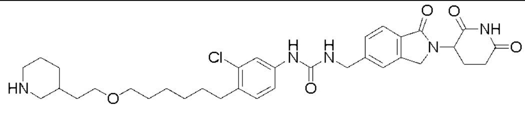 B39 | 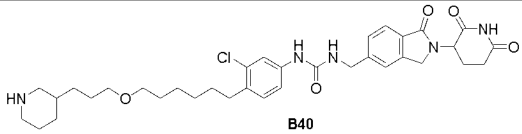 B40 |
| 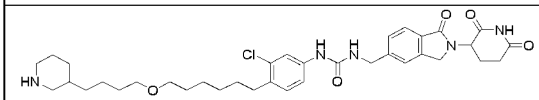 B41 | 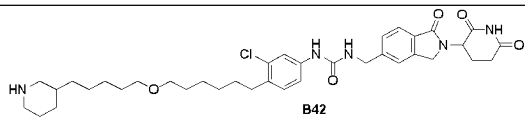 B42 |
| 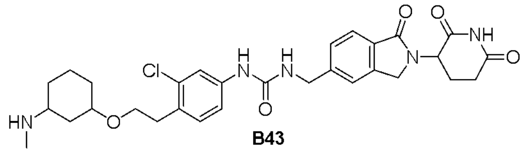 B43 | 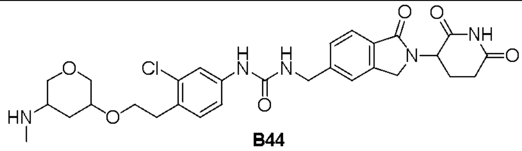 B44 |
| 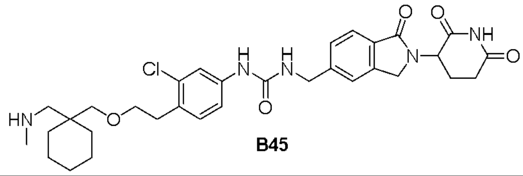 B45 | 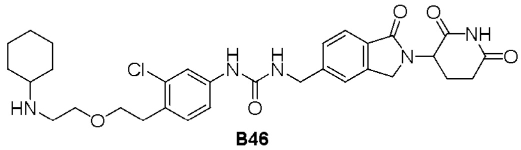 B46 |
| 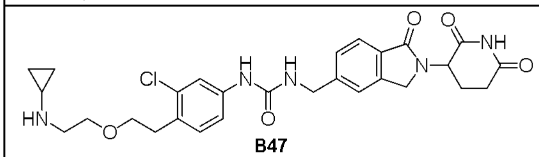 B47 | 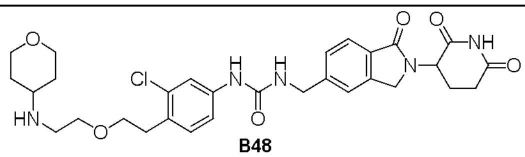 B48 |
| 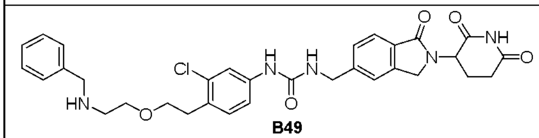 B49 | 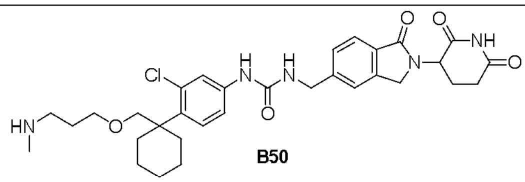 B50 |
| 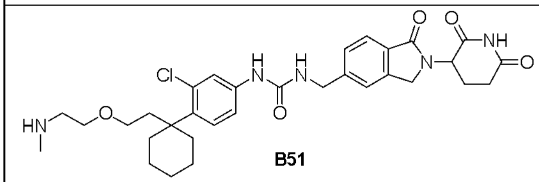 B51 | 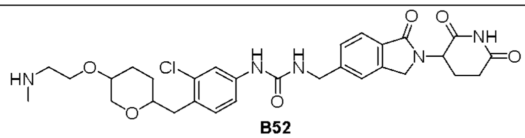 B52 |
| 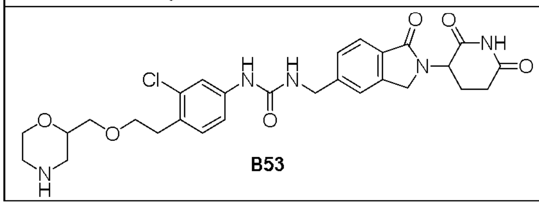 B53 | 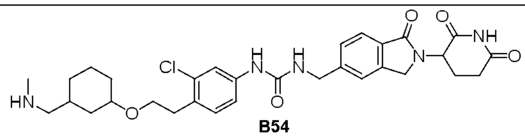 B54 |

| | |
|---|---|
| 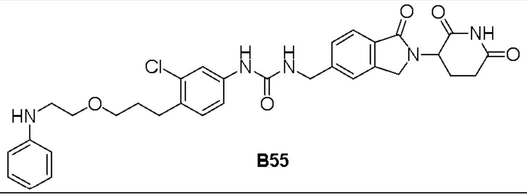 B55 | 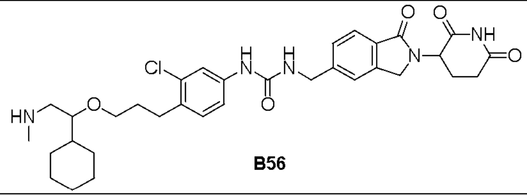 B56 |
| 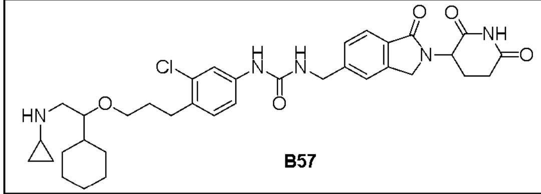 B57 | 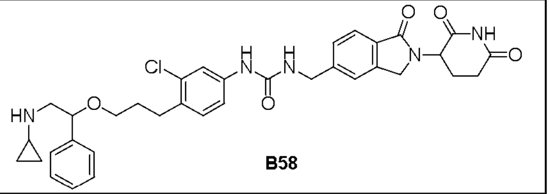 B58 |
| 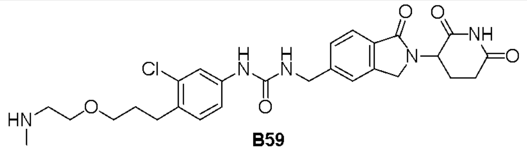 B59 | 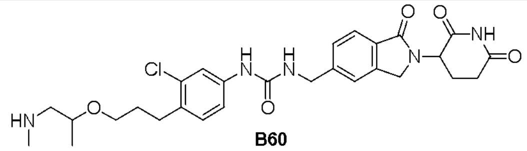 B60 |
| 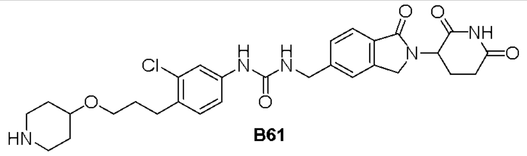 B61 | 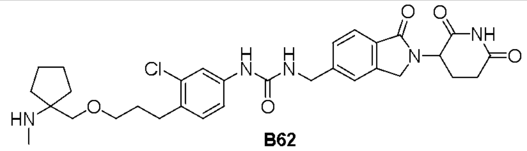 B62 |
| 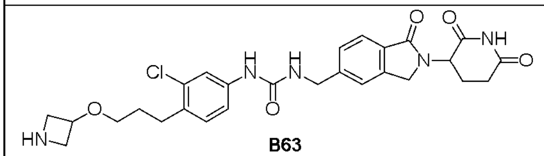 B63 | 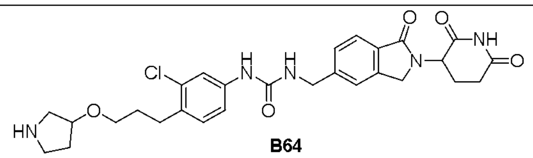 B64 |
| 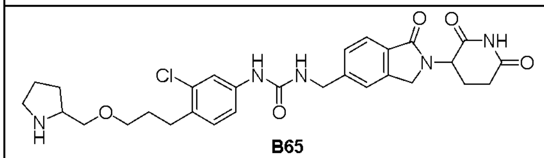 B65 | 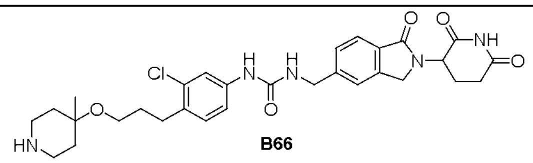 B66 |
| 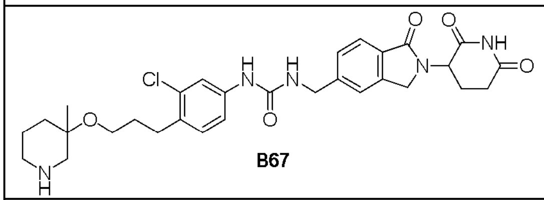 B67 | 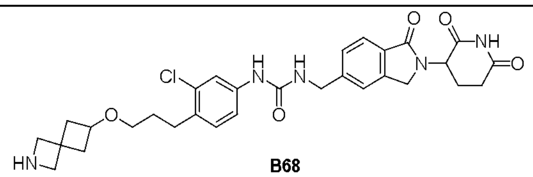 B68 |
| 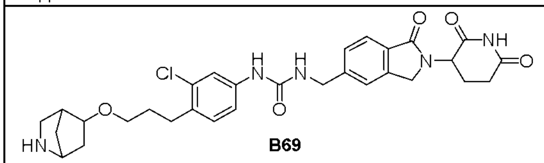 B69 | 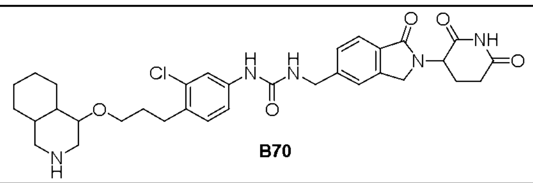 B70 |
| 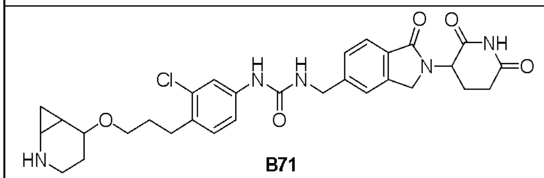 B71 | 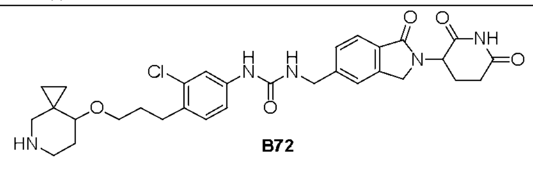 B72 |
| 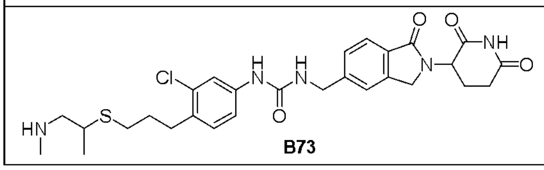 B73 | 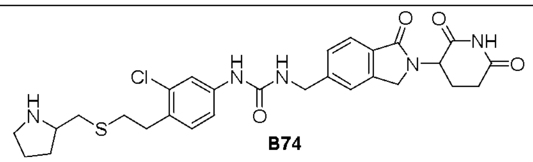 B74 |

| | |
|---|---|
| 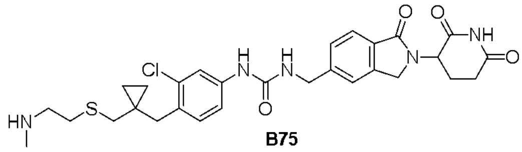 B75 | 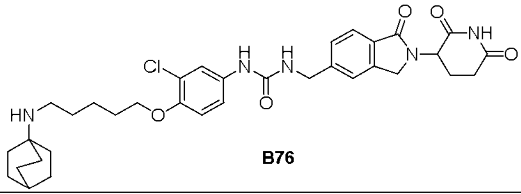 B76 |
| 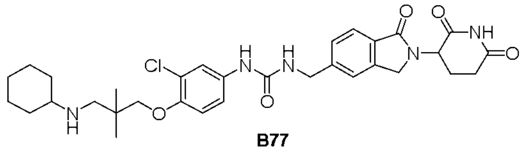 B77 | 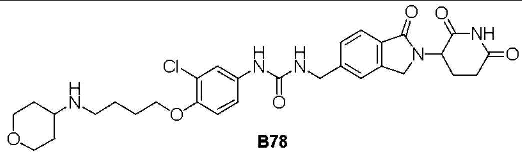 B78 |
| 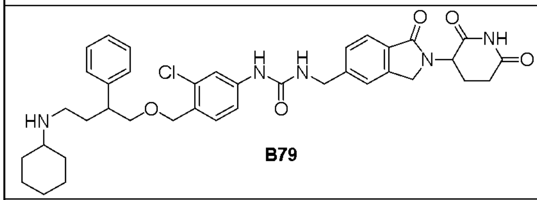 B79 | 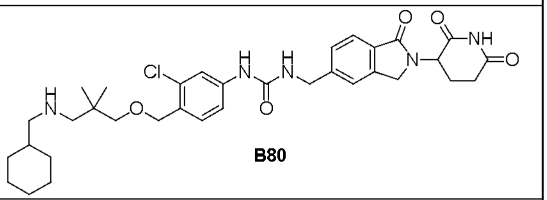 B80 |
| 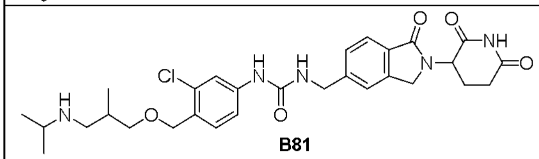 B81 | 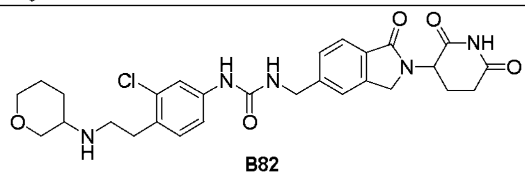 B82 |
| 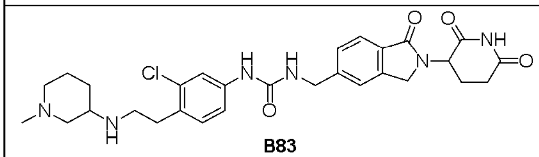 B83 | 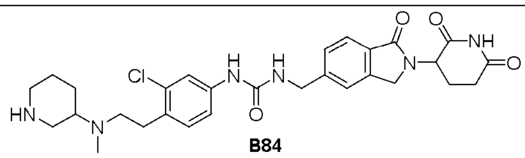 B84 |
| 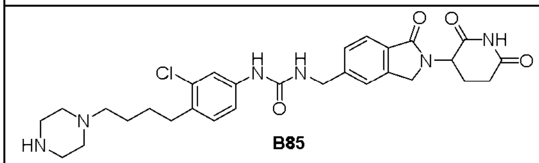 B85 | 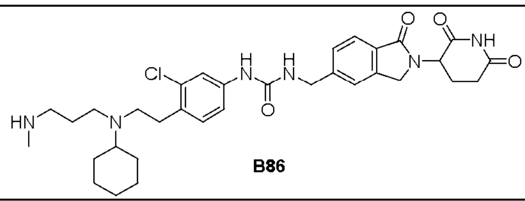 B86 |
| 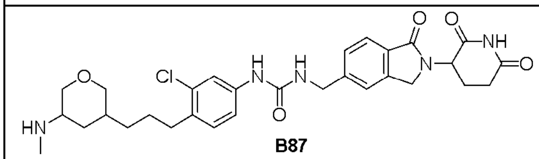 B87 | 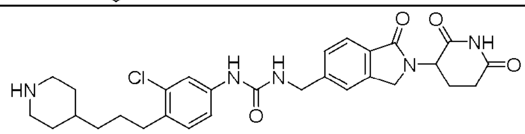 B88 |
| 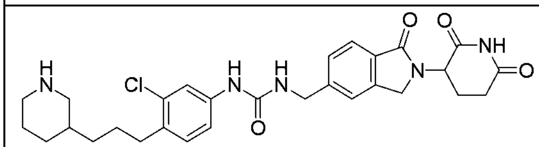 B89 | 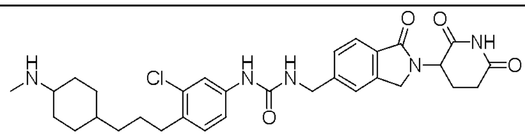 B90 |
| 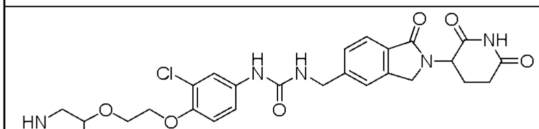 B91 | 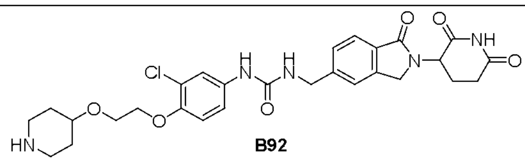 B92 |
| 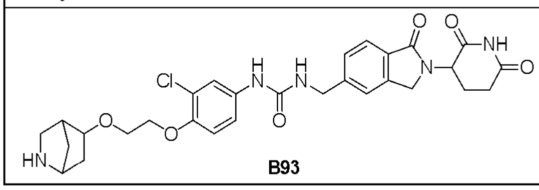 B93 | 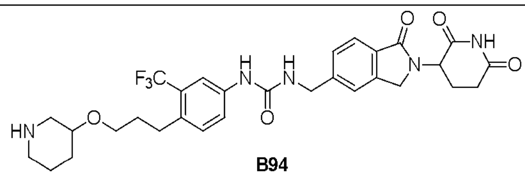 B94 |

| | |
|---|---|
| 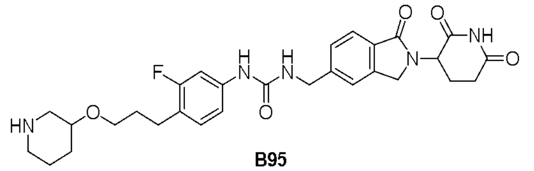 B95 | 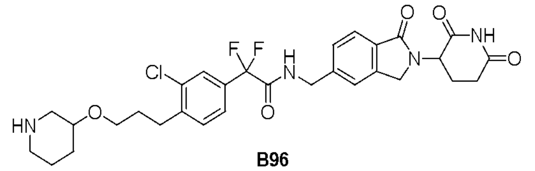 B96 |

.

**8.** An antibody-drug conjugate of the following formula (II), and a pharmaceutically acceptable salt, a hydrate, a solvate, a stereoisomer or an isotopically labeled compound thereof:

$$Ab\text{-}(L\text{-}D)_d \qquad II$$

wherein:

Ab is selected from an antibody or an antigen-binding fragment;
L is a linker moiety, with one end attached to Ab and the other end attached to a bioactive molecule D;
D is a structure formed by attaching the compound represented by formula (I) according to claim 1 to L;
d is selected from an integer or a decimal of 1-8, such as 1, 2, 3, 4, 5, 6, 7, or 8;
when d is a decimal, d refers to an average number of linker-drug molecules conjugated to each antibody unit.

**9.** The antibody-drug conjugate, and the pharmaceutically acceptable salt, the hydrate, the solvate, the stereoisomer or the isotopically labeled compound thereof according to claim 8, wherein D is a structure formed by attaching the compound represented by formula (I-1) according to claim 2 or the compounds represented by formula (I-2) to formula (I-12) according to claim 3 to L; further preferably, D is a structure formed by attaching compounds B1-B96 according to claim 7 to L, preferably, via a nitrogen atom, and further preferably, via a nitrogen atom on $R_2$ or attached to $R_2$.

**10.** The antibody-drug conjugate, and the pharmaceutically acceptable salt, the hydrate, the solvate, the stereoisomer or the isotopically labeled compound thereof according to claim 8, wherein Ab is selected from an antibody targeting HER2, DLL3, Claudin18.2, or BCMA, or an antigen-binding fragment thereof.

**11.** The antibody-drug conjugate, and the pharmaceutically acceptable salt, the hydrate, the solvate, the stereoisomer or the isotopically labeled compound thereof according to claim 8, wherein -L- is selected from -S-L'-T-C-, wherein S is an active functional group for attachment to the antibody, L' is a spacer fragment or a chemical bond, T is an optionally present functional group triggering cleavage of the linker, and C is an optionally present self-immolative spacer fragment.

**12.** The antibody-drug conjugate, and the pharmaceutically acceptable salt, the hydrate, the solvate, the stereoisomer or the isotopically labeled compound thereof according to claim 11, wherein S is formed from any group Sr that reacts with the antibody or the antigen-binding fragment Ab, and Sr preferably comprises a sulfhydryl reactive group, an amino reactive group, a carboxyl reactive group, a disulfide bridging group, etc.; for an antibody into which an unnatural amino acid is introduced, Sr can also comprise a click chemistry reactive group such as ketone, hydrazine or hydrazide, azide, or alkyne such as cyclotonic alkyne, cyclopropene or diene;

preferably, Sr comprises a methylsulfonylpyrimidine group or a maleimide group;
preferably, Sr further comprises any linker fragment $S_L$, wherein $S_L$ is optionally substituted $C_{1\text{-}10}$ alkylene, alkenylene or alkynylene, $C_{6\text{-}12}$ arylene or $C_{3\text{-}12}$ cycloalkylene, $C_{2\text{-}11}$ heteroarylene or $C_{2\text{-}11}$ heterocyclylene, or a combination thereof, and is optionally interrupted by O, CO, NH, or a combination thereof; further preferably, $S_L$ comprises alkynyl or a polyethylene glycol fragment; further preferably, $S_L$ comprises cyclohexyl, phenyl, triazolyl, piperidinyl, or piperazinyl.

**13.** The antibody-drug conjugate, and the pharmaceutically acceptable salt, the hydrate, the solvate, the stereoisomer or the isotopically labeled compound thereof according to claim 11, wherein L' comprises a hydrophilic modification fragment such as a polyethylene glycol fragment or a polysarcosine fragment; or L' comprises an amino acid residue having a tertiary amine or quaternary ammonium group in the side chain.

**14.** The antibody-drug conjugate, and the pharmaceutically acceptable salt, the hydrate, the solvate, the stereoisomer or the isotopically labeled compound thereof according to claim 11, wherein T is a peptide fragment, specifically a

divalent peptide group comprising 1 to 8 (specifically 1, 2, 3, 4, 5, 6, 7, or 8) optionally substituted natural or non-natural, L- or D-amino acid residues, wherein each of the amino acid residues is the same or different, and is a residue of an amino acid independently selected from the following: alanine (Ala), cysteine (Cys), aspartic acid (Asp), glutamic acid (Glu), phenylalanine (Phe), glycine (Gly), histidine (His), isoleucine (Ile), lysine (Lys), leucine (Leu), methionine (Met), asparagine (Asn), proline (Pro), glutamine (Gln), arginine (Arg), serine (Ser), threonine (Thr), valine (Val), tryptophan (Trp), tyrosine (Tyr), citrulline (Cit), norvaline (Nva), norleucine (Nle), selenocysteine (Sec), pyrrolysine (Pyl), homoserine, homocysteine, desmethylpyrrolysine, and analogs of the amino acids described above; e.g., -ValCit-, -CitVal-, -AlaAla-, -AlaCit-, -CitAla-, -AsnCit-, -CitAsn-, -CitCit-, -ValGlu-, -GluVal-, -SerCit-, -CitSer-, -LysCit-, -CitLys-, -AspCit-, -CitAsp-, -AlaVal-, -ValAla-, -PheAla-, -AlaPhe-, - PheLys-, -LysPhe-, -ValLys-, -LysVal-, -AlaLys-, -LysAla-, -PheCit-, -CitPhe-, -LeuCit-, -CitLeu-, -IleCit-, - CitIle-, -PheArg-, -ArgPhe-, -CitTrp-, -TrpCit-, -AlaAlaAla-, -PhePheLys-, -LysPhePhe-, -DPhePheLys-, - DLysPhePhe-, -GlyPheLys-, -LysPheGly-, -GlyPheLeuGly-, -GlyLeu-PheGly-, -GluValCit-, -AlaLeuAlaLeu-, - GlyGlyGly-, -GlyGlyGlyGly-, -GlyPheValGly-, -GlyValPheGly-, -GlyGly-PheGly-, or -GlyGlyValGly-; preferably, L' is located at both ends of the peptide fragment T or between any two amino acids or replaces any one amino acid. 14. The antibody-drug conjugate, and the pharmaceutically acceptable salt, the hydrate, the solvate, the stereoisomer or the isotopically labeled compound thereof according to claim 12, wherein formula (II) has a structure represented by formula (II-a1) or formula (II-a2):

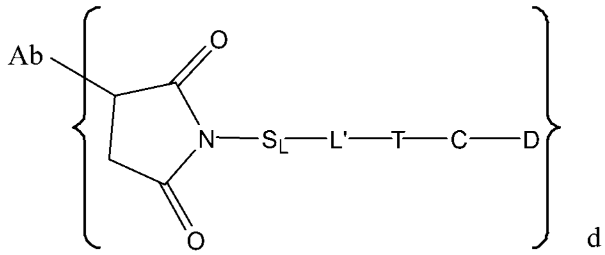

(II-a1)

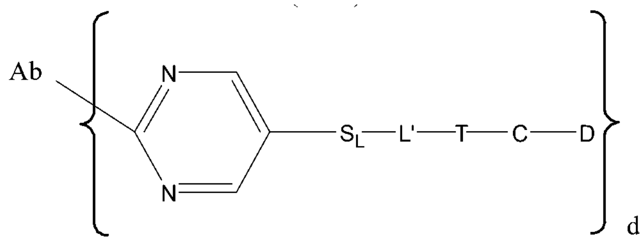

(II-a2)

wherein Ab, $S_L$, L', T, C, D, and d have the same meanings as in claim 12;
preferably, formula (II) has structures represented by formula (II-b1) to formula (II-b4):

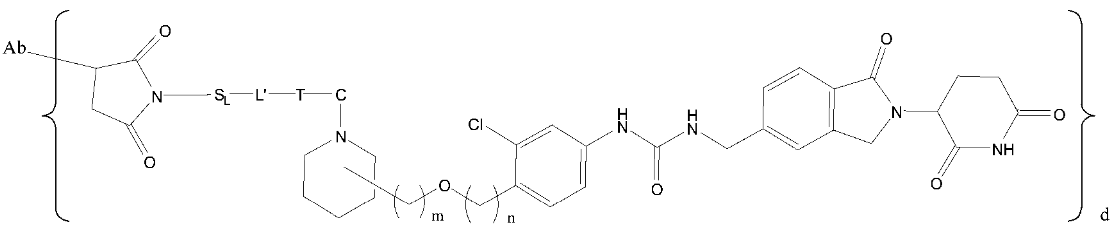

(II-b1)

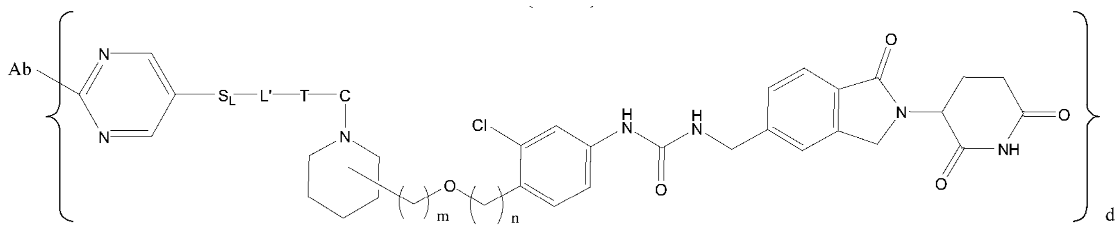

(II-b2)

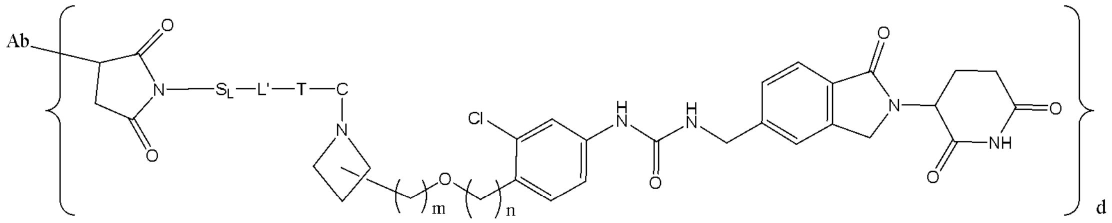

(II-b3)

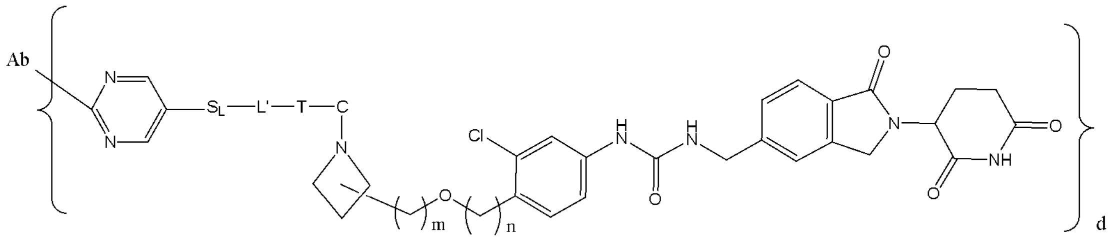

(II-b4)

further preferably, formula (II) has structures represented by formula (II-c1) to formula (II-c5):

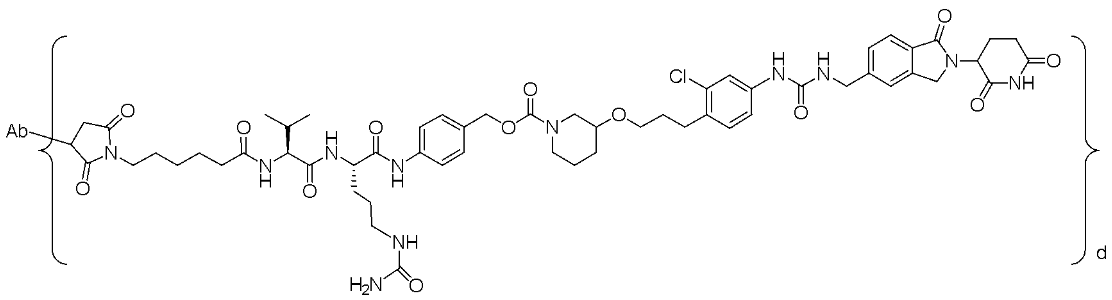

(II-c1)

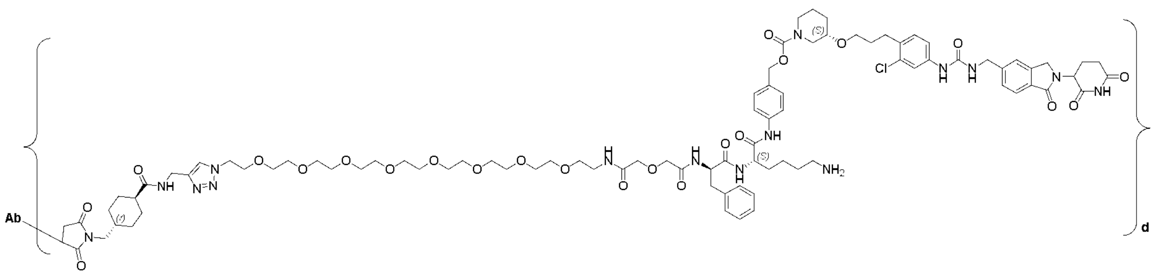

II-c2

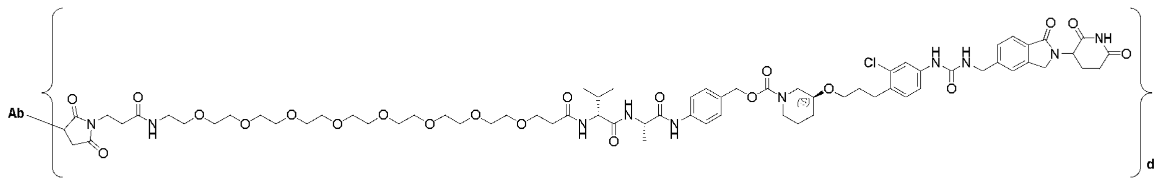

II-c3

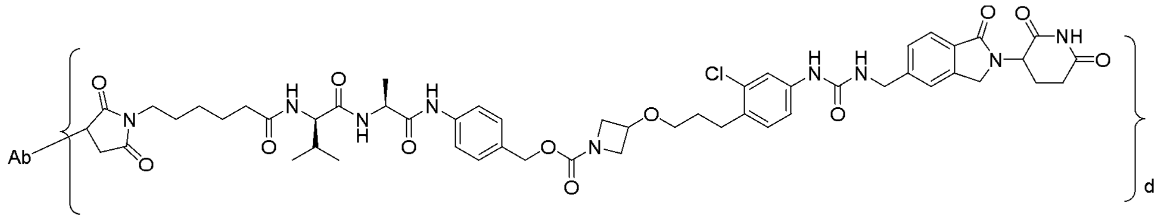

II-c4

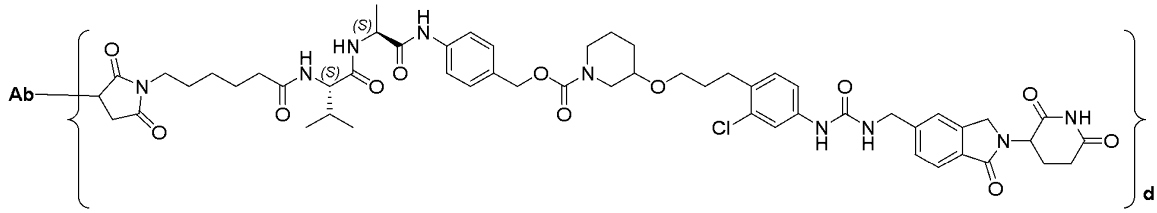

II-c5

**15.** The antibody-drug conjugate of formula (II), and the pharmaceutically acceptable salt, the hydrate, the solvate, the stereoisomer or the isotopically labeled compound thereof according to claim 8, wherein Ab is selected from trastuzumab (combination of heavy and light chains: SEQ ID NOs. 1 and 2) , pertuzumab (combination of heavy and light chains: SEQ ID NOs. 3 and 4), SYM003 (combination of heavy and light chains: SEQ ID NOs. 5 and 6), SYM004 (combination of heavy and light chains: SEQ ID NOs. 7 and 8), and belantamab (combination of heavy and light chains: SEQ ID NOs. 9 and 10).

**16.** Use of the compound, or the tautomer, the mesomer, the racemate, the enantiomer or the diastereoisomer thereof, and the pharmaceutically acceptable salt, the hydrate or the solvate thereof according to any one of claims 1-7, or the antibody-drug conjugate, and the pharmaceutically acceptable salt, the hydrate, the solvate, the stereoisomer or the isotopically labeled compound thereof according to any one of claims 8-15 in the preparation of a medicament for treating cancer.

**17.** The use according to claim 16, wherein the cancer comprises liver cancer, kidney cancer, lung cancer (e.g., small cell lung cancer and non-small cell lung cancer), gastric cancer, esophageal cancer, urethral cancer, bladder cancer, colon cancer, rectal cancer, prostate cancer, breast cancer, ovarian cancer, pancreatic cancer, melanoma, hematological tumors or glioblastoma multiforme, lymphoma (e.g., Hodgkin lymphoma, non-Hodgkin lymphoma, or relapsed anaplastic large cell lymphoma), cervical cancer, uterine cancer, endometrial cancer, salivary gland cancer, glioma, neuroblastoma, sarcoma, colorectal cancer, leukemia (e.g., acute lymphoblastic leukemia, acute myeloid leukemia, acute promyelocytic leukemia, chronic myeloid leukemia, or chronic lymphocytic leukemia), bone cancer, skin cancer, thyroid cancer, etc.; the cancer is preferably a cancer associated with abnormal expression of HER2, Claudin18.2, DLL3, or BCMA; further preferably, the cancer associated with abnormal expression of HER2 comprises lung cancer, breast cancer (e.g., ductal breast carcinoma), ovarian cancer, endometrial cancer, gastric cancer, and prostate cancer, the cancer associated with abnormal expression of DLL3 comprises lung cancer (e.g., small cell lung cancer and non-small cell lung cancer), and the cancer associated with abnormal expression of BCMA comprises lymphoma (multiple myeloma).

**18.** A pharmaceutical composition, comprising the compound, or the tautomer, the mesomer, the racemate, the enantiomer or the diastereoisomer thereof, and the pharmaceutically acceptable salt, the hydrate or the solvate thereof according to any one of claims 1-7, or the antibody-drug conjugate, and the pharmaceutically acceptable salt, the hydrate, the solvate, the stereoisomer or the isotopically labeled compound thereof according to any one of claims

8-15, and one or more pharmaceutically acceptable excipients.

**19.** A compound of formula (III):

Sr-L'-T-C-D (III)

wherein Sr, L', T, C, and D have the same definitions as in claim 8; preferably, the compound of formula (III) has a structure of formula (III-a):

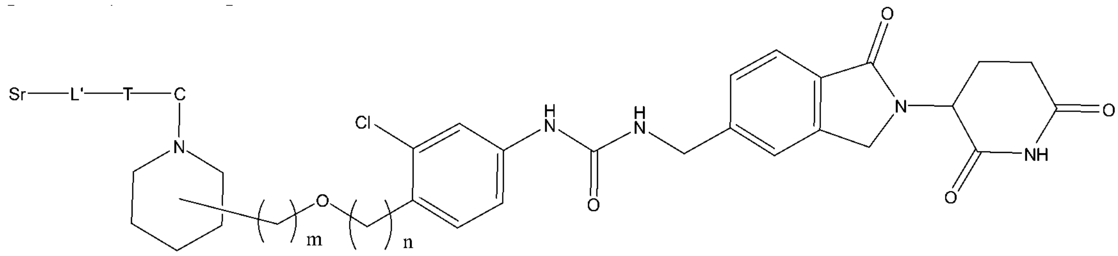

III-a1

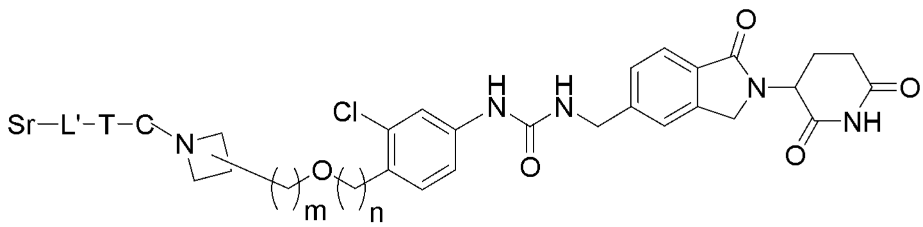

III-a2

wherein m and n are each independently selected from an integer of 0-6, such as 0, 1, 2, 3, 4, 5, or 6; preferably, Sr comprises a maleimide group or a methylsulfonylpyrimidine group, L' is absent, T is a peptide fragment, and C is

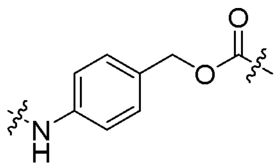

;

further preferably, the compound of formula (III) comprises compounds of formula (III-b1) to formula (III-b5):

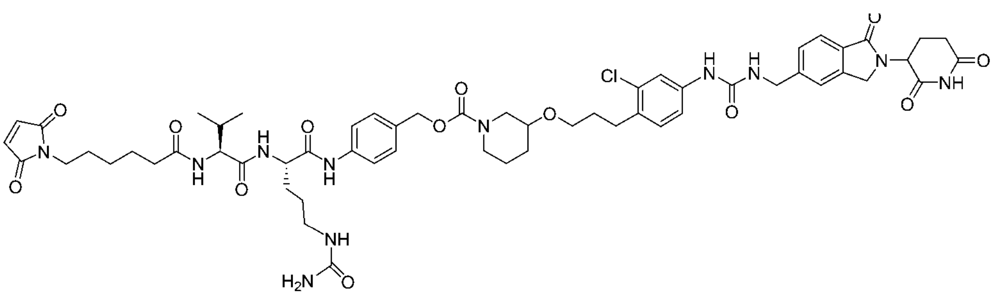

III-b1

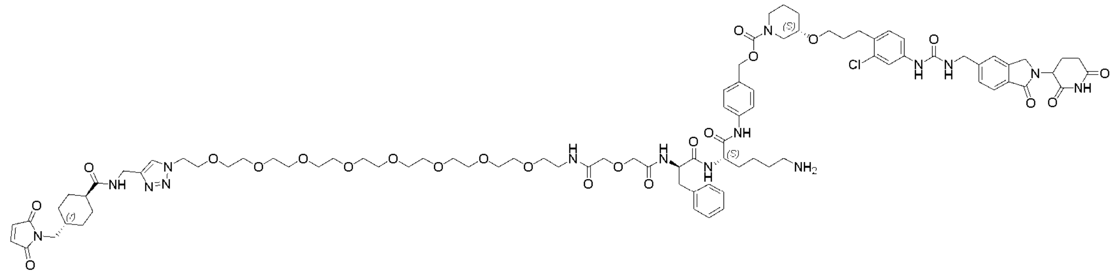

III-b2

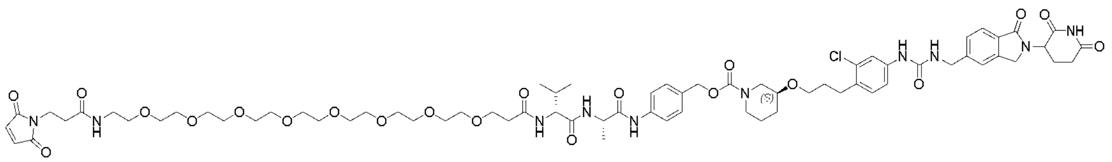

III-b3

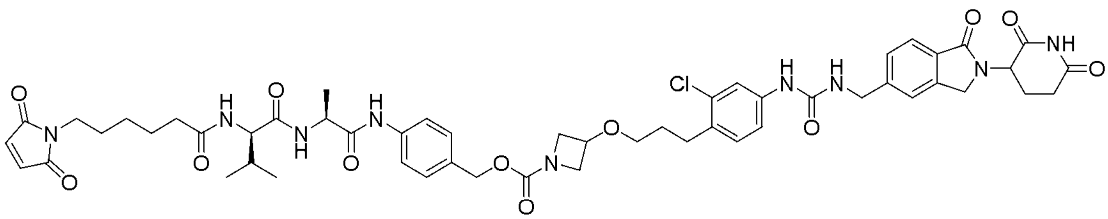

III-b4

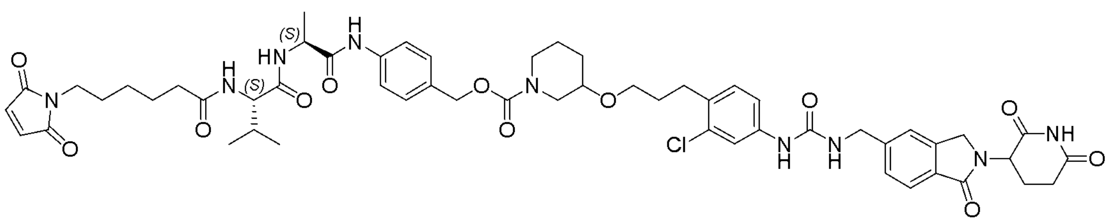

III-b5

**20.** Use of the compound of formula (I) according to claim 1 in the preparation of an antibody-drug conjugate.

## INTERNATIONAL SEARCH REPORT

International application No. **PCT/CN2024/076883**

**A. CLASSIFICATION OF SUBJECT MATTER**

C07D 401/14(2006.01)i; C07D 401/04(2006.01)i; A61K 47/68(2017.01)i; A61K 39/00(2006.01)i; A61P 35/00(2006.01)i

According to International Patent Classification (IPC) or to both national classification and IPC

**B. FIELDS SEARCHED**

Minimum documentation searched (classification system followed by classification symbols)

IPC: C07D A61K A61P

Documentation searched other than minimum documentation to the extent that such documents are included in the fields searched

Electronic data base consulted during the international search (name of data base and, where practicable, search terms used)

CNTXT, VEN, STN(Registry, Caplus), CNKI, 万方, WANFANG: 偶联, 缀合, 降解, 度胺, 分子胶, 连接链, 癌, 肿瘤, conjugate, degradate, domide, molecular glue, linker, cancer, tumor, structural formula search

**C. DOCUMENTS CONSIDERED TO BE RELEVANT**

| Category* | Citation of document, with indication, where appropriate, of the relevant passages | Relevant to claim No. |
|---|---|---|
| X | WO 2021198965 A1 (ORUM THERAPEUTICS INC.) 07 October 2021 (2021-10-07) claims 1, 39-40, 42, 49, and 50 | 1-20 |
| X | CN 114262319 A (NANCHANG AORUI PHARMACEUTICAL CO., LTD.) 01 April 2022 (2022-04-01) claims 7, 9, and 11 | 1-4, 16-18 |
| A | WO 2022254376 A1 (ORUM THERAPEUTICS INC.) 08 December 2022 (2022-12-08) entire document | 1-20 |
| A | WO 2022254377 A1 (ORUM THERAPEUTICS INC.) 08 December 2022 (2022-12-08) entire document | 1-20 |
| A | CN 102264720 A (CELGENE CORP. et al.) 30 November 2011 (2011-11-30) entire document | 1-20 |
| A | CN 106660991 A (CELGENE CORP.) 10 May 2017 (2017-05-10) entire document | 1-20 |

☐ Further documents are listed in the continuation of Box C. ☑ See patent family annex.

* Special categories of cited documents:

"A" document defining the general state of the art which is not considered to be of particular relevance

"D" document cited by the applicant in the international application

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| **26 May 2024** | **29 May 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/076883**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2024/076883**

| Patent document cited in search report | Publication date (day/month/year) | Patent family member(s) | Publication date (day/month/year) |
|---|---|---|---|
| WO 2021198965 A1 | 07 October 2021 | CA 3173118 A1 | 07 October 2021 |
| | | JP 2023519974 A | 15 May 2023 |
| | | KR 20230051120 A | 17 April 2023 |
| | | IL 296846 A | 01 November 2022 |
| | | EP 4126067 A1 | 08 February 2023 |
| | | AU 2021249532 A1 | 27 October 2022 |
| | | BR 112022019532 A2 | 06 December 2022 |
| | | MX 2022011825 A | 04 January 2023 |
| | | US 2023338564 A1 | 26 October 2023 |
| | | CN 115867322 A | 28 March 2023 |
| | | IN 202217060781 A | 25 August 2023 |
| | | VN 96049 A | 25 July 2023 |
| CN 114262319 A | 01 April 2022 | CN 114262319 B | 05 May 2023 |
| WO 2022254376 A1 | 08 December 2022 | EP 4346909 A1 | 10 April 2024 |
| | | KR 20240040067 A | 27 March 2024 |
| | | AU 2022287316 A1 | 14 December 2023 |
| | | AU 2022287316 A9 | 04 January 2024 |
| | | CA 3222182 A1 | 08 December 2022 |
| | | CO 2023016711 A2 | 26 February 2024 |
| | | TW 202313124 A | 01 April 2023 |
| | | IL 308811 A | 01 January 2024 |
| WO 2022254377 A1 | 08 December 2022 | AU 2022284372 A1 | 14 December 2023 |
| | | AU 2022284372 A9 | 11 January 2024 |
| | | CO 2023016712 A2 | 26 February 2024 |
| | | IL 308812 A | 01 January 2024 |
| | | TW 202313022 A | 01 April 2023 |
| | | KR 20240042598 A | 02 April 2024 |
| | | EP 4346910 A1 | 10 April 2024 |
| | | CA 3222185 A1 | 08 December 2022 |
| CN 102264720 A | 30 November 2011 | TW 201022227 A | 16 June 2010 |
| | | TWI 469974 B | 21 January 2015 |
| | | US 2016115161 A1 | 28 April 2016 |
| | | US 9550766 B2 | 24 January 2017 |
| | | IL 234164 A | 31 July 2016 |
| | | AU 2009311477 A1 | 14 May 2010 |
| | | AU 2009311477 B2 | 30 October 2014 |
| | | KR 20170007531 A | 18 January 2017 |
| | | ES 2662407 T3 | 06 April 2018 |
| | | MX 361467 B | 06 December 2018 |
| | | WO 2010053732 A1 | 14 May 2010 |
| | | BRPI 0919942 A2 | 23 December 2014 |
| | | BRPI 0919942 B1 | 19 February 2019 |
| | | EP 2985281 A2 | 17 February 2016 |
| | | EP 2985281 A3 | 30 March 2016 |
| | | EP 2985281 B1 | 27 December 2017 |
| | | US 2012252843 A1 | 04 October 2012 |
| | | US 8318773 B2 | 27 November 2012 |
| | | IL 234167 A | 28 September 2017 |
| | | CA 2957226 A1 | 14 May 2010 |
| | | CA 2957226 C | 14 August 2018 |

Form PCT/ISA/210 (patent family annex) (July 2022)

**INTERNATIONAL SEARCH REPORT**
**Information on patent family members**

International application No.
**PCT/CN2024/076883**

| Patent document cited in search report | Publication date (day/month/year) | Patent family member(s) | | | Publication date (day/month/year) |
|---|---|---|---|---|---|
| | | CA | 2741299 | A1 | 14 May 2010 |
| | | CA | 2741299 | C | 28 March 2017 |
| | | RU | 2011121567 | A | 10 December 2012 |
| | | RU | 2527952 | C2 | 10 September 2014 |
| | | HK | 1154859 | A1 | 04 May 2012 |
| | | JP | 2015227343 | A | 17 December 2015 |
| | | JP | 6034924 | B2 | 30 November 2016 |
| | | SMT | 201600025 | B | 25 February 2016 |
| | | IL | 212435 | A0 | 30 June 2011 |
| | | IL | 212435 | A | 30 June 2015 |
| | | IL | 234162 | A | 30 June 2016 |
| | | US | 2017088538 | A1 | 30 March 2017 |
| | | US | 9796698 | B2 | 24 October 2017 |
| | | UA | 107783 | C2 | 25 February 2015 |
| | | NI | 201100082 | A | 26 October 2011 |
| | | AR | 106942 | A2 | 07 March 2018 |
| | | SI | 2358697 | T1 | 29 February 2016 |
| | | PE | 20110547 | A1 | 04 August 2011 |
| | | JP | 2014224119 | A | 04 December 2014 |
| | | JP | 5998179 | B2 | 28 September 2016 |
| | | IL | 234165 | A | 31 August 2017 |
| | | MX | 2011004470 | A | 31 May 2011 |
| | | US | 2013045976 | A1 | 21 February 2013 |
| | | US | 9227954 | B2 | 05 January 2016 |
| | | IL | 228597 | A0 | 31 December 2013 |
| | | IL | 228597 | A | 31 January 2017 |
| | | KR | 20180000750 | A | 03 January 2018 |
| | | CO | 6361916 | A2 | 20 January 2012 |
| | | AR | 106945 | A2 | 07 March 2018 |
| | | ES | 2559388 | T3 | 11 February 2016 |
| | | US | 2012122865 | A1 | 17 May 2012 |
| | | US | 8222249 | B2 | 17 July 2012 |
| | | EP | 2358697 | A1 | 24 August 2011 |
| | | EP | 2358697 | B1 | 21 October 2015 |
| | | AR | 106963 | A2 | 07 March 2018 |
| | | IL | 234168 | A | 31 July 2016 |
| | | CL | 2011000957 | A1 | 16 September 2011 |
| | | IL | 234163 | A | 30 June 2016 |
| | | HUE | 026105 | T2 | 30 May 2016 |
| | | DK | 2358697 | T3 | 25 January 2016 |
| | | PL | 2358697 | T3 | 29 April 2016 |
| | | SG | 10201703508 | QA | 29 June 2017 |
| | | IL | 234166 | B | 30 April 2018 |
| | | SG | 195613 | A1 | 30 December 2013 |
| | | HRP | 20151366 | T1 | 15 January 2016 |
| | | PT | 2358697 | E | 03 February 2016 |
| | | AR | 106943 | A2 | 07 March 2018 |
| | | ZA | 201102941 | B | 25 July 2012 |
| | | EP | 3354646 | A1 | 01 August 2018 |
| | | KR | 20110089162 | A | 04 August 2011 |

Form PCT/ISA/210 (patent family annex) (July 2022)

## INTERNATIONAL SEARCH REPORT
**Information on patent family members**

International application No.
**PCT/CN2024/076883**

| Patent document cited in search report | Publication date (day/month/year) | Patent family member(s) | Publication date (day/month/year) |
|---|---|---|---|
| | | KR 101696938 B1 | 16 January 2017 |
| | | PE 20140963 A1 | 06 August 2014 |
| | | AR 106944 A2 | 07 March 2018 |
| | | NZ 592425 A | 26 April 2013 |
| | | AR 074001 A1 | 15 December 2010 |
| | | JP 2012507541 A | 29 March 2012 |
| | | JP 5603876 B2 | 08 October 2014 |
| | | US 2010204227 A1 | 12 August 2010 |
| | | US 8129375 B2 | 06 March 2012 |
| CN 106660991 A | 10 May 2017 | MX 2020007485 A | 14 September 2020 |
| | | IL 276946 A | 29 October 2020 |
| | | IL 276946 B | 01 August 2022 |
| | | JP 2021011495 A | 04 February 2021 |
| | | JP 2017525757 A | 07 September 2017 |
| | | JP 6779870 B2 | 04 November 2020 |
| | | US 2018221361 A1 | 09 August 2018 |
| | | CO 2017000223 A2 | 31 March 2017 |
| | | PL 3166937 T3 | 28 February 2020 |
| | | HRP 20191821 T1 | 27 December 2019 |
| | | EP 3166937 A1 | 17 May 2017 |
| | | EP 3166937 B1 | 04 September 2019 |
| | | DK 3166937 T3 | 21 October 2019 |
| | | US 2017007590 A1 | 12 January 2017 |
| | | US 9808451 B2 | 07 November 2017 |
| | | BR 112017000550 A2 | 14 November 2017 |
| | | BR 112017000550 B1 | 10 January 2023 |
| | | RS 59619 B1 | 31 January 2020 |
| | | AU 2015287688 A1 | 02 February 2017 |
| | | AU 2015287688 B2 | 02 May 2019 |
| | | SG 10202010780 WA | 30 December 2020 |
| | | HUE 046000 T2 | 28 January 2020 |
| | | ZA 201700217 B | 25 April 2018 |
| | | CA 2954784 A1 | 14 January 2016 |
| | | CA 2954784 C | 07 March 2023 |
| | | CY 1122340 T1 | 27 January 2021 |
| | | SA 517380693 B1 | 14 April 2021 |
| | | US 2018021325 A1 | 25 January 2018 |
| | | US 9968596 B2 | 15 May 2018 |
| | | PT 3166937 T | 12 December 2019 |
| | | EP 3594211 A1 | 15 January 2020 |
| | | EP 3594211 B1 | 25 October 2023 |
| | | SI 3166937 T1 | 31 January 2020 |
| | | US 2022387413 A1 | 08 December 2022 |
| | | EA 201790174 A1 | 31 July 2017 |
| | | EA 033633 B1 | 12 November 2019 |
| | | US 2020163948 A1 | 28 May 2020 |
| | | US 11241423 B2 | 08 February 2022 |
| | | JP 2023011732 A | 24 January 2023 |
| | | KR 20170036698 A | 03 April 2017 |
| | | KR 102477960 B1 | 16 December 2022 |

Form PCT/ISA/210 (patent family annex) (July 2022)

**INTERNATIONAL SEARCH REPORT**
**Information on patent family members**

International application No.
**PCT/CN2024/076883**

| Patent document cited in search report | Publication date (day/month/year) | Patent family member(s) | | | Publication date (day/month/year) |
|---|---|---|---|---|---|
| | | LT | 3166937 | T | 25 November 2019 |
| | | NZ | 765933 | A | 29 October 2021 |
| | | ECSP | 17002135 | A | 31 March 2017 |
| | | NZ | 728127 | A | 27 August 2021 |
| | | AR | 101189 | A1 | 30 November 2016 |
| | | IL | 250008 | A0 | 30 March 2017 |
| | | IL | 250008 | B | 30 September 2020 |
| | | US | 2016009683 | A1 | 14 January 2016 |
| | | US | 9499514 | B2 | 22 November 2016 |
| | | TW | 201613887 | A | 16 April 2016 |
| | | TWI | 660952 | B | 01 June 2019 |
| | | WO | 2016007848 | A1 | 14 January 2016 |
| | | ES | 2760003 | T3 | 12 May 2020 |
| | | SG | 11201700126 | RA | 27 February 2017 |
| | | MX | 2017000408 | A | 01 May 2017 |
| | | CL | 2017000075 | A1 | 11 August 2017 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202310116140
- WO 2021198965 A1


### Non-patent literature cited in the description


- **BECK A** ; **REICHERT JM**. Antibody-drug conjugates: Present and future. *MAbs*, 2014, vol. 6, 15-17
- **MCCOMBS J R** ; **OWEN S C**. Antibody drug conjugates: design and selection of linker, payload and conjugation chemistry. *The AAPS journal*, 2015, vol. 17, 339-351